(19) **Europäisches Patentamt / European Patent Office / Office européen des brevets**

(11) **EP 4 751 770 A1**

(12) # EUROPEAN PATENT APPLICATION

(43) Date of publication:
**03.06.2026  Bulletin 2026/23**

(21) Application number: **25218235.7**

(22) Date of filing: **25.11.2025**

(51) International Patent Classification (IPC):
**A61N 7/00** *(2006.01)*   **A61N 7/02** *(2006.01)*
**A61K 9/00** *(2006.01)*   *A61M 37/00* *(2006.01)*

(52) Cooperative Patent Classification (CPC):
**A61N 7/00; A61K 9/0009; A61N 7/02;**
A61M 37/0092

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **29.11.2024  FR 2413251**

(71) Applicant: **AlphaOnco**
**75016 Paris (FR)**

(72) Inventor: **Alphandéry, Edouard**
**75016 Paris, France (FR)**

(54) **INTERFERENCE SYSTEM COMPRISING AT LEAST TWO  SOURCES OF RADIATION EMITTING TWO BEAMS OF RADIATION INTERFERING IN A BODY PART OR REGION TO BE TREATED AND AT LEAST ONE NANOPARTICLE**

(57)    The invention relates to radiation heating system comprising at least two orientated radiation emitting elements with a treated region of interference of the at least two radiation beams emitted by the at least two radiation emitting elements, and at least one nanoparticle comprised in the interference region.

## Description

## FIELD OF THE INVENTION

The field of the invention is that of an interference system comprising at least two sources of radiations emitting at least two beams of radiation interfering in the body part or region to be treated in the presence of at least one nanoparticles.

## TECHNICAL BACKGROUND

It has previously been shown that it was possible to destroy tumors by heating them under high intensity focused radiations (HIFU), (A. Napoli et al, High-intensity focused radiation for prostate cancer, Expert Rev Med Devices, 17, P. 427-433 (2020)). HIFU necessitates high heating temperatures and an apparatus generating radiations of high intensity with a beam, which is highly focused. HIFU is not easy to perform and requires the use of a rather complex apparatus. Here, we propose the design of a simpler equipment than that used to perform HIFU that preferentially works under lower intensity, and preferentially generates radiation beams that are not as focused as those used in HIFU.

## DESCRIPTION OF THE INVENTION

Preferentially, this invention introduces a new apparatus that can enable performing radiation heating or exposure of a pathological site such as a tumor under or by a lower intensity of radiation than that used during HIFU. This apparatus can comprise at least two sources of radiation or radiation emitting elements whose surfaces or emitting part or piezo element can be orientated with a certain or given angle, preferentially between the at two axis of propagation of the at least two beams of radiation, or between at least one axis of propagation of at least one beam of radiation and the horizontal or vertical axis or an axis crossing the body part or region to be treated or an axis such as the axis of elongation of at least one part of the system, in such a way that the at least two radiation beams emitted by the at least two radiation sources or radiation emitting elements interfere, preferentially spatially and/or temporally, in a region, designated as the interference or common region, which also preferentially comprises or is comprised the body part or region to be treated, which is also preferentially treated and/or heated and/or imaged. As an illustration, among the different types of systems, we can distinguish two different types of probe or system or part of the system introduced here. The first one can comprise at least one or two heating radiation emitting element(s) or radiation source(s) and at least one imaging radiation emitting element with the interference region consisting in the region of interference between the imaging and heating radiation beams. The second one can consist of at least two heating radiation emitting elements or sources of radiations with the interference region consisting in the region of interference between the two heating radiation beams emitted by the two heating radiation emitting elements or sources of radiation. In addition, this new type of system, also designated as radiation system, preferentially designed as LIR for low intensity radiation, can emit at least one or two unfocused radiation beam(s) or at least one or two radiation beam(s) that is/are less focused than that/those used in HIFU, as well as at least one or two radiation beam(s) of low intensity or of lower intensity than in HIFU, preferentially with a series of advantageous features compared with HIFU, such as: i) a potential reduced cost of fabrication, which is essentially due to the fact that LIR can require much less power to operate than HIFU, ii) a heated region of potentially larger size for LIR than for HIFU, which can lead to the treatment of a larger volume of a diseased body part or region during each treatment or treatment session, iii) a potentially lower heating tumor temperature of typically 43 to 55 °C, preferentially 45 °C, for LIR compared with typically 80-90 °C for HIFU. The method for using radiations of low intensity in the treatment of tumor was described in a previous patent (Pat Appl. Number US 16/486574 entitled *"Nanoparticle sequentially exposed to low intensity acoustic waves for medical or cosmetic applications"*). Here, we describe the probe or system or part of it enabling to generate such radiations in the presence of nanoparticles. As observed by us (C. El Hedjaj et al, Full Disappearance of PC3-Luc Prostate Tumors Mediated by Hyperthermia Under Low Intensity Ultrasound Application in the Presence of Magnetosomes, Adv. Therap., 2400281 (2025). DOI: 10.1002/adtp.202400281), exposing tumors to low intensity ultrasound leading to moderate tumor heating temperature (typically 43-45 °C on average), leads to tumor growth retardation in the absence of nanoparticles and to full tumor disappearance in the presence of nanoparticles, being therefore less efficient in destroying tumors in the absence than in the presence of nanoparticles, which is the reason why we introduce nanoparticles in the system.

In some cases, the radiation emitting element can be or comprise a transducer or piezo element or piezo element or an element or equipment or apparatus or source of radiation or part of it or the surface or emitting part or piezo element of it, preferentially emitting radiation or at least one beam of radiation. In some cases, at least two radiation beams preferentially emitted by at least one or two radiation emitting elements interfere, preferentially spatially, when they interfere or are located or comprised in the same or at least two different region(s), preferentially at the same or at least two different time point(s), preferentially comprising an interference region or a region in which the interreference occurs or the body part or region to be treated, partly or fully.

In some cases, the interference region can be designated as the common region or the region of interference or common region of at least two or three beams of radiation, preferentially in some cases comprising at least one nanoparticle.

In some cases, the interference region can comprise at least 1 or 2 common regions or the sum of the common regions, where the common region is preferentially the region of overlap or superposition of two or at least two beams of radiations, where the interference region is preferentially the region of overlap or superposition of all beams of radiations preferentially emitted by the sources of radiation preferentially of the system according to the invention.

In some other cases, at least two radiation beams preferentially emitted by at least one or two radiation emitting elements interfere, preferentially temporally, when they interfere or are located or comprised in the same or at least two different regions, preferentially at the same or at least two different time point(s), preferentially comprising an interference region or a region in which the interreference occurs or the body part or region to be treated, partly or fully..

In some cases, the interference region or region in which the interreference occurs is a body part or region or pathological site or tumor, partly or fully.

The present invention preferentially concerns a probe or system or part of the system, preferentially an interference one, preferentially a radiation probe or system or part of the system or radiation transrectal probe or system or part of the system, for treating a body part or region of an individual, preferentially at least one pathological site, tumor, cancer, solid tumor, metastatic or primar or localized or organ confined pathological site or tumor, or prostate, preferentially prostate cancer or tumor or hyperplasia, preferentially by hyperthermia and/or by exposing the body part or region to radiations.

The invention relates to a system or an interfering or interacting system, preferentially for treating a body part or region of an individual, preferentially through the interaction or interference of at least one, two, three, four or n beam(s) of radiation, where n is preferentially an integer, preferentially different from 0 or 1, preferentially larger or equal than 1, 2, 3, 4, 5, 10, 25, 30, 40, 50, 75 or 100, where the system preferentially comprises two parts:

- A first part preferentially comprising or consisting of at least two different sources of radiation preferentially emitting at least two different beams of radiation, preferentially having at least one property selected in the group consisting of:

  i) They preferentially do not interfere or interact preferentially in at least one region preferentially comprising at least one source of radiation, preferentially the emission region, and/or preferentially not comprising the boy part to be treated,
  ii) They preferentially interfere or interact preferentially in at least one interfering or interacting region, which is preferentially comprised in or preferentially comprises the body part or region to be treated and/or preferentially not comprised in at least one region preferentially comprising at least one source of radiation;
  iii) They are preferentially emitted or comprise at least one axis or direction preferentially of propagation preferentially orientated in the direction of the interference region or body part or region to be treated;

- A second part preferentially comprising or consisting of at least one nanoparticle or preferentially of an assembly of at least two nanoparticles,

  wherein part or all of the first part preferentially does not contain or comprise or differs from part or all of the second part;
  wherein preferentially the at least two sources of radiation of the first part comprise preferentially at least one or two emitting element(s) or element(s) emitting at least one or two radiation(s) or beam(s) of radiation, which are preferentially different in type or location or path or direction of propagation;
  wherein preferentially the at least two emitting element(s) are or consist of or comprise a segment, a length, a spire, a conductor of current or electromagnetic wave or radiation, a probe or system or part of the system, an optical fiber, and/or a transducer or piezo element or piezo element, fully or in part, and/or a surface or emitting part or piezo element, a volume, a length of it/them,
  wherein preferentially part or all of the first part, second part, emission region and/or interference region, is/are characterized by at least one property selected in the group consisting of:

    i) The first and second parts are preferentially separated from each other, preferentially by at least 1 nm, 1 $\mu$m, 1 mm, or 1 cm;
    ii) The first part is preferentially comprised in the emission region and/or outside of the body part or region to be treated or interference region;
    iii) The second part is preferentially comprised outside of the emission region and/or inside the body part or region to be treated or interference region;
    iv) The emission and interference regions are preferentially separated from each other, preferentially by at least 1 nm, 1 $\mu$m, 1 mm, or 1 cm;

  wherein preferentially in a first configuration of the system, at least one beam of radiation preferentially emitted by the first part is focused, preferentially by the second part that preferentially absorbs, reflects, transmits, diffracts, amplifies, increases, decreases or maintains the at least one beam of radiation or at least one of its parameter preferentially more strongly than a portion of body part or region not comprising the nanoparticle or through the narrowing of the beam during its transmission preferentially between the emission and interference regions, wherein preferentially in a second configuration of the system, at least one beam of radiation emitted by the first part is un-focused, preferentially in the absence of the second part or through the propagation of the beam outside of at least one portion of body part or region comprising at least one nanoparticle or through the widening of the beam of radiation during its transmission preferentially between the emission and interference region, wherein preferentially at least one parameter of the at least two beams is larger, smaller or different in the interference region or body part or region to be treated than in at least one emission region or in at least one region located outside the interference region or body part or region to be treated or between the emission and interference region, preferentially to avoid the production of a too large amount of heat and/or radical species in at least one region located outside the interference region or body part or region to be treated or between the emission and interference region; wherein preferentially the at least one parameter of the at least one beam of radiation is selected in the group consisting of: the intensity, power, energy frequency and/or associated amount of produced heat and/or radical species produced by the at least one beam of radiation, preferentially per unit length, surface or emitting part or piezo element or volume of body part or region to be treated, emitting region, interference region, region outside the emission or interference region, and/or emitting element; wherein preferentially part or all of the second part is comprised in the interference region or body part or region to be treated, wherein preferentially the second part preferentially absorbs, reflects, transmits, diffracts, amplifies, increases, decreases or maintains the at least one beam of radiation to which it is exposed or at least one of its parameters, wherein preferentially the second part optionally emits or reflects or transmits or diffracts another beam of radiation or beam of radiation originating from the interference or interaction between at least one or two beam(s) of radiation and at least one nanoparticle, wherein preferentially the other beam of radiation has at least one parameter that differs from that of the beam of radiation to which it has initially been exposed; wherein preferentially the other beam of radiation is preferentially emitted by another emitting element, preferentially different from the source of radiation, preferentially comprising at least one nanoparticle, wherein preferentially the at least one nanoparticle of the second part comprises at least two different sides and/or facets, wherein preferentially at least two different sides or facets are exposed to at least two different beams of radiation preferentially emitted by at least two different sources of radiation or emitting elements, wherein preferentially the at least two different sides and/or facets have at least one property selected in the group consisting of:

i) At least one different surface or emitting part or piezo element or volume or cross section, preferentially in terms of composition, location, size, capacity to absorb or diffract or reflect or emit or diffuse radiation, heat, cold and/or at least one compound or atom or ion comprised or originating from the at least one nanoparticle such as radical oxygen species,

ii) At least one distance separating at least two different sides or facets or at least two different locations comprised in these different sides or facets larger than 0.1 or 1 nm,

iii) At least one distance separating at least two different sides or facets or at least two different locations comprised in these different sides of facets smaller than at least one nanoparticle diameter or than 1 $\mu$m, 100 nm, 10 nm or 1 nm,

iv) The at least two different sides or facets comprise at least one surface or emitting part or piezo element or volume that is partly or fully exposed to at least one different part of the body part or region,

v) The temperature or quantity of radical oxygen species produced or emitted individually by one side or facet exposed to one beam of radiation is smaller than the temperature or quantity of radical oxygen species produced or emitted collectively by at least two different sides or facets exposed to at least two different beams of radiation, and

vi) A temperature diffusion preferentially occurring between at least two different sides or facets occurring preferentially more easily or more efficiently than between two locations of the body part or region, preferentially separated by the same or a similar distance than that which separates at least two different facets or sides, preferentially not comprising at least one nanoparticle.

Preferentially the at least two different emitting elements belong to at least two different sources of radiation are comprised in at least two different locations, preferentially comprised in part or fully above, below, on the right side, on the left side, and/or on at least one or two side(s) of the body part or region to be treated or of at least one cross-section of the body part or region to be treated.

Preferentially at least one or two source(s) of radiation is or are preferentially located outside of the interference region or body part or region to be treated, preferentially at a distance of more than 1 $\mu$m, 100 nm, 10 nm or 1 nm from the body part or region to be treated or interference region.

Optionally the body part or region to be treated or interference region is a region that is heated and/or produces or comprises radical species, preferentially following or by or through the interference of the at least two beams of radiation, preferentially in the presence of the second part, preferentially in some cases by more than $10^{-5}$, $10^{-3}$, $10^{-1}$, 0, 1, 5 or 10 °C, preferentially in some other cases by less than $10^5$, $10^3$, 150, 100, 50, 25 or 15°C, preferentially in comparison with the temperature of at least region outside the heated region or not exposed to at least one or two beam(s) of radiation or portion of body part or region not comprising the at least one nanoparticle.

In some cases, the number of beam(s) of radiation, source(s) of radiation, and/or beam(s) of radiation emitted per each source of radiation is/are larger than 0, 1, 2, 3, 4, 5, 10, 20, 25, 30, 40, 50 or 10.

In some other cases, the number of beam(s) of radiation, source(s) of radiation, and/or beam(s) of radiation emitted per each source of radiation is/are smaller than $10^5$, $10^3$, 500, 250, 100, 50, 20, 10, 5, 2, 1 or 0.

In some other cases, the beam(s) of radiation, source(s) of radiation, and/or beam(s) of radiation emitted per each source of radiation is/are located below, above, on top of, on, on the same side, on a different side, at the center, on the edge, inside, outside, discontinuously, or continuously with respect to an imaging probe, the system, at least one part of the system, interference or emission region or region separating the emission and interference region, at least one nanoparticle.

The invention relates to a radiation heating probe or system or part of the system preferentially for treating a body part or region of an individual preferentially by hyperthermia,

wherein the heating probe or system or part of the system preferentially comprises at least two radiation emitting elements or radiation emitting element surfaces or emitting part or piezo element, wherein each radiation emitting element or radiation emitting element surface or emitting part or piezo element preferentially has at least one axis of radiation beam emission orientated in the direction of a heated region,

wherein the heating probe or system or part of the system is preferentially configured so that at least two radiation beams emitted by the at least two radiation emitting elements or radiation emitting element surfaces or emitting part or piezo element preferentially interfere in the heated region,

wherein the heated region is preferentially located at a distance of more than 0.1 cm from the surface or emitting part or piezo element of at least one radiation emitting element or from the surface of the body part or region,

wherein the heated region preferentially has a temperature that is larger, preferentially by at least 0, 0.1 or 1 °C, than the temperature of the individual or than the physiological temperature or than 36.5 or 37.5 °C,

wherein the heated region preferentially has a temperature that is lower than the temperature of thermo-ablation or $10^5$, $10^3$, 60 or 80°C,

preferentially wherein the heated region has a larger temperature or acoustic pressure than the temperature or acoustic pressure of at least one of the following locations: i) the radiation emitting element surface or emitting part or piezo element, ii) the surface of the body part or region, and/or iii) at least one volume of the body part or region located between the radiation emitting element surface or emitting part or piezo element or surface of the body part or region or individual and the heated region. In some cases, the radiation emitting element surface or emitting part or piezo element is the surface or emitting part or piezo element of the radiation emitting element.

In some cases, the heated region is, is in comprised in or comprises the body part or region to be treated or the interference region, in part or in full. The invention also relates to a probe or system or part of the system according to the invention, preferentially for treating a body part or region of an individual, preferentially by hyperthermia,

which preferentially consists of two parts:

- A first part, which preferentially comprises at least 1, 2, 3, 4, 5 or 10 radiation emitting element(s), preferentially radiation imaging probe or system or part of the system(s);

- A second part, which preferentially comprises at least 1, 2, 3, 4, 5 or 10 radiation radiation emitting element(s), which are preferentially different in terms of size, location and/or orientation of the radiation beams that they emit than those of the first part, preferentially radiation heating probe or system or part of the system(s);

wherein the first part preferentially comprises at least 1, 2, 3, 4, 5 or 10 imaging radiation emitting element(s), or radiation emitting element(s) preferentially generating an image or used under the conditions that produce an image, of the body part or region to be treated,

wherein the second part preferentially comprises at least at least 1, 2, 3, 4, 5 or 10 heating radiation emitting element(s) or radiation emitting element(s) preferentially generating heat or used under the conditions that produce heat, in or within the body part or region to be treated,

wherein the center or bottom of the at least one first source of radiation or imaging radiation emitting element or at least one component or part of the imaging radiation emitting element is preferentially separated from at least one second source of radiation or the heating radiation emitting element or at least one component or part of the heating radiation emitting element preferentially by a distance that is larger than: i) $10^{-3}$, 0.1, 0.5, 1, 2 or 5 cm, or ii) half diameter of the interference region or tumor or body part or region to be treated,

wherein the probe or system or part of the system preferentially comprises at least two sources of radiation or radiation emitting elements or radiation emitting element surfaces or emitting part or piezo element, wherein at least one or two or each source(s) of radiation or radiation emitting element(s) or radiation emitting element surface(s) or emitting part or piezo element(s) preferentially has at least one axis of radiation beam or beam emission preferentially orientated in the direction of the body part or region to be treated or interference region,

wherein the probe or system or part of the system is preferentially configured so that at least one or two radiation beam(s) preferentially emitted by the at least one or two radiation source(s) or radiation emitting element(s) or radiation emitting element surface(s) or emitting part or piezo element(s) preferentially interfere in the body part or region to be treated or interference region,

In one embodiment of the invention, the interference of the at least two radiation beam(s) or beam(s) of radiation is a superposition of the at least two beams, preferentially in the same location or focal point or region or foci, preferentially at the same time, preferentially comprised in the interference region or body part or region to be treated, wherein the superposition is preferentially characterized by a higher temperature, beam intensity, pressure, preferentially of the at least two beams, preferentially compared with at least one beam, preferentially by at least 0, $10^{-10}$, $10^{-1}$, 1, 5, 10, 50 °C, W, W/cm, W/cm$^2$, or W/cm$^3$ or %, wherein the same location is preferentially a location preferentially comprising at least one or two beam(s) of radiation, preferentially a location of size smaller than $10^3$, 100, 50, 20, 10, 5, 2, 1 cm or % preferentially within or of the interference region or body part or region to be treated, wherein the same time is preferentially a duration of superposition of the at least two beams, preferentially in a first case of duration smaller than $10^5$, $10^3$, 100, 50, 10, 5, 2, 1, 0, $10^{-3}$ second(s), minute(s) or day(s), preferentially in a second case of duration larger than $10^{-10}$, $10^{-3}$, $10^{-1}$, 0, 1, 5 or 10 second(s), minute(s) or day(s).

In another embodiment of the invention, the interference of the at least two radiation beam(s) or beam(s) of radiation is a coexistence preferentially without superposition of the at least two beams, preferentially in at least two different locations or focal points or regions or foci, preferentially in some cases at the same time, preferentially in some other cases at a different time, preferentially comprised in the interference region or body part or region to be treated, wherein the coexistence is preferentially characterized by a higher temperature, beam intensity, pressure, preferentially of the at least two beams, preferentially in at least two different locations, preferentially compared with at least one beam, preferentially localized in at least one location, preferentially by at least 0, $10^{-10}$, $10^{-1}$, 1, 5, 10, 50 °C, W, W/cm, W/cm$^2$, or W/cm$^3$ or %, wherein the at least one or two location(s) preferentially has/have at least one property selected in the group consisting of: i) it/they comprise(s) at least one or two beam(s) of radiation, ii) it/they have size(s) smaller than $10^3$, 100, 50, 20, 10, 5, 2, 1 cm or % preferentially within or of the interference region or body part or region to be treated, iii) it/they have size(s) larger than $10^{-5}$, $10^{-3}$, $10^{-1}$, 0, 1, 5 or 10 cm or % preferentially within or of the interference region or body part or region to be treated, iv) the at least two locations are separated from each other by more than $10^{-10}$, $10^{-5}$, $10^{-3}$, $10^{-1}$, 0, 1, 5, or 10 mm or cm, v) the at least two locations are separated from each other by less than $10^{10}$, $10^5$, $10^3$, 10, 5, 2, 1 mm or cm, wherein the same time is preferentially a duration during which the at least two beams coexist, wherein a different time is preferentially a combination of a first duration during which the at least one first beam exists and of a second duration during which the at least one second beam exists, where the first and second durations are preferentially separated by more than $10^{-5}$, $10^{-3}$, 0, 1, 5, 10 or 100 second(s), minute(s) or day(s) in a first case, by less than $10^5$, 10, 1 or 0 second(s), minute(s) or day(s) in second case, wherein the duration is preferentially smaller than $10^5$, $10^3$, 100, 50, 10, 5, 2, 1, 0, or $10^{-3}$ second(s), minute(s) or day(s) in a third case, wherein the duration is preferentially larger than $10^{-10}$, $10^{-3}$, $10^{-1}$, 0, 1, 5 or 10 second(s), minute(s) or day(s) in a fourth case.

In another embodiment of the invention, the heated region is located at a distance of more than 0, $10^{-5}$ or 0.1 cm from the surface or emitting part or piezo element of at least one source of radiation or radiation emitting element or from the surface of at least one body part or region.

In another embodiment of the invention, the heated region has a temperature that is larger, preferentially by at least 0, $10^{-5}$, 1 or 5 °C, than the temperature of the individual or than the physiological temperature or than 36.5 or 37.5 °C.

In another embodiment of the invention, the heated region has a temperature that is lower than the temperature of thermo-ablation or $10^3$ or 100 or 80 or 60°C,

In another embodiment of the invention, the probe or system or part of the system preferentially is not or does not comprise an apparatus generating ablation or thermal ablation or coagulative necrosis or cavitation or inertial cavitation or high intensity focused radiation.

The invention also relates to the system according to the invention, which is a system of double interference or interaction, preferentially in which or characterized by:

- a first interference or interaction preferentially resulting from or characterized by or comprising a first type of interference or interaction between at least two beams of radiation preferentially in the interference or interaction region or region of body part or region to be treated;
- a second interference or interaction preferentially resulting from or characterized by or comprising a second type of interference or interaction between at least one beam of radiation and at least one nanoparticle preferentially in the interference or interaction region or region of body part or region to be treated.

In another embodiment of the invention, the second and first types of interferences or interactions occur coherently or additively or the second type of interference or interaction is strengthened or the temperature increase or amount of produced radical species resulting from the second type of interference or interaction is increased or strengthened by being combined or by coexisting with the first type of interference or the coexistence or combination of the first and second type of interference or interaction leads to a larger or more localized or more focused/focalized, temperature increase or amount of produced radical species, preferentially occurring within the nm scale or at the scale of the at least one nanoparticle, or at a scale smaller than at least one cell or biological material, compared with the presence of the first or second type of interference alone, preferentially occurring preferentially occurring within the $\mu$m or mm scale or at a scale larger than at least one cell or biological material. In another embodiment of the invention, the first and second types of interferences or interactions occur or are comprised in a first and second different interference or interaction regions or locations, focal points or regions.

In an embodiment, the region of interference of the first type is the location, region or portion of body part or region or body part or region to be treated where the interference of first type occurs or takes place.

In another embodiment, the region of interference of the second type is the location, region or portion of body part or region or body part or region to be treated where the interference of second type occurs or takes place.

In some cases, the regions of interference of the first and second types overlap.

In some other cases, the regions of interference of the first and second types are separated, preferentially in some cases by more than $10^{-10}$, $10^{-5}$, $10^{-1}$, 0, 1, 5 or 10 nm, mm or m, preferentially in some other cases by less than $10^{10}$, $10^5$, 10, 5, 2, 1 or 0 nm, mm or m.

In another embodiment of the invention, the second type of interference or interaction region is comprised in the first interference or interaction region or location, focal point or focal region.

In another embodiment of the invention, the first and/or second type(s) of interference occurs in the body part or region to be treated.

In another embodiment of the invention, the first and/or second type(s) of interference is optimal when the overlap between the interference region and body part or region to be treated is maximal or the best, *i.e.* preferentially the at least two beams of radiation interfere totally or mainly or predominantly in the body part or region to be treated preferentially with or in the presence of at least one nanoparticle and not or in minority in at least one region outside the body part or region to be treated or without at least one nanoparticle.

In another embodiment of the invention, the first and/or second type(s) of interference occur(s) in a different manner or with a different strength or lead to a different temperature increase or to the production of a different amount of radical species in the core or inner part of the at least one nanoparticle than in the coating or surface or outer surface of the at least one nanoparticle.

In another embodiment of the invention, the first and/or second type(s) of interference occur(s) more strongly or with higher strength or lead(s) to a larger temperature increase or to the production of more radical species in the presence of an assembly of at least two nanoparticles, than in the presence of an individual or single nanoparticle,

In another embodiment of the invention, the assembly of the at least two nanoparticles has preferentially at least one property selected in the group consisting of: i) they are arranged in a geometric figure, ii) they are linked or associated to each other, preferentially through binding or coating material, and iii) they are separated by less than 10, 1, 0.1 $\mu$m or than the diameter of at least one nanoparticle.

The invention also relates to the probe or system or part of the system according to the invention, wherein at least one first part of the system or the first and at least one second part of the system or second part or at least a portion of it/them are assembled in at least one of the following manners:

- The at least one first part of the system or first part is embedded inside the at least one second part of the system or second part or inside a portion of the second part;
- The at least one first and second part of the system can be detached or separated from each other or used separately one from another, preferably before or after the treatment of the body part or region; and
- The at least one first and second part of the system can be attached to each other, preferably during the treatment of the body part or region.

The invention also relates to the system according to the invention, which is a system of double interference or interaction, wherein:

- At least two different sources of radiation of the first part preferentially emit at least two different beams of radiation that produce or is associated with or comprises the first interference or first type of interference;
- At least one beam of radiation emitted by the first part preferentially produces or is associated with or comprises the second interference or second type of interference when it interferes or interacts with at least one nanoparticle of the second part.

The invention also relates to the probe or system or part of the system or system according to the invention, wherein the second part is a single use or disposable equipment.

In some cases, a single use equipment is an equipment that is used or designed to be used for a single treatment or session of treatment of the probe or system or part of the system, preferentially heating probe or system or part of the system.

In some cases, a single use equipment is disposed of after its use.

The invention also relates to the probe or system or part of the system according to the invention, wherein the first part is a permanent equipment or is not a single use or disposable equipment.

In some cases, a permanent equipment is an equipment that is used or designed to be used for more than 1, 2, 5 treatment(s) or session(s) of treatment of the probe or system or part of the system, preferentially heating probe or system or part of the system.

In some cases, a single use equipment is disposed of after its use.

In some cases, the probe or system or part of the system or part of it or the first or second part is a medical device.

In some cases, the probe or system or part of the system can be or comprise a heating probe or system or part of the system or a probe or system or part of the system comprising at least one source of radiation, radiation emitting element, heating radiation emitting element, *i.e.* a probe or system or part of the system that produces or can produce heat, preferentially by a heating radiation emitting element, preferentially above physiological temperature, preferentially of the body part or region to be treated. In some other cases, the probe or system or part of the system can be or comprise an imaging probe or system or part of the system or a probe or system or part of the system comprising at least one imaging radiation emitting element, *i.e.* a probe or system or part of the system that produces or can produce an image, preferentially by a imaging radiation emitting element, preferentially of the body part or region to be treated.

In some cases, the probe or system or part of the system can be an imaging and/or heating probe or system or part of the system, preferentially an imaging and/or heating radiation probe or system or part of the system.

In some cases, the heating probe or system or part of the system can be an accessory or an add-on or complementary material or medical device to a radiation imaging probe or system or part of the system, preferentially to a transrectal or boy part imaging probe or system or part of the system or to an imaging probe or system or part of the system of a body part or region or a tumor.

In some other cases, the heating probe or system or part of the system is a disposable or single-use equipment.

In still some other cases, the at least one radiation source, nanoparticle, heating probe or system or part of the system is a medical device.

In still some other cases, the at least one radiation source, nanoparticle, heating probe or system or part of the system is a drug.

In still some other cases, the at least one radiation source, nanoparticle, heating probe or system or part of the system is a composition or preparation or equipment or apparatus.

In still some other cases, the heating probe or system or part of the system is attached to or embedded with or associated with or in contact with or to an imaging or heating or radiation-emitting or ionizing device or equipment or element, preferentially an imaging or heating or radiation-emitting or ionizing device or equipment or element or probe or system or part of the system.

In still some other cases, the system or part of it can be or comprise an imaging or heating or radiation-emitting or ionizing device or drug or equipment or element or probe or apparatus or piece or composition or preparation, comprising preferentially at least one component emitting or receiving radiation, preferentially an imaging or heating or electromagnetic or ionizing radiation, and preferentially at least one support to or with which the at least one source of radiation or radiation emitting element is attached, preferentially in such way that the angle between the emitting part or piezo element or surface of the radiation emitting element or source of radiation and the axis of elongation of the support is preferentially larger than 5, 10, 20, 30 or 45 °, preferentially to enable the radiation beam(s) emitted by the at least one source of radiation or radiation emitting element, preferentially its surface or emitting part or piezo element, to be orientated in the direction of the heated or interference region or body part or region to be treated, preferentially such that the radiation beam(s) cross(s) the heated or interference region or body part or region to be treated.

In some cases, the radiation beam(s) can be emitted in a direction called the axis of radiation beam emission.

In some cases, the system or part of the system can be or comprise a probe, a module, a source of radiation, and/or a radiation emitting element or equipment.

In still some other cases, the system or part of the system is selected in the group consisting of: i) a heating or cooling system or part of the system, ii) an imaging system or part of the system iii) a system or part of the system emitting electromagnetic radiation and iv) a a system or part of the system emitting ionizing radiation..

In still some other cases, the heating probe or system or part of the system is connected to other equipment(s) than radiation emitting element(s) generating the radiations or enabling the generation of the radiations.

In some cases, the probe or system or part of the system can be an imaging probe or system or part of the system, *i.e.* a probe or system or part of the system that produces or can produce an image, preferentially by an imaging radiation emitting element, preferentially of the body part or region or pathological site.

In one embodiment, the probe or system or part of the system, preferentially the transrectal probe or system or part of the system, comprises at least two radiation emitting elements, preferentially mono-element radiation emitting elements, which are preferentially positioned in the following manner:

a) the two radiation emitting elements are attached to a support, preferentially a transrectal support or support comprised in at least one body part or region; and
b) the angle between the radiation emitting element surface or emitting part or piezo element and the horizontal axis or the axis of elongation of the rectum or the axis of elongation of the support on two which the at least two Radiation emitting elements are attached, is comprised between 1 and 90°C, preferentially between 10 and 80 °, more preferably between 20 ° and 80°;

wherein the at least two Radiation emitting elements are preferentially the radiation emitting part or piezo elements of the probe or system or part of the system.

In one embodiment of the invention, the probe or system or part of the system, preferentially transrectal probe or system or part of the system, is connected to a signal generator and/or an amplifier to generate radiation.

In one embodiment, the probe or system or part of the system is an equipment, device, preferentially a medical device, most preferentially a medical device of class I, II, IIa, IIb or III.

In some cases, the probe or system or part of the system can be inserted or located inside an individual or body part or region of an individual. In some other cases, it can be positioned or located at the surface of an individual or body part or region of an individual.

In one embodiment of the invention, the probe or system or part of the system is used to image, detect, destroy, and/or treat the body part or region of an individual.

In some cases, the individual can be a human or an animal or an assembly of cell(s) or biological material, preferentially alive or active in some cases, preferentially inactive in some other cases, preferentially denatured in still some other cases, preferentially in interactions in some other cases.

In one embodiment of the invention, the probe or system or part of the system or its Radiation emitting element emits radiations, and is then preferentially designated by radiation or radiation-emitting probe or system or part of the system.

In some cases, the probe or system or part of the system preferentially emits at least one type of radiations preferentially selected among imaging and therapeutic or heating or ionizing or electromagnetic or cooling radiations.

In some cases, the probe or system or part of the system preferentially images, detects, heals, heats, cools, ionizes, and/or exposed to electromagnetic radiation, preferentially at least one body part or region, preferentially in some cases without emitting radiation, preferentially in some other cases by emitting radiations.

In some cases, the source of radiation or imaging radiation emitting element emits imaging radiations. In some other cases, the source of radiation or heating radiation emitting element emits heating radiations.

In still some other cases, at least two different radiations or beams of radiations or sources of radiations or imaging radiations are different from therapeutic or heating radiations or from each other in at least one of the following manners:

1) a first type of radiations or imaging radiations has a lower intensity or power than a second type of radiation or therapeutic or heating radiation;
2) a first type of radiation or imaging radiation has a different frequency from a second type of radiation or therapeutic radiation, preferentially a higher frequency than the frequency of the second type of radiation or therapeutic radiation.

In some cases, a first type of or Radiation emitting element emitting radiations at high frequency can lead to radiation imaging of high resolution or to a high level of focalization of the first type of radiation. A radiation emitting element emitting radiations at high frequency can be desired or chosen to yield high resolution imaging or to produce heat preferentially locally.

In some cases, a radiation emitting element emitting radiations of low frequency can lead to radiations penetrating deeply in the body part or region of an individual. A radiation emitting element emitting radiations at low frequency can be desired or chosen to yield radiations that penetrate deeply in a body part or region.

The invention also relates to the system according to the invention, wherein the at least two different source(s) of radiation preferentially emit at least two different radiations or beams of radiation, preferentially characterized by at least one property selected in the group consisting of:

i) At least two different beams of radiation preferentially have at least two different values of parameters selected in the group consisting of: orientations or directions of propagation or angles between their direction of propagation and the horizontal or vertical axis $(O_1, O_2)$, intensity $(I_1, I_2)$, power $(P_1, P_2)$, energy $(E_1, E_2)$, width $(W_1, W_2)$, depth of penetration $(D_1, D_2)$, frequency $(F_1, F_2)$, and wavelength $(W_1, W_2)$; and

ii) The at least two radiations are preferentially of at least two different types of radiations;

wherein the first beam preferentially has at least one parameter selected in the group consisting of: $O_1, I_1, P_1, E_1, W_1, D_1, F_1$ and $W_1$;
wherein the second beam preferentially has at least one parameter selected in the group consisting of:

$O_2, I_2, P_2, E_2, W_2, D_2, F_2$ and $W_2$;
wherein preferentially $(O_1-O_2)/(O_1+O_2)$, $(I_1-I_2)/(I_1+I_2)$, $(I_1-I_2)/I_1$, $(I_1-I_2)/I_2$, $(P_1-P_2)/(P_1+P_2)$, $(P_1-P_2)/P_1$, $(P_1-P_2)/P_2$, $(E_1-E_2)/(E_1+E_2)$, $(E_1-E_2)/E_1$, $(E_1-E_2)/E_2$, $(D_1-D_2)/(D_1+D_2)$, $(D_1-D_2)/D_1$, $(D_1-D_2)/D_2$, $(F_1-F_2)/(F_1+F_2)$, $(F_1-F_2)/F_1$, $(F_1-F_2)/F_2$, $(W_1-W_2)/(W_1+W_2)$, $(W_1-W_2)/W_1$, and/or $(W_1-W_2)/W_2$, in absolute value or not, is/are larger than 0, $10^{-5}$, $10^{-3}$, $10^{-1}$, 1, 5, 10, 50, 75, 100 or $10^4$ preferentially%,
wherein the at least one parameter is preferentially measured or exists in at least one region selected in the group consisting of:

i) At least one emission region,
ii) At least one interference region, and
iii) At least one region separating at least one emission region and at least one interference region.

In some cases, the frequency of the radiations preferentially emitted by at least one source of radiation or radiation emitting element can be larger than $10^{-5}$, $10^{-3}$, $10^{-1}$, 0, 1, 10, $10^3$ or $10^5$ MHz.

In some other cases, the frequency of the radiations preferentially emitted by the at least one source of radiation or radiation emitting element can be smaller than $10^5$, $10^3$, 10, 0, 1, $10^{-1}$, $10^{-3}$ or $10^{-5}$ MHz.

In still some other cases, the penetration depth of the radiations preferentially emitted by the at least one source of radiation or radiation emitting element in the body part or region can be larger than $10^{-10}$, $10^{-5}$, $10^{-1}$, 0, 1, 10 or $10^3$ cm.

In still some other cases, the penetration depth of the radiations preferentially emitted by the at least one source of radiation or radiation emitting element in the body part or region can be smaller than $10^{10}$, $10^5$, $10^3$, 10, 1, 0, $10^{-1}$, $10^{-3}$ or $10^{-5}$ cm.

In still some other cases, the intensity or power of the radiations preferentially emitted by the at least one source of radiation or radiation emitting element is smaller than $10^{10}$, $10^5$, $10^3$, 10, 5, 2, 1, 0, $10^{-1}$, $10^{-3}$ or $10^{-5}$ Watt preferentially per cm or $cm^2$ or $cm^3$ preferentially of radiation emitting element surface or emitting part or piezo element or length, surface or volume of body part or region.

In still some other cases, the intensity or power of the radiations preferentially emitted by the at least one source of radiation or radiation emitting element is larger than $10^{-10}$, $10^{-5}$, $10^{-3}$, $10^{-1}$, 0, 1, 5, 10, $10^3$ or $10^5$ Watt preferentially per cm or $cm^2$ or $cm^3$ preferentially of radiation emitting element surface or emitting part or piezo element or length, surface or volume of body part or region.

In another embodiment of the invention, the imaging radiation preferentially emitted by the emitting element or imaging radiations serve(s) to detect or identify or delineate the region where the therapeutic or heating radiations should be applied or interference region or body part or region to be treated. In some cases, another imaging method such as a non-radiation or non-echo-graphic imaging method, magnetic resonance imaging, scanner, or computing tomography is used to image the region where the radiations, the therapeutic or heating radiations should be applied or interference region or body part or region to be treated. In some other cases, the imaging method and other imaging method are combined, for example by first identifying and/or locating the region where the radiations, the therapeutic or heating radiations should be applied or interference region or body part or region to be treated and then by inserting or transferring the elements of identification and/or location of such region in the imaging method, in such a way that the imaging method can then preferentially guide the probe or system or part of the system so that the radiations, therapeutic or heating radiations are applied in the region where they should be applied or interference region or body part or region to be treated.

In another embodiment of the invention, the probe or system or part of the system is a transrectal probe or probe of the body part or region or system or part of the system, preferentially a transrectal radiation probe or radiation probe of a body part or region or system or part of the system, *i.e.* preferentially a probe or system or part of the system that is inserted or located inside or at the surface of the rectum or body part or region or organ partly or fully. In some cases, the probe or system or part of the system or at least one element of the probe or system or part of the system can be in contact with the rectum or body part or region or organ or part of the rectum or body part or region or organ such as the rectal wall or wall or surface of the body part or region or organ.

In some cases, the probe or system or part of the system, preferentially the transrectal probe or or probe of the body part or region or system or part of the system, does not heat the rectal wall or wall or surface of the body part or region or the body part or region, preferentially by more than $10^{-1}$, 0, 1, 5, 10, $10^2$ or $10^3$ °C, preferentially above the physiological temperature or above 25 °C, 36 °C or 37 °C.

In another embodiment of the invention, the probe or system or part of the system comprises at least 1 radiation source or radiation emitting element with at least one radiation emitting element parameter selected in the group consisting of: i) radiation emitting element size, ii) radiation emitting element geometry, iii) radiation emitting element focusing or non-focusing ability, iv) radiation emitting element location, and v) radiation emitting element orientation.

In some cases, the radiation emitting element is a mono-element radiation emitting element, *i.e.* preferentially a radiation emitting element that emits radiations through or from a single surface, preferentially a radiation emitting element that emits only one radiation beam.

In some other cases, the radiation emitting element is a multi-element radiation emitting element, *i.e.* preferentially a radiation emitting element that emits through or from different or separated surfaces or at least 2, 3 or 4 surfaces, preferentially a radiation emitting element that emits at least 2, 3 or 4 radiation beams.

In another embodiment of the invention, the probe or system or part of the system comprises at least 2, 3, 4, 5, 6, 7, 8, 10, 15, 20, 50 or 100 radiation emitting elements.

In some cases, the at least two radiation emitting elements are separated from each other in such a way that the at least two radiations or radiation beam(s) can be emitted from different or at least two different locations or that the at least two radiations that they emit can be separated, for example by measuring that there is no or very attenuated radiation, or radiation of intensity smaller than $10^5$, $10^3$, 100, 10, 5, 2, 1, $10^{-3}$ or $10^{-5}$ Hz or W preferentially per cm or mm, preferentially in at least one region separating the at least two radiations or radiation emitting elements.

In some other cases, the number of source(s) of radiation or emitting elements is increased preferentially to generate radiations from an increasing number of different locations or different radiation emitting element surfaces or emitting part or piezo elements.

In still some other cases, at least one parameter of source of radiation or radiation emitting element parameter is adjusted or chosen in such a way that the radiations generated by the at least two radiation sources or radiation emitting elements interfere, preferentially constructively, where such interference preferentially results in or is associated with or consists in or is the addition in part or in full of the temperature, pressure, intensity, power or acoustic power generated by each source of radiation or radiation emitting element taken individually, where such interference preferentially occurs or is located in the body part or region preferentially to be treated or pathological site, where such interference preferentially does not occur or is not located in the body part or region outside the body part or region to be treated or healthy site or leads to a higher temperature, pressure, intensity, power or acoustic power preferentially of or generated by the radiation preferentially in at least one region of the body part or region to be treated than in at least one region outside the body part or region to be treated.

In still some other cases, the at least two sources of radiations or radiation emitting elements generate at least two radiation beams that interfere in an interference region characterized by a temperature, pressure, intensity, power or acoustic power or homogeneity of at least one of these parameters preferentially of or generated by the radiation, which is increased when the number of sources of radiation or radiation emitting elements or radiation beams originating from at least two different radiation sources or radiation emitting elements is increased.

In one embodiment of the invention, the probe or system or part of the system, preferentially the transrectal probe or probe of the body part or region or system or part of the system, comprises at least one of the following element or part:

1) A first part consisting in at least one source of radiation or heating radiation emitting element;
2) A second part consisting in at least one other source of radiation or an imaging radiation emitting element or imaging probe or system or part of the system or echograph;
3) A third part consisting of or comprising a cooling part, cooling balloon and/or transmitting material;
4) A fourth part consisting of a support to which or with which or on top of which the first, second, and/or third part(s) is/are attached or bound or linked or assembled.

In some cases, the fourth part is a part that contains the connections, e.g. solid or electronic ones, preferentially between at least two different parts of the system such as the first and/or second part(s) and/or the equipment generating the acoustic signal and/or the power supply.

In some cases, at least one part of the system such as the fourth part has at least one property selected in the group consisting of:

> 1) It is elongated, *i.e.* it has a length larger than a width or thickness;
> 2) It can be inserted in the rectum or body part or region or can be positioned at the surface or in or above or inside or below or outside the body part or region preferentially to be treated; and
> 3) It is or serves as a support or connector to or with which at least one or two different part(s) of the system, such as the first and/or second part is/are attached;

In one embodiment of the invention, the angle between surface or emitting part or piezo element of the source of radiation or radiation emitting element and the horizontal or vertical axis or at least one axis of the body part or region to be treated or the axis of elongation of the rectum or body part or region preferentially to be treated or the axis of elongation of the at least one part of the system or fourth part or support of the probe or system or part of the system preferentially on two which the at least one source of radiation or radiation emitting element is preferentially attached, designated as angle a.

In some cases, a is larger than 0, 1, 5, 10, 30, 45, 50, 70, 90, 120, 150, 180, 200, 250, 300, 350, or 380 °.

Preferentially, the surface or emitting part or piezo element of the source of radiation or radiation emitting element is the surface or emitting part or piezo element that emits the radiations or from which or through which radiations are emitted.

Preferentially, the surface or emitting part or piezo element of the source of radiation or radiation emitting element is the external surface or the exterior of the emitting part or piezo element of the source of radiation or radiation emitting element or the surface that is exposed to or in contact with at least one part of the system or the fourth part of the probe or system or part of the system or the balloon or the inner part of the balloon, preferentially water.

In another embodiment, the angle a is smaller than 380, 350, 300, 250, 200, 180, 150, 120, 90, 70, 50, 45, 30, 10, 5, 1 or 0 °.

In still another embodiment, the angle a is comprised between 0 and 360 °, between 0 and 180 °, between 0 and 90 °, between 0 and 45 °, between 1 and 50 °, between 1 and 90°C, between 10 and 80 °, more preferably between 20 ° and 80°.

In still another embodiment of the invention, the at least one source of radiation or radiation emitting element, preferentially designated by source of radiation or radiation emitting element i or $T_i$, has a surface, which makes an angle $a_i$ between the radiation emitting element surface of $T_i$ and the horizontal or vertical axis or at least one axis of the body part or region to be treated or the axis of elongation of the rectum or body part or region preferentially to be treated or the axis of elongation of the at least one part of the system or fourth part or support of the probe or system or part of the system preferentially on two which the at least one source of radiation or radiation emitting element is preferentially attached.. In one embodiment of the invention, the at least one source of radiation or radiation emitting element $T_i$ is emitting or producing or associated with the emission of at least one radiation beam $B_i$. In some cases, $B_i$ has a at least one property selected in the group consisting of: i) it has a width that is preferentially smaller than the width or diameter of the source of radiation or radiation emitting element $T_i$ or of its surface, preferentially at a given time during its propagation, preferentially when it is in the ii) it has a the direction of propagation perpendicular or inclined by less than 90, 45, 30, 10, 5 or 1 °c from the axis perpendicular to surface of the source of radiation or radiation emitting element or emitting part or piezo element, and iii) it has in some cases at least one frequency, pressure, power, intensity, acoustic or not, that is/are preferentially such or is/are preferentially sufficiently low, preferentially lower than $10^{20}$, $10^{10}$, $10^5$, $10^3$, 100, 10, 5, 2, 1, $10^{-3}$ MHz, KHz, H, W, $W/cm^2$ or Pa, that it/they do(es) not result in the production of heat or nanoparticle movement or cavitation preferentially inertial, or that it/they result(s) in or produce(s) a temperature increase or nanoparticle movement of magnitude smaller than 0.1, 1, 5, 10, 100, $10^4$ or $10^5$ °C or cm, iv) it has in some other cases at least one frequency, pressure, power, and intensity, acoustic or not, that is/are preferentially such or is/are preferentially sufficiently large, preferentially larger than $10^{-20}$, $10^{-10}$, $10^{-5}$, $10^{-3}$, 0.1, 1, 10 or 100 MHz, KHz, H, W, $W/cm^2$ or Pa, that it/they do(es) result(s) in the production of heat or nanoparticle movement or cavitation preferentially inertial, or that it/they result(s) in or produce(s) a temperature increase or nanoparticle movement of magnitude larger than 0.1, 1, 5, 10, 100, $10^4$ or $10^5$ °C or cm.

In another embodiment of the invention, at least two sources of radiation or radiation emitting elements $T_i$ and $T_j$ ($i \neq j$) emit or produce or are associated with the emission of at least two radiation beams $B_i$ and $B_j$ ($i \neq j$), wherein $B_i$ and $B_j$ have or are associated with at least one property selected in the group consisting of:

> 1) at least one frequency, pressure, power, intensity, acoustic or not, of the radiations or $B_i$ and/or $B_j$ is lower than a threshold value above which hyperthermia occurs , preferentially lower than $10^{20}$, $10^{10}$, $10^5$, $10^3$, 100, 10, 5, 2, 1, $10^{-3}$ MHz, KHz, H, W, $W/cm^2$ or Pa, preferentially in a first region where $B_i$ and $B_j$ do not interfere or don't propagate jointly or don't meet or cross each other, preferentially at least one region outside the interference region or body part or region to be treated or the emission region partly or fully, preferentially in a region where hyperthermia does not occur;

2) at least one frequency, pressure, power, intensity, acoustic or not, of the radiations or $B_i$ and/or $B_j$ larger than the threshold above which hyperthermia occurs, preferentially larger than $10^{-20}$, $10^{-10}$, $10^{-5}$, $10^{-3}$, 0.1, 1, 10 or 100 MHz, KHz, H, W, W/cm$^2$ or Pa, preferentially in the region where $B_i$ and $B_j$ interfere or propagate jointly or meet or cross each other, preferentially the interference region or body part or region to be treated or region outside the emission region partly or fully, preferentially in a region where hyperthermia occurs.

In another embodiment of the invention, the at least one parameter of $T_i$ and/or $T_j$, such as the angle $a_i$ and/or $a_j$, the size and/or geometry of the source of radiation or radiation emitting element $T_i$ and/or $T_j$, the focusing or un-focusing property of the source of radiation or radiation emitting element $T_i$ and/or $T_j$, is/are chosen or selected so that:

i) the beams $B_i$ and $B_j$ meet or cross each other or interfere in the body part or region to be treated or in the region that need to be heated or in the interference region or in the at least one pathological site or in the tumor; and/or
ii) the beams $B_i$ and $B_j$ do not meet or do not cross each other or don't interfere outside the body part or region to be treated or region that needs to be heated or outside the interference region or at least one pathological site or outside the tumor;

In still another embodiment of the invention, the number of different sources of radiation or radiation emitting elements $T_i$ is increased preferentially to achieve at least one of the following effects:

i) increase the number of different locations or inclinations from which or at which radiation beams are emitted;
ii) reduce the at least one frequency, pressure, power, intensity, acoustic or not, of the radiation(s), preferentially associated with each individual acoustic beam $B_i$, preferentially to prevent the generation of heat by each individual acoustic beam $B_i$ preferentially outside of the interference region or body part or region to be treated or emission region or outside of the region that needs to be heated or outside of at least one pathological site;
iii) increase the at least one frequency, pressure, power, intensity, acoustic or not, of the radiation(s), preferentially generated by the different beams $B_i$ in the interference region or body part or region to be treated or outside the emission region or in the region that needs to be heated or at least one pathological site.

In still another embodiment of the invention, the at least two different parts of the systems or two sources of radiations or radiation emitting elements $T_i$ and $T_j$, which are preferentially attached or bound to or in contact with at least one part of the system such as the support (part 4 of the probe or system or part of the system), are separated by a distance $D_1$, where $D_1$ is preferentially a distance with at least one property selected in the group consisted of: i) $D_1$ is the distance that separates the bottom or central or given or top part of at least one part of the system or $T_i$ from the bottom or central or given or top part of another part of the system or $T_j$, ii) $D_1$ is the distance that separates at least one part of the system or the part of $T_i$ in contact with the support (part 4) of the probe or system or part of the system from at least another part of the system or the part of $T_j$ in contact with the support (part 4) of the probe or system or part of the system, and iii) $D_1$ is the distance that separates the center of at least one part of the system or $T_i$ or of the external surface of at least one part of the system or $T_i$ from the center of at least another part of the system $T_j$ or of the external surface of at least another part of the system or $T_j$. In one embodiment of the invention, $D_1$ is smaller than 100, 50, 20, 10, 5, 2, 1, or 0.1 cm. In some cases, $D_1$ is small enough to maintain the at least two parts of the system or radiation sources or emitting elements sufficiently close from each other, preferentially to enable the at least two radiation beams generated by the at least two radiation emitting elements to meet or interfere, preferentially within the interference region or body part or region to be treated or pathological site.

In one embodiment of the invention, $D_1$ is larger than 0.1, 1, 5, 10, 20, 50 or 100 cm. In some cases, $D_1$ is large enough to enable inserting between at least two parts of the system or radiation sources or radiation emitting elements an imaging probe or system or part of the system such as an echograph or radiation imaging apparatus, where the imaging probe or system or part of the system preferentially serves to detect the position or temperature or physiological parameter or temperature of the body part or region to be treated and/or to position the system or part of it in such a way that the at least two beams of radiation cross the body part or region to be treated, where the transrectal probe or system or part of the system is preferentially or should be positioned to heat the pathological site or body part or region to be treated with the at least two sources of radiations or radiation emitting elements.

In still another embodiment of the invention, $D_1$ is comprised between 0.1 and 100 cm, 1 and 100 cm, 1 and 50 cm, 1 and 20 cm, or 1 and 5 cm.

In still another embodiment of the invention, the distance $D_2$ is the distance between:

i) at least one location of the support or one first part of the system, preferentially selected among: 1) a given location in the support or one first part of the system, 2) a given location along the elongation axis of the support or one first part of the system, 3) a given location on the external surface of the support or one first part of the system, 4) a given location on or in the part of the support or one first part of the system, which is preferentially the part that is not bound to or attached to or in contact with or does not comprise $T_i$ and/or $T_j$ and/or the balloon and/or the transmitting material and/or the first part and/or the second part and/or the third part of the probe or system or part of the system, and 4) the location in the support or one first part of the system that is the closest from the region that needs to be heated or pathological site or interference region or body part or region to be treated,
and

ii) at least one location of pathological site or body part or region to be treated or interference region, preferentially selected among: 1) the bottom of the pathological site or body part or region to be treated or interference region or the location in the pathological site or body part or region to be treated or interference region that is the closest from the transrectal probe or system or part of the system or at least one part of the support, and 2) the central part or center or middle of the pathological site or body part or region to be treated or interference region,

In still another embodiment of the invention, the distance $D_2$ is larger than 0, 0.1, 1, 2, 3, 4, 5, 10, 20, 50 or 100 cm.

In some cases, the distance $D_2$ is kept sufficiently large to prevent heating at least one of the following locations, or heating at least one of the following locations is avoided or prevented or minimized, preferentially by more than 0, $10^{-3}$, 1, 5, 10, $10^2$ or $10^3$ °C, preferentially above physiological temperature or above the temperature of the body part or region before or without the application of the radiations, wherein the following locations are selected in the group consisting of i) a part or the whole of the external surface of the body part or region of an individual, ii) the rectal wall, iii) the part of the body part or region of the individual, which is located between the probe or system or part of the system or at least one part of the probe or system or part of the system and the pathological site or region that needs to be heated or body part or region to be treated or interference region, iv) at least one part of the body part or region that is located at a distance or depth from the probe or system or part of the system or at least one part of the probe or system or part of the system and the pathological site or region that needs to be heated or body part or region to be treated or interference region that is smaller 10000, 100, 50, 20, 10, 5, 2, 1, or 0.1 cm, and v) at least one other part of the body part or region that is located at a distance or depth from the probe or system or part of the system or at least one part of the probe or system or part of the system and the pathological site or region that needs to be heated or body part or region to be treated or interference region that is larger than 0, 0.1, 0.5, 1, 2, 5, 10 or 50 cm.

In the case where the probe or system or part of the system is a transrectal probe or system or part of the system and the body part or region to be treated is a pathological site such as a solid or prostate tumor, the distance $D_2$ is preferentially larger or equal than the thickness of the skin or organ or vein or artery or tissue or healthy site preferentially its/their surface or rectal wall of preferentially 0.01 to 100 or $10^5$ mm or 6 to 8 mm, preferentially to prevent heating the skin, organ or vein or artery or tissue or healthy site preferentially its/their surface or rectal wall that can be heat sensitive.

In the case where the probe or system or part of the system is applied directly or in direct contact or indirectly or in indirect contact or without contact on top or on or at the surface of or above or below the skin or organ or vein or artery or tissue or healthy site of an individual and the pathological site is located above or below the skin or organ or vein or artery or tissue or healthy site of the individual, the distance $D_2$ can be larger than or equal to the thickness or diameter or depth of the skin or organ or vein or artery or tissue or healthy site that preferentially separates the probe or system or part of the system or at least on part of the probe or system or part of the system from the pathological site or body part or region to be treated or interference region or at least one location that it/they comprise.

In the case where the probe or system or part of the system is applied directly or in direct contact or indirectly or in indirect contact or without contact on top or on or at the surface of or above or below a body part or region of an individual that separates the probe or system or part of the system from the pathological site or body part or region to be treated or interference region, also designated by separating body part or region, the distance D2 can be larger or equal than the thickness, width, length, diameter of the separating body part or region.

In another embodiment of the invention, the probe or system or part of the system heats the heating area or the region that needs to be heated or body part or region to be treated or interference region.

In some cases, i or j is equal or larger than 0, 1, 2, 3, 4, 5, 6, 7, 8, 9, 10, 12, 15, 20, 30, 40, 50, 60, 75, 80, 90, 100, 150, 200, or 500.

In some other cases, i or j is equal or smaller than 500, 200, 150, 100, 90, 80, 75, 60, 50, 40, 30, 20, 15, 12, 10, 9, 8, 7, 6, 5, 4, 2, 1, or 0.

In one embodiment of the invention, the source of radiation or radiation emitting element is or comprises an element emitting ultrasonic or acoustic or electromagnetic or ionizing or particle or thermal radiation or a radiation emitting element that converts or transforms a current or an alternating current, preferentially alimenting the probe or system or part of the system, into radiations. It can be a heating or an imaging radiation emitting element.

In another embodiment of the invention, the source of radiation or radiation emitting element is or comprises an element emitting ultrasonic or acoustic or electromagnetic or ionizing or particle or thermal radiation or a radiation emitting element that converts radiations into a current or an alternating current. It can be a heating or an imaging radiation emitting element.

In another embodiment of the invention, the source of radiation or radiation emitting element, preferentially its surface, is the surface from which the radiations are emitted, preferentially after or following alimentation of the probe or system or part of the system preferentially with currents or after or following the conversion of the current, preferentially alternating current, into a radiation or radiation signal.

In another embodiment of the invention, the radiation emitting element surface is the surface receiving the radiations, preferentially followed by the conversion of the radiations into currents or alternating currents.

In one embodiment of the invention, the axis of emission of the radiation beam is the axis or direction, preferentially crossing or travelling through the body part or region preferentially to be treated or interference region or at least one direction, surface or volume of the body part or region preferentially to be treated or interference region, preferentially of or with maximum or larger intensity, power, frequency of the radiations, preferentially compared with the intensity, power, frequency of the radiations outside the body part or region to be treated or interference region.

The invention relates to the probe or system or part of the system according to the invention, wherein preferentially a radiation transmission material such as water or gel or metal or organic material or gas or air or liquid or solid, which preferentially transmits radiations, is preferentially located between the source of radiation or radiation emitting element or its surface and the surface preferentially external of a body part or region or individual or rectum or skin or organ or vein or artery or tissue or healthy site, wherein the radiation transmission material is preferentially a cooling material or cooling part,

wherein the cooling material or cooling part has preferentially at least one property selected in the group consisting of:

1) It is cooled down by a cooling material such as water or gel or liquid or solid or gas preferentially comprised in a balloon or embedding material;
2) It is located above or below or at the surface of the radiation sources or the at least two radiation emitting elements and/or in-between the at least two radiation emitting elements and below the rectal wall or rectum or skin or organ or vein or artery or tissue or healthy site or body part or region or surface of the individual;
3) It is or is maintained at a temperature that is lower by at least $10^{-5}$, $10^{-1}$, 1, 5, or 10 °C preferentially than the temperature of at least one location selected in the group consisting of: 1) the heated or interference region or body part or region to be treated, 2) the surface of the source of radiation or radiation emitting element, and 3) the surface of the rectum or skin or organ or vein or artery or tissue or healthy site or body part or region or individual or rectum,

4) It enables or does not prevent the propagation of radiations, preferentially between the at least two sources of radiation or radiation emitting elements and the rectal wall or the surface of the skin or organ or vein or artery or tissue or healthy site or the body part or region or individual or rectum; and

5) It does not contain gazes or air, or it contains less than 1, 5, 10 or 50% in volume or mass of gazes or air.

The invention also relates to the system according to the invention, wherein the at least two different source(s) of radiation preferentially emit at least two different radiations or beams of radiation, preferentially characterized by at least one property selected in the group consisting of:

i) At least two different beams of radiation have at least two similar or same values of parameters preferentially selected in the group consisting of: orientations or directions of propagation or angles between their direction of propagation and the horizontal or vertical axis ($O_1$, $O_2$), intensity ($I_1$, $I_2$), power ($P_1$, $P_2$), energy ($E_1$, $E_2$), width ($W_1$, $W_2$), depth of penetration ($D_1$, $D_2$), frequency ($F_1$, $F_2$), and wavelength ($W_1$, $W_2$); and

ii) The at least two radiations are of the same or similar type of radiations;

wherein preferentially the first beam as at least one parameter selected in the group consisting of: $O_1$, $I_1$, $P_1$, $E_1$, $W_1$, $D_1$, $F_1$ and $W_1$;
wherein preferentially the second beam as at least one parameter selected in the group consisting of: $O_2$, $I_2$, $P_2$, $E_2$, $W_2$, $D_2$, $F_2$ and $W_2$;
wherein preferentially $(O_1-O_2)/(O_1+O_2)$, $(I_1-I_2)/(I_1+I_2)$, $(I_1-I_2)/I_1$, $(I_1-I_2)/I_2$, $(P_1-P_2)/(P_1+P_2)$, $(P_1-P_2)/P_1$, $(P_1-P_2)/P_2$, $(E_1-E_2)/(E_1+E_2)$, $(E_1-E_2)/E_1$, $(E_1-E_2)/E_2$, $(D_1-D_2)/(D_1+D_2)$, $(D_1-D_2)/D_1$, $(D_1-D_2)/D_2$, $(F_1-F_2)/(F_1+F_2)$, $(F_1-F_2)/F_1$, $(F_1-F_2)/F_2$, $(W_1-W_2)/(W_1+W_2)$, $(W_1-W_2)/W_1$, and/or $(W_1-W_2)/W_2$, in absolute value or not, is/are smaller than $10^{20}$, $10^{10}$, $10^5$, $10^3$, 100, 75, 50, 10, 5 or 1 preferentially %, wherein preferentially the at least one parameter is preferentially measured or exists in at least one region selected in the group consisting of:

i) At least one emission region,
ii) At least one interference region, and
iii) At least one region separating at least one emission region and at least one interference region.

The invention also relates to the system according to the invention, preferentially comprising a third part, which is preferentially an imaging element or equipment that preferentially has at least one property selected in the group consisting of:

i) it preferentially is or comprises a scanner or part of it;

ii) it preferentially is or comprises a MRI or part of it;

iii) it preferentially comprises at least one imaging element, which is preferentially located in the region that preferentially receives or absorbs or is exposed to at least one radiation or beam of radiation, which is preferentially emitted or reflected or diffracted partly or fully by the at least one nanoparticle or body part or region or interference region or part of it;

iv) it preferentially comprises at least two imaging elements, which are located in two different regions that receive or absorb or are exposed to at least two different radiations or two different beams of radiation, which are preferentially emitted or reflected or diffracted partly or fully by at least two different nanoparticles or two different sides or facets of at least one nanoparticle or two different portions of the body part or region or two different portions of the interference region;

v) it preferentially receives or absorbs or is exposed to at least one radiation or one beam of radiation,

vi) it preferentially detects or has sufficient sensitivity or has a sufficiently large number or more than one sensor or transducer or piezo element or piezo element, preferentially located in different regions, to detect the difference in at least one value of at least one parameter between the at least two beams of radiation, which are preferentially emitted or reflected or diffracted partly or fully by at least two different nanoparticles or two different sides or facets of at least one nanoparticle or two different portions of the body part or region or two different portions of the interference region;

vii) it is preferentially comprised between or in a different region of the at least two emitting elements of the first part;

viii) it preferentially serves to detect or has sufficient imaging sensitivity or contrasting strength or ability to detect at least one entity, partly or fully, selected in the group consisting of: i) at least one nanoparticle of the second part, ii) at least one body part or region, iii) at least one interference region, iv) at least one emitting region, and v) at least one region located between the emitting and interference or common regions;

ix) it preferentially has an imaging sensitivity or imaging strength that is increased or enhanced by the presence of the at least one nanoparticle of the second part;

x) it preferentially serves to detect or has sufficient imaging sensitivity or contrasting strength or ability to detect the at least one beam emitted by the at least one source of radiation or the at least two different beams emitted by the at least two different beams of radiation, preferentially through the detection.

The invention also relates to the system according to the invention, wherein the interference or common region and/or body part or region to be treated comprise(s) more pathological cells than healthy cells or at least one pathological site that is larger than at least one healthy site.

The invention also relates to the system according to the invention, wherein the body part or region designates or comprises at least one surface, volume, compartment, segment, dimension, size of a body part or region or a cell, organelle, biological material, protein, enzyme, DNA, RNA, in part or fully, healthy or pathological.

The invention also relates to the system according to the invention, wherein the interference region is preferentially a heated region that is preferentially heated at a temperature that preferentially has at least one property selected in the group consisting of:

i) it is preferentially larger, preferentially by at least 0.1 °C, than the temperature of the individual or than the physiological temperature or than 36.5 or 37.5 °C,

ii) it is preferentially lower than the temperature of thermo-ablation or 1000 or 100 or 80 or 60°C.

The invention also relates to the system according to the invention, which is preferentially configured so that preferentially at least two different radiation beams are preferentially emitted in at least one of the following manners:

i) Optionally, during a period of time $t_1$, at least one beam of at least one part of the system preferentially the third part is applied or emitted from at least one part of the system preferentially the third part and this beam is then preferentially reflected back or returned to the third part, preferentially to obtain an image of the interference region, preferentially by imaging at least one nanoparticle of the second part preferentially comprised in the interference region or body part or region to be treated;

ii) Optionally, during a period of time $t_2$, a least one first beam of at least one part of the system preferentially the first part is applied, preferentially to trigger a disturbance in the interference region with minimal production of heat;

iii) During a period of time $t_3$, at least one first beam and at least one second beam of the second part are applied, preferentially to heat the interference region, preferentially without heating the region outside the interference region or by producing a larger amount of heat in the interference region than in the region outside of the interference region, preferentially by more than $10^{-10}$, $10^{-3}$, $10^{-1}$, 0, 1 or 5 °C;

The invention also relates to the system according to the invention, wherein the interference region or body part or region to be treated is preferentially located at more than $10^{-5}$, $10^{-3}$, $10^{-1}$, 0, 1, 5 or 10 cm of at least one source of radiation or part of it or the emission region or from the surface, volume or part of it/them.

The invention also relates to the system according to the invention, which is not an apparatus generating high intensity focused ultrasound.

The invention also relates to the system according to the invention, wherein the radiation is selected from group consisting of: i) wave(s) such as electromagnetic, sound, acoustic or particle wave(s), ii) electromagnetic radiation, iii) acoustic radiation forces, iv) radiation forces, v) radiation pressures, vi) irradiation, vii) a magnetic or electric field, viii) laser light, ix) light produced by a lamp, x) light emitted at a single wavelength, xi) light emitted at multiple wavelengths, xii) a ionizing radiation, xiii) microwave, xiv) radiofrequencies, xv) alpha particles or radiation, xvi) beta particles or radiation, xvii) gamma particles or radiation, xviii) X-ray particles or radiation, xix) neutron particles or radiation, xx) proton particles or radiation, xxi) electron particles or radiation, xxii) ion particles or radiation, xiii) neutrino particles or radiation, xiv) muon particles or radiation, xv) meson particles or radiation, xvi) photon particles or radiation, xvii) infra-sounds, xviii) sounds, xix) ultra-sounds, xx) hyper sounds, xxi) heating radiation, xxii) laser, xxiii) alternating magnetic field, xxiv) uniform magnetic field, and xxv) magnetic field with a gradient.

The invention also relates to the system according to the invention, wherein the first part preferentially emits n beams of radiation, which are preferentially unfocused,

wherein the intensity of the at least one beam of radiation or ultrasound emitted by the at least one emitting element of the first part is preferentially:

- Preferentially $i_j$ in an emission region (j) or at the surface of a emitting element (j) or transducer or piezo element (j), wherein $1 < j < n$; and/or

- Preferentially $i_j/b_j$ in the interference region in the absence of emission of at least one other beam of radiation or ultrasound different from (j), preferentially due to the attenuation of the radiation (j) or beam of radiation (j) between the emission region (j) or surface of emitting element or transducer or piezo element (j) and interference region, wherein preferentially $b_j$ is an attenuation coefficient of the at least one beam of radiation

(j), wherein preferentially $b_j > 1$ or $1 < b_j < 2$;

wherein the intensity of the n beams of radiation or ultrasound emitted by the n emitting elements of the first part, which interfere partly or fully in the interference region, is:

- $$i = \sum_{j=1}^{j=n} i_j$$ where i is the sum of n beam

intensities $i_j$ at emission regions or at the surface of the different emitting elements or transducer or piezo elements as resulting from the emission of radiation from n emission regions or at the surfaces of n emitting elements or transducer or piezo elements;

- larger than or equal to $i_j/b_j$ or $i_j$ in the interference region;
and/or

- smaller than or equal to $$i = \sum_{j=1}^{j=n} i_j$$ in the interference region,
wherein n is preferentially larger than 0, 1, 2, 3, 4, 5, 10 or 100.

The invention also relates to the system according to the invention, wherein the first parts preferentially emits m beams of radiation, which are preferentially focused,

wherein the intensity of the at least one focalized beam of radiation or ultrasound emitted by the at least one emitting element of the first part is:

- $i_k$ in an emission region (k) or at the surface of a emitting element (k) or transducer or piezo element (k), wherein $1 < k < m$;
and/or

- $f_k \cdot i_k$ in the interference region in the absence of emission of at least one other beam of radiation or ultrasound different from (k), preferentially due to the amplification or increase or focalization or narrowing of the radiation (k) or beam of radiation (k) between the emission region (k) or surface of emitting element or transducer or piezo element (k) and interference region, wherein preferentially $f_k$ is the coefficient of amplification or increase or focalization or narrowing of the radiation or radiation beam intensity or width or frequency (k), wherein preferentially $f_k > 1$;

wherein the intensity of the m radiations or beams of radiations preferentially emitted by m emitting elements of the first part, which preferentially interfere partly or fully in the interference region, is:

i) $i = \sum_{k=1}^{k=m} i_k$ where i is the sum of the different emission regions or at the surface of the different emitting elements or transducer or piezo elements as resulting from the emission of radiation from m emission regions or at the surfaces of m emitting elements or transducer or piezo elements;

ii) larger than or equal to $i_k$ or $i = \sum_{k=1}^{k=m} i_k$ in the interference region; and/or

iii) smaller than or equal to $i = \sum_{k=1}^{k=m} f_k \cdot i_k$ in the interference region,

wherein m is preferentially larger than 0, 1, 2, 3, 4, 5, 10 or 100.

The invention also relates to the system according to the invention, wherein the first and third parts or at least a portion of them are assembled or associated or arranged in at least one of the following manners:

- The third part is preferentially embedded inside the first part or inside a portion of the first part;
- The first and third parts are preferentially detached or separated from each other or used separately one from another, preferentially before or after at least one treatment of the body part or region;
- The first and third parts are preferentially attached to each other or associated together or assembled together, preferentially during at least one treatment of the body part or region;
- The first and third parts are preferentially located on the same side relatively to the body part or region;
- The first and third parts are preferentially located on the opposite side relatively to the body part or region; wherein the side relatively to the body part or region is selected among: the right side of the body part or region, left side of the body part or region, the side above the body part or region, and the side below the body part or region;

The invention also relates to the system according to the invention, wherein at least one part of the system, preferentially the first part, at least one emitting element and/or at least one nanoparticle of the second part, is a single use or disposable equipment.

The invention also relates to the system according to the invention, wherein at least one part of the system, preferentially the third part, is a permanent equipment or is not a single use or disposable equipment.

The invention also relates to the system according to the invention, wherein a material transmitting radiation or ultrasound, such as water, air or body part or region without at least one nanoparticle, which preferentially transmits ultrasounds or at least one radiation, preferentially without interacting with, being absorbed by, being reflected by, heating or exciting at least one nanoparticle, is located between the at least one emitting element such as transducer or piezo element or transducer or piezo element surface and at least one nanoparticle, body part or region, interference region, individual, and/or rectum, and/or surface of it or them,

wherein the radiation or ultrasound transmission material is preferentially a cooling material or cooling part or material that transmits material without reducing or leading to an intensity of the radiation or ultrasound to 0 or a vanishing value, preferentially in the interference region, or a material that comprises well aligned or ordered or interacting atoms or molecules preferentially in sufficiently large number or without a too large separation between them to enable the transmission of a first type of radiation such as ultrasound or acoustic wave that preferentially rely on the presence of such structure for its transmission, or a material that comprises a low or no atoms or less than $10^{10}$, $10^5$, $10^3$, 10, 5, 2, or 1 atom(s) or ion(s) or molecule(s) per $m^3$, $cm^3$, $\mu m^3$ or $nm^3$, preferentially to enable the transmission of a second type of radiation such as electromagnetic waves that preferentially rely on the presence of such structure or relatively empty space to enable its transmission, preferentially by avoiding that such radiation is absorbed and therefore weakened by the transmission material;

wherein the transmission material or cooling part preferentially has at least one property selected in the group consisting of:

i) It is preferentially cooled down water or transmission material preferentially comprised in a balloon or an embedding material;

ii) It is preferentially located above or below or through or on the side of at least one or two emitting element(s) or transducer or piezo element(s) and/or in-between at least two emitting elements or transducer or piezo elements and/or above or below or through or on the side of the rectal wall or body part or region or interference region or surface of the individual;

iii) Preferentially It is or is maintained at a temperature that is lower by at least $10^{-5}$, $10^{-1}$, 1, 5, or 10 °C than the temperature of at least one location selected in the group consisting of: 1) the heated or interference region, 2) the emitting region or surface of the transducer or piezo element, and 3) the surface of the body part or region or individual or rectum,

iv) Preferentially, it enables or does not prevent the propagation of radiation or ultrasounds, preferentially between the at least two emitting elements or transducer or piezo elements and the rectal wall or the surface of the body part or region or individual or rectum or interference region;

v) Preferentially, it does not contain gazes or air, or it contains less than 1, 5, 10 or 50% in volume or mass of gazes or air preferentially when the first type of radiation is used; and

vi) Preferentially, it contains gazes or air, or it contains more than $10^{-20}$, $10^{-10}$, $10^{-5}$, $10^{-1}$, 0, 1, 5, 10 or 50% in volume or mass of gazes or air preferentially when the second type of radiation is used;

The invention also relates to the system according to the invention, where at least one radiation or ultrasound beam emitted by at least one emitting element or transducer or piezo element has preferentially at least one property selected in the group consisting of:

i) it is preferentially orientated towards or in the direction of the interference region or body part or region to be treated, *i.e.* that preferentially the axis or direction of the beam crosses the interference region or body part or region to be treated;

ii) it preferentially has a width or length or diameter or surface or volume or size that does not differ by more than 100% or 1 nm or $\mu$m, or mm, or cm or meter than the width or length or diameter or surface or volume or size of the at least one emitting element or transducer or piezo element or body part or region to be treated,

iii) it preferentially has an intensity lower than $10^{10}$, $10^{5}$, 100, 20, 10, 5, 2 or 1 W or Watt per cm or Watt per $cm^2$ or Watt per $cm^3$ of length, surface or volume of at least one emitting element or transducer or piezo element surface or body part or region to be treated, and

iv) it preferentially has a frequency that is larger than $10^{-10}$, $10^{-5}$, $10^{-3}$, 0, 1, 10, 100, or $10^3$ kHz, wherein preferentially the percentage in ii) is preferentially equal to $(S_{UB}-S_{BP})/S_{UB}$ or $(S_{UB}-S_{BP})/(S_{UB}+S_{BP})$, with $S_{UB}$ being the a width or length or diameter or surface or volume or size of the radiation or ultrasound beam and $S_{BP}$ being the width or length or diameter or surface or volume or size of the body part or region or interference region.

The invention also relates to the system according to the invention, which is a kit comprising the first and/or third part(s) and at least one nanoparticle of the second part.
The invention also relates to the system according to the invention, which is transrectal or at least one part of it is configured to be inserted inside an organism, partly or fully.
The invention also relates to the system according to the invention, wherein the system or at least one part of it is configured to be located outside an organism, partly or fully.

The invention also relates to the system according to the invention, which comprises at least two emitting elements or radiation sources, such as traducers, preferentially mono-elements, which are preferentially positioned in the following manner:

a. preferentially, the at least two emitting elements are attached to or comprise two different supports, which preferentially enable the orientation of the beam in the direction of the body part or region or interference region;
b. preferentially, the at least two emitting elements are connected to at least one power supply and/or energy or radiation converter, which preferentially enables the generation or production of the radiation, preferentially through the conversion or transformation of a current or voltage into either an acoustic or mechanical wave or into an electromagnetic wave or into a particle radiation;
c. preferentially, the at least two emitting elements transmit or emit at least one radiation;
d. preferentially, the angle between at least one first emitting element or at least one first beam emitted by at least one first emitting element and the perpendicular to the tangent of at least one first location of at least one surface of the body part or region or interference region is smaller than 180, 90, 45, 30, 10, 5, 2 or 1 °C;
e. preferentially, the angle between at least one second emitting element or at least one second beam emitted by at least one second emitting element and the perpendicular to the tangent of at least one second location of at least one surface of the body part or region or interference region is smaller than 180, 90, 45, 30, 10, 5, 2 or 1 °C;
f. preferentially, the distance between the first and second locations is larger than $10^{-5}$, $10^{-3}$, 1, 0, 5, 10 or 100 nm or cm, preferentially to avoid that the first and second beams fully interfere at the surface of the body part or region or interference region, preferentially to enable the first and second beams to fully interfere at the center of the body part or region or interference region, preferentially to result in a higher radiation intensity at the center of the body part or region or interference region than at its surface, preferentially by a factor of more than 0, 0.1, 1, 2, 5 or 10.

In one embodiment of the invention, the system is connected to a generator and/or amplifier to generate radiation or ultrasound or in a signal resulting in the production of a radiation.
The invention also relates to the system according to the invention, wherein the at least one nanoparticle of the second part emits at least one radiation, which is preferentially different, larger or smaller from the radiation emitted by the at least one emitting element, preferentially in terms of strength, intensity, energy, power, frequency, wavelength, and/or type.

The invention also relates to the system according to the invention, wherein the at least one emitting element or source of radiation has at least one dimension with at least one property selected in the group consisting of: i) it is comprised between 1 and 50000 mm, preferentially between 5 and 5000 mm, more preferably between 20 and 45 mm, ii) it is larger than $10^{-5}$, 0.5 or 1 cm, iii) it is lower than 100, 50, 20, 10, 5 or 2 cm, and ii) its diameter $D_T$ does not differ by more than 100% from the diameter of the body part or region to be treated or tumor or interference region $D_{BP}$,

> where this percentage is preferentially equal to $(D_T-D_{BP})/D_T$,
> where the dimension of the at least one emitting element is preferentially selected among the group consisting of: a surface, volume, width, a length, a diameter, a size and a thickness of the emitting element or source of radiation or transducer or piezo element.

The invention also relates to the system according to the invention, wherein the body part or region to be treated is a pathological site or a site that comprises at least one pathological cell, tumor cell, virus, or bacterium or at least one component, DNA, RNA, protein, enzyme of a pathological cell, tumor cell, virus, or bacterium.

The invention also relates to the system according to the invention, wherein the body part or region to be treated is preferentially a cancer or tumor selected from the group consisting of: the cancer of an organ, cancer of blood, cancer of a system of a living organism, adrenal cancer, anal cancer, bile duct cancer, bladder cancer, bone cancer, brain cancer, breast cancer, cervical cancer, colon/rectum cancer, endometrial cancer, esophagus cancer, eye cancer, gallbladder cancer, heart cancer, kidney cancer, laryngeal and hypopharyngeal cancer, leukemia, liver cancer, lung cancer, nasal cavity and paranasal sinus cancer, nasopharyngeal cancer, neuroblastoma, non-Hodgkin lymphoma, oral cavity and oropharyngeal cancer, osteosarcoma cancer, ovarian cancer, pancreatic cancer, pancreatic penile cancer, prostate cancer, retinoblastoma, rhabdomyosarcoma, salivary gland cancer, sarcoma, skin cancer, small intestine cancer, stomach cancer, testicular cancer, thymus cancer, thyroid cancer, uterine cancer, uterine sarcoma cancer, vaginal cancer, vulvar cancer, Walden Strom macroglobulinemia Wilms tumor, Castleman disease Ewing family of tumor, gastrointestinal carcinoid tumor, gastrointestinal stromal tumor, myelodysplastic syndrome pituitary tumor, and a cancerous disease such as gestational trophoblastic disease, Hodgkin disease, Kaposi sarcoma, malignant mesothelioma, and multiple myeloma.

The invention also relates to the system according to the invention, wherein preferentially the at least one or two nanoparticle(s) of the second part is or belongs to or is comprised, in the group of nanoparticles selected from: 1) a nanoparticle or particle or assembly of atoms, ions, or molecules of size larger than $10^{-5}$, $10^{-3}$, $10^{-1}$, 0 or 1 nm, 2) a nanoparticle or particle or assembly of atoms, ions, or molecules of size smaller than $10^5$, $10^3$, 100, 50, 20 or 10 nm, 3) a nanoparticle with a core, preferentially metallic or magnetic or crystallized, 4) a nanoparticle with a coating, preferentially surrounding the core partly or fully, preferentially binding or associating at least two nanoparticles together, 5) a nanosphere, 6) a crystallized nanoparticle, 7) an amorphous nanoparticle, 8) a magnetic nanoparticle, 9) a nanoparticle that is formed or assembled in the body part or region or individual, 10) a nanoparticle that is formed or assembled outside the body part or region or individual, 11) a nano-capsule, 12) a dendrimer, 13) a carbon nanotube, 14) a lipid/solid nanoparticle, 15) a lipid or protein or DNA or RNA based nanoparticle, 15) a nanoparticle with an inner aqueous environment surrounded by a layer, preferentially a stabilizing layer, most preferentially a phospholipid layer, 16) a multilayer nanoparticle, 17) a polymeric nanoparticle, 18) a quantum dot, 19) a metallic nanoparticle, 20) a polymeric micelle or nanoparticle, 21) a carbon based nano-structure, 22) a nanobubble, 23) a nanosome, 24) a pharmacyte, 25) a niosome, 26) a nanopore, 27) a microbivore, 28) a liposome, 29) a virus, preferentially recombinant, 30) a herbal nanoparticle, an 31) antibody, 32) a vesicle, 33) a nanoparticle, 34) at least two nanoparticles with a specific geometric arrangement such as an arrangement in chain, circle, square, rectangle, lozenge or triangle, and 35) at least one nanoparticle with at least 1, 2, 5, 10 or 100 side(s) or facet(s), preferentially different.

The invention also relates to the system according to the invention, wherein preferentially the at least one or two nanoparticle(s) of the second part is not or does not belong to or is not comprised, in at least one element of the group of nanoparticles selected from: 1) a nanoparticle or particle or assembly of atoms, ions, or molecules of size larger than $10^{-5}$, $10^{-3}$, $10^{-1}$, 0 or 1 nm, 2) a nanoparticle or particle or assembly of atoms, ions, or molecules of size smaller than $10^5$, $10^3$, 100, 50, 20 or 10 nm, 3) a nanoparticle with a core, preferentially metallic or magnetic or crystallized, 4) a nanoparticle with a coating, preferentially surrounding the core partly or fully, preferentially binding or associating at least two nanoparticles together, 5) a nanosphere, 6) a crystallized nanoparticle, 7) an amorphous nanoparticle, 8) a magnetic nanoparticle, 9) a nanoparticle that is formed or assembled in the body part or region or individual, 10) a nanoparticle that is formed or assembled outside the body part or region or individual, 11) a nano-capsule, 12) a dendrimer, 13) a carbon nanotube, 14) a lipid/solid nanoparticle, 15) a lipid or protein or DNA or RNA based nanoparticle, 15) a nanoparticle with an inner aqueous environment surrounded by a layer, preferentially a stabilizing layer, most preferentially a phospholipid layer, 16) a multilayer nanoparticle, 17) a polymeric nanoparticle, 18) a quantum dot, 19) a metallic nanoparticle, 20) a polymeric micelle or nanoparticle, 21) a carbon based nano-structure, 22) a nanobubble, 23) a nanosome, 24) a pharmacyte, 25) a niosome, 26) a nanopore, 27) a microbivore, 28) a liposome, 29) a virus, preferentially recombinant, 30) a herbal nanoparticle, an 31) antibody, 32) a vesicle, 33) a nanoparticle, 34) at least two nanoparticles with a specific geometric arrangement such as an arrangement in chain, circle, square, rectangle, lozenge or triangle, and 35) at least one nanoparticle with at least 1, 2, 5, 10 or 100 side(s) or facet(s), preferentially different.

In one embodiment of the invention, the at least one nanoparticle is a magnetic nanoparticle, preferentially predominantly, partly or fully, that preferentially has at least one magnetic property preferentially selected in the group consisting of: diamagnetic, paramagnetic, superparamagnetic, ferrimagnetic, and ferromagnetic, preferentially at a temperature below the blocking temperature or below 273, 100, 50, 20, 10, 1 or 0.1 K.

In one embodiment of the invention, the at least one nanoparticle is not a magnetic nanoparticle, preferentially predominantly, partly or fully,

In one embodiment of the invention, the at least one nanoparticle is a crystallized nanoparticle, preferentially predominantly, partly or fully, preferentially comprises at least one crystallographic plane or direction.

In one embodiment of the invention, the at least one nanoparticle is not a crystallized nanoparticle, preferentially predominantly, partly or fully,

In one embodiment of the invention, the at least one nanoparticle is a metallic nanoparticle, preferentially predominantly, partly or fully, preferentially comprises at least 1, 10 or $10^3$ metal or metallic atoms or ions, identical or different ones.

In one embodiment of the invention, the at least one nanoparticle is not a metallic nanoparticle preferentially predominantly, partly or fully

In one embodiment of the invention, the at least one nanoparticle is a nanoparticle that preferentially exists before or after the use of the system or part of it, preferentially predominantly, partly or fully,

In one embodiment of the invention, the at least one nanoparticle is not a metallic nanoparticle.

In another embodiment of the invention, the second part comprises at least 1, 5, 10, $10^3$, $10^5$ nanoparticles preferentially per cm or $cm^2$ or $cm^3$ preferentially of body part or region preferentially to be treated or interference region.

In another embodiment of the invention, the second part comprises less than $10^{20}$, $10^5$, $10^3$, 100, 50, 10, 2, or 1 nanoparticle(s), preferentially per cm or $cm^2$ or $cm^3$ preferentially of body part or region preferentially to be treated or interference region.

The invention also relates to the system according to the invention, wherein the at least two beams of radiation preferentially emitted by the at least two sources of radiation preferentially interfere in an interference region preferentially having at least one property selected in the group consisting of:

i) preferentially, the intensity or frequency or power or pressure of radiation is larger or different or smaller in the interference region than in at least one emission region or in at least one region outside of the interference region,
ii) preferentially, the heat or temperature is larger in the interference region than in at least one emission region or in at least one region outside of the interference region,
iii) preferentially, the number or concentration or size or activity of at least one nanoparticle is larger in the interference region than in at least one emission region or in at least one region outside of the interference region.

The invention also relates to the system according to the invention, wherein the interference region is preferentially subdivided in at least one, two or three part(s) selected among:

i) preferentially, a first part of the interference region in which the at least two beams of radiation emitted by the at least two sources of radiation interfere, which preferentially comprises at least one core of at least one nanoparticle preferentially comprised in a first portion of the body part or region, preferentially leading to an increase of radiation intensity or frequency or power or pressure or heat,
ii) preferentially, a second part of the interference region in which the at least two beams of radiation emitted by the at least two sources of radiation don't interfere, which preferentially comprises at least one side, surface, length of at least one nanoparticle preferentially comprised in a second portion of the body part or region, preferentially not leading to an increase or leading to less of an increase of radiation intensity or frequency or power or pressure or heat preferentially than in the first part, and
iii) preferentially, a third part of the interference region in which the at least two beams of radiation emitted by the at least two sources of radiation interfere, which is comprised in a third portion the body part or region without at least one nanoparticle, preferentially not leading to an increase or leading to less of an increase of radiation intensity or frequency or power or pressure or heat preferentially than in the first or second part,

In one embodiment of the invention, the at least two beams interfere more or more strongly or more intensely in at least one core of at least one nanoparticle than in at least one side, surface, length of at least one nanoparticle.

In another embodiment of the invention, the interference region comprises or is comprised in the first, second and/or third portion(s) of the body part or region to be treated, partly or fully.

The invention also relates to the system according to the invention for use in a method of treatment of a body part or region, wherein preferentially:

- preferentially in or during a first step, at least one nanoparticle of the second part is injected in the body part or region;
- Optionally, in a second step, the body part or region or interference region is imaged or detected;
- preferentially in or during a third step, the body part or region or interference region is exposed to at least two different beams of radiation and/or heated;

wherein optionally at least one step of the method is repeated more than once.

In some cases, the interference region is or is comprised in or comprises the body part or region to be treated.

In some other cases, the interference region is larger, preferentially by at least 0, $10^{-3}$, 1, 2, 5, 10, $10^3$ or $10^5$ nm, cm, m or $10^{-10}$, $10^{-3}$, $10^{-1}$, 0, 1, 5, 10, 25, or 50%, than the size or volume or diameter of the body part or region to be treated.

In still some other cases, the interference region is smaller, preferentially by at least 0, $10^{-3}$, 1, 2, 5, 10, $10^3$ or $10^5$ nm, cm, m or $10^{-10}$, $10^{-3}$, $10^{-1}$, 0, 1, 5, 10, 25, or 50%, than the size or volume or diameter of the body part or region to be treated.

In still some other cases, the emission region, interference region, body part or region to be treated, at least one region outside of it/them, and/or at least one region between the emission region and interference region, or between the emission region and body part or region to be treated or between the interference region and body part or region to be treated is smaller than $10^{50}$, $10^{10}$, $10^5$, $10^3$, 100, 50, 20, 10, 5, 2, 1, 0, $10^{-3}$ $m^3$, $cm^3$ or $mm^3$.

In still some other cases, the emission region, interference region, body part or region to be treated, at least one region outside of it/them, and/or at least one region between the emission region and interference region, or between the emission region and body part or region to be treated or between the interference region and body part or region to be treated is larger than $10^{-20}$, $10^{-5}$, $10^{-3}$, 0.1, 0, 1, 2, 5, 10 or $10^3$ $m^3$, $cm^3$ or $mm^3$.

In one embodiment of the invention, the interference region is the region of interference of at least two radiation beams, preferentially $B_i$ and $B_j$, wherein the interference region preferentially has at least one property selected in the group consisting of:

i) The interference region is such that at least one frequency, pressure, power, and intensity, acoustic or not, of the at least two beams is/are preferentially such or is/are preferentially sufficiently large, preferentially larger than $10^{20}$, $10^{-10}$, $10^{-5}$, $10^{-3}$, 0.1, 1, 10 or 100 MHz, KHz, H, W, $W/cm^2$ or Pa, that it/they do(es) result(s) in the production of heat or nanoparticle movement or cavitation preferentially inertial, or that it/they result(s) in or produce(s) a temperature increase or nanoparticle movement of magnitude larger than 0.1, 1, 5, 10, 100, $10^4$ or $10^5$ °C or cm, or is larger inside the interference region than outside the interference region, preferentially by at least 0, $10^{-3}$, $10^{-1}$, 1, 5, 10 or $10^3$ W (Watt), W per cm, W per $cm^2$ or W per $cm^3$ or Hz, kHZ, MHz, preferentially in some cases per radiation emitting element surface or body part or region, or is larger inside the interference region than outside the interference region, preferentially by at least $10^{-3}$, 0, 1, 5, 10, 25, 50, 75 or 90%, where this percentage is preferentially equal to (P2-P1)/P1, where P1 and P2 are preferentially equal to the intensity or power or power density of the at least two radiation beams inside and outside the interference region, respectively;

ii) The interference region is such that the radiation beam used for imaging crosses or interferes with the radiation beam used for heating in the interference region;

iii) The interference region is the overlapping region between: a) the region that is heated, preferentially the region above physiological temperature, preferentially by more than 0, $10^{-5}$, $10^{-3}$, $10^{-1}$, 1, 2, 5 or 10 °C, preferentially above 37, 39, 40, 41, 45 °C, preferentially below 100, 75, 55 or 40 °C, and b) the region that is imaged, where the overlapping region preferentially does not comprise the rectal wall or the radiation emitting element surface or a volume or body part or region localized at a distance of less than 10, 5, 2, 1 or 0.5 cm from the radiation emitting element surface;

iv) The interference region is a region or volume that is not cooled down or that is not in direct contact with the radiation emitting element surface or with the surface of the body part or region or with the transmitting material;

v) The interference region is a region or volume or body part or region that overlaps with or corresponds to the pathological site, preferentially the tumor, preferentially by at least 0, 1, 5, 10, 25, 50, 75, 90 or 99%, where this percentage preferentially corresponds to $(V_{IR}-V_{BP})/V_{BP}$, where $V_{IR}$ and $V_{BP}$ are preferentially the volumes of the interference region and body part or region, respectively;

vi) The interference region is a region or volume or body part or region that is preferentially located at a distance of more than 0, $10^{-5}$, $10^{-3}$, 10-1, 1, 5, 10 or 100 cm from the radiation

emitting element surface, the surface of the body part or region or the transmitting material. In some cases, the transmitting material can be a material located between the radiation emitting element surface and the body part or region or the surface of the body part or region or individual.

In some cases, the surface of the body part or region can be the external surface of the body part or region, preferentially the surface of the body part or region or individual that is in contact with the transmitting material, water, a gel, or the radiation emitting element surface.

In one embodiment of the invention, the heated region is the region of the body part or region that is heated, preferentially by the probe or system or part of the system, preferentially be at least one radiation emitting element of the probe or system or part of the system, preferentially above physiological temperature or above 37, 38, 40, 41, 43, 45, 50, 55, 60, 70 or 75 °C, preferentially below 100, 75, 60 or 55°C.

In one embodiment of the invention, the imaging region is the region of the body part or region that is imaged, preferentially by the probe or system or part of the system, preferentially be at least one radiation emitting element of the probe or system or part of the system, preferentially corresponding to the pathological site or a tumor, most preferentially a prostate tumor.

The invention also relates to the radiation heating probe or system or part of the system according to the invention, wherein at least one parameter, acoustic or not, of the heated region is larger in the presence than in the absence of at least one nanoparticle, wherein the parameter is selected in the group consisting of: i) the temperature, ii) the pressure, preferentially acoustic or of radiation, iii) the radiation frequency, iv) the radiation or acoustic energy preferentially of absorption, preferentially the radiation or acoustic energy absorption rate, and v) cavitation, inertial or not.

The invention also relates to a kit comprising the probe or system or at least one first part of the system according to the invention and at least one second part of the system or one nanoparticle.

The invention also relates to the radiation heating probe or system or part of the system according to the invention, wherein the at least one nanoparticle enables the interference of the at least two radiation beams to occur in the heated region or region of interference, preferentially in the nanoparticle region or region comprising at least one nanoparticle that is comprised in the heated region or interference region.

In some cases, the nanoparticle region is the region that comprises at least 1, 2, 5, 10, $10^3$ or $10^5$ nanoparticle(s) preferentially per $cm^3$ of body part or region.

In some other cases, the nanoparticle region is the region that comprises less than $10^5$, $10^3$, 100, 50, 20, 10, 5, 2 or 1 nanoparticle(s) preferentially per $cm^3$ of body part or region.

In still some other cases, the nanoparticle is a particle that has at least one dimension lower than 1000, 100, or 10 nm.

In still some other cases, the nanoparticle is a particle that has at least one dimension larger than 0, 1, 5, 10 or 100 nm.

In some other cases, the nanoparticle is magnetic, metallic, amorphous or crystallized.

In some cases, the nanoparticle can absorb radiation or be a radiation absorber or absorb more the radiation than the body part or region or diffuse the heat or diffuse heat more than the body part or region.

In some cases, the nanoparticles are different from the body part or region.

In some cases, a part of the system and/or at least one source of radiation can be a heating module.

In some cases, the body part or region can be or comprise or be comprised in the body part or region to be treated.

In some other cases, the body part or region can be or comprise or be comprised in at least one region outside the body part or region to be treated.

In still some other cases, the body part or region can be or comprise or be comprised in the emission region, the interference region or at least one region outside or between the emission and/or interference region(s).

The invention also relates to the probe or system or part of the system according to the invention, wherein the body part or region to be treated is a pathological site or a site that comprises at least one pathological cell, tumor cell, virus, or bacterium or at least one component, DNA, RNA, protein, enzyme preferentially belonging to or originating from a pathological cell, tumor cell, virus, or bacterium. The invention also relates to the probe or system or part of the system according to the invention, wherein the body part or region to be treated is a cancer or tumor preferentially selected from the group consisting of: the cancer of an organ, cancer of blood, cancer of a system of a living organism, adrenal cancer, anal cancer, bile duct cancer, bladder cancer, bone cancer, brain cancer, breast cancer, cervical cancer, colon/-rectum cancer, endometrial cancer, esophagus cancer, eye cancer, gallbladder cancer, heart cancer, kidney cancer, laryngeal and hypopharyngeal cancer, leukemia, liver cancer, lung cancer, nasal cavity and paranasal sinus cancer, nasopharyngeal cancer, neuroblastoma, non-Hodgkin lymphoma, oral cavity and oropharyngeal cancer, osteosarcoma cancer, ovarian cancer, pancreatic cancer, pancreatic penile cancer, prostate cancer, retinoblastoma, rhabdomyosarcoma, salivary gland cancer, sarcoma, skin cancer, small intestine cancer, stomach cancer, testicular cancer, thymus cancer, thyroid cancer, uterine cancer, uterine sarcoma cancer, vaginal cancer, vulvar cancer, Walden Strom macroglobulinemia Wilms tumor, Castleman disease Ewing family of tumor, gastrointestinal carcinoid tumor, gastrointestinal stromal tumor, myelodysplastic syndrome pituitary tumor, and a cancerous disease such as gestational trophoblastic disease, Hodgkin disease, Kaposi sarcoma, malignant mesothelioma, and multiple myeloma.

In another other embodiment of the invention, the body part or region is a part or a portion or the whole individual or organism, where the individual or organism is or comprises preferentially at least one prokaryote or eukaryote, living organism, mammal, plant, virus, bacterium, and/or pathological cell such as tumor cell.

In still another embodiment of the invention, the treatment of the body part or region is or consists in destruction, a heating, a removal, a detection, a replacement, and/or a compositional or size or physicochemical or structural change of the body part or region.

In one embodiment of the invention, the body part or region is the body part or region of an individual, animal, or human.

In an embodiment of the invention, the body part or region comprises more than or at least 1, 2, 5, 10, or 100 similar or different organism(s), apparatus, organ(s), tissue(s), cell(s), or biomolecule(s).

In some cases, the body part or region can be all or part of the head, neck, shoulder, arm, leg, knee, foot, hand, ankle, elbow, trunk, inferior members, or superior members.

In some other cases, the body part or region can be or belong to an organ, the musculoskeletal, muscular, digestive, respiratory, urinary, female reproductive, male reproductive, circulatory, cardiovascular, endocrine, circulatory, lymphatic, nervous (peripheral or not), ventricular, enteric nervous, sensory, or integumentary system, reproductive organ (internal or external), sensory organ, endocrine glands. The organ or body part or region can be human skeleton, joints, ligaments, tendons, mouth, teeth, tongue, salivary glands, parotid glands, submandibular glands, sublingual glands, pharynx, esophagus, stomach, small intestine, duodenum, jejunum, ileum, large intestine, liver, gallbladder, mesentery, pancreas, nasal cavity, pharynx, larynx, trachea, bronchi, lungs, diaphragm, kidneys, ureters, bladder, urethra, ovaries, fallopian tubes, uterus, vagina, vulva, clitoris, placenta, testes, epididymis, vas deferens, seminal vesicles, prostate, bulbourethral glands, penis, scrotum, pituitary gland, pineal gland, thyroid gland, parathyroid glands, adrenal glands, pancreas, heart, arteries, veins, capillaries, lymphatic vessel, lymph node, bone marrow, thymus, spleen, gut-associated lymphoid tissue, tonsils, brain, cerebrum, cerebral hemispheres, diencephalon, brainstem, midbrain, pons, medulla, oblongata, cerebellum, spinal cord, choroid plexus, nerves, cranial nerves, spinal nerves, ganglia, eye, cornea, iris, ciliary body, lens, retina, ear, outer ear, earlobe, eardrum, middle ear, ossicles, inner ear, cochlea, vestibule of the ear, semicircular canals, olfactory epithelium, tongue, taste buds, mammary glands, and/or skin.

In some cases, the body part or region or organ can belong to the blood circulation or circulatory system. In some cases, the body part or region can be or comprise at least one tumor, cancer, virus, bacterium, or pathological cell.

In one embodiment of the invention, the body part or region is or comprises water, an excipient, a solution, a suspension, at least one chemical element, organic material, or gel, which can be synthetic or produced by a living organism.

Preferably, the body part or region of an individual, also designated as the body part or region, represents or is part of an individual or a whole individual, where the individual is preferentially a human, an animal, or an organism, preferentially a living or inactivated or dead organism, comprising at least one prokaryotic or eukaryotic cell.

In one embodiment of the invention, the body part or region is alive (or not), is any tissue, water, medium, substance, cell, organelle, organ protein, lipid, DNA, RNA, biological material, preferentially localized in a specific region of an individual, preferentially originating or extracted from such region. In an embodiment of the invention, the body part or region comprises a pathological site, a healthy site, and/or a nanoparticle or composition region.

In one embodiment of the invention, the body part or region is or comprises a pathological site or pathological cells.

In some cases, the pathological site can be defined as an unhealthy site, or a site that is in a different condition from a site of a healthy individual, or the site of an unhealthy individual.

In some cases, the body part or region or pathological site can comprise pathological cells, such as tumor cells, bacteria, eukaryotic or prokaryotic cells, as well as viruses or other pathological material. Pathological cells can be cells that are: i) not arranged or working as they usual do in a healthy individual, ii) dividing more quickly than healthy cells, iii) healthy cells having undergone a transformation or modification, iv) dead, sometimes due to the presence of a virus or to other organisms, or v), in contact, in interaction, with foreign material not belonging to the individual, such as viruses, where viruses can possibly penetrate, colonize, or replicate in these cells.

In some cases, pathological cells can be assimilated to viruses or to other organisms or entities that colonize cells or target cells or destroy cells or use cells or enter in interaction with cells, preferentially to enable their own reproduction, multiplication, survival, or death.

In some cases, a pathological site can comprise healthy cells, preferentially with a lower number, activity or proliferation, than that of pathological cells.

In one embodiment of the invention, the body part or region is or comprises at least one healthy site and/or at least one healthy cells.

In some cases, the healthy site can be defined as a site or region that comprises healthy cell(s), where a healthy cell can be defined as a cell that belongs to a healthy individual or to the body part or region of a healthy individual.

In some cases, the healthy site can surround the pathological site when it is preferentially located at a distance of less than 1 or $10^{-9}$ m from the pathological site.

In some cases, the pathological cells can be cells comprised in a pathological site.

In some cases, the healthy cells can be cells comprised in a healthy site.

In some cases, the composition or body part or region or at least one region or at least one part of the system can be exposed to or emit radiations.

In some other cases, the composition or body part or region or at least one region or at least one part of the system is not exposed to or does not emit radiations.

In some cases, the body part or region can be treated preferentially by the probe or system or part of the system and/or hyperthermia.

In one embodiment of the invention, the probe or system or part of the system is used to carry out hyperthermia or hyperthermia or thermal or heat or cold treatment preferentially of the pathological site or tumor.

In another embodiment of the invention, hyperthermia or thermal or heat or cold treatment is or consists in the heating or thermal treatment or moderate heating or thermal treatment or mild heating or thermal treatment of the body part or region or pathological site or tumor.

In some cases, hyperthermia or thermal treatment can result in or be associated with the death of at least one cell of the body part or region or of the individual or living organism, preferentially or dominantly or at least partly through apoptosis or through a mechanism that triggers a message of self-destruction or suicide, preferentially from at least one first cell, preferentially a pathological one, which is heated or is both heated and in contact with or comprises at least one nanoparticle, and at least another second cell, preferentially another pathological cell, that is not heated or that is less heated than the first cell and that is not both heated and in contact with at least one nanoparticle.

In one embodiment of the invention, hyperthermia or thermal treatment is different from thermo-ablation or ablation.

In another embodiment of the invention, hyperthermia or thermal treatment is or consists in the heating and/or cooling of the body part or region.

In some cases, hyperthermia or thermal treatment or at least one step of the method according to the invention is or consists in the heating of the body part or region above physiological temperature or above 37, 39, 41 or 43 °C.

In some other cases, hyperthermia or thermal treatment or at least one step of the method according to the invention is or consists in the heating of the body part or region below 100, 75, 60 or 55 °C.

In still some other cases, hyperthermia or thermal treatment or at least one step of the method according to the invention is or consists in heating of the body part or region during less than $10^5$, $10^3$, 10, 5, 2 or 1 minute(s) or second(s).

In still some other cases, hyperthermia or thermal treatment or at least one step of the method according to the invention is or consists in heating or thermal treatment of the body part or region during more than $10^{-5}$, $10^{-3}$, $10^{-1}$, 0, 1, 2, 5, 10 or $10^2$ 1 minute(s) or second(s).

In another embodiment of the invention, the body part or region preferentially to be treated is exposed to radiations or the body part or region is treated preferentially by radiations when it receives or absorbs the radiations, preferentially when the body part or region or interference region receives or absorbs more radiations than at least one region that surrounds the body part or region or interference region.

The invention also relates to the probe or system or part of the system according to the invention, which is used for the treatment of the body part or region, preferentially heating or thermal treatment of the body part or region, preferentially when the probe or system or part of the system comprises at least one imaging radiation emitting element, preferentially for heating the body part or region, preferentially when the probe or system or part of the system comprises at least one source of radiation or heating radiation emitting element.

In one embodiment, the treatment comprises or is at least one treatment selected in the group consisting of: i) the treatment of an infection, ii) the treatment of a disease, preferentially a cancerous or tumoral or bacterial or viral disease, iii) radiotherapy, iv) photodynamic therapy, v) acoustic or ultrasonic treatment, vi) surgery, vii) focal therapy, viii) cryotherapy, ix) biopsy, x) anesthesia, xi) chemotherapy, xii) immunotherapy, and xiii) sonodynamic therapy.

In another embodiment, the treatment does not comprise or is not at least one treatment selected in the group consisting of: i) the treatment of an infection, ii) the treatment of a disease, preferentially a cancerous or tumoral or bacterial or viral disease, iii) radiotherapy, iv) photodynamic therapy, v) acoustic or ultrasonic treatment, vi) surgery, vii) focal therapy, viii) cryotherapy, ix) biopsy, x) anesthesia, xi) chemotherapy, xii) immunotherapy, and xiii) sonodynamic therapy.

In some cases, the disease can be due to the malfunction of an organ or body part or region preferentially of an individual.

In some cases, the treatment can be the therapy and/or the diagnosis of a disease or a cosmetic treatment. In some cases, the treatment can induce the death, destruction, denaturation, or inactivation of at least 1 biological material, such as cell, preferentially pathological cell, RNA, DNA, protein, lipid, or enzyme, where the death of cell can occur through apoptosis or necrosis.

In some cases, a pathological site is a site preferentially of the body part or region that comprises at least 1, 5, 10 or $10^3$ pathological or tumor cell(s), preferentially that does not comprise at least 1, 5, 10 or $10^3$ healthy cell(s), preferentially per $mm^3$, $cm^3$ of body part or region or tumor.

The invention also relates to the treatment of a disease preferentially selected from the group consisting of: a disease associated with a proliferation of cells that is different from the cellular proliferation in a healthy individual, a disease associated with the presence of pathological cells in the body part or region, a disease associated with the presence of a pathological site in an individual or body part or region, a disease or disorder or malfunction of the body part or region, a disease associated with the presence of radio-resistant or acoustic-resistant cells, an infectious disease, an auto-immune disease, a neuropathology, a cancer, a tumor, a disease comprising or due to at least one cancer or tumor cell, a cutaneous condition, an endocrine disease, an eye disease or disorder, an intestinal disease, a communication disorder, a genetic disorder, a neurological disorder, a voice disorder, a vulvovaginal disorder, a liver disorder, a heart disorder, a heating disorder, a mood disorder, anemia, preferentially iron anemia, and a personality disorder.

In some cases, the disease or disorder can be the disease or disorder of or belonging to the individual or body part or region, or the disease or disorder from which the individual is suffering.

In one embodiment of the invention, the cancer or tumor selected from the group consisting of: the cancer of an organ, cancer of blood, cancer of a system of a living organism, adrenal cancer, anal cancer, bile duct cancer, bladder cancer, bone cancer, brain cancer, breast cancer, cervical cancer, colon/rectum cancer, endometrial cancer, esophagus cancer, eye cancer, gallbladder cancer, heart cancer, kidney cancer, laryngeal and hypopharyngeal cancer, leukemia, liver cancer, lung cancer, nasal cavity and paranasal sinus cancer, nasopharyngeal cancer, neuroblastoma, non-Hodgkin lymphoma, oral cavity and oropharyngeal cancer, osteosarcoma cancer, ovarian cancer, pancreatic cancer, pancreatic penile cancer, prostate cancer, retinoblastoma, rhabdomyosarcoma, salivary gland cancer, sarcoma, skin cancer, small intestine cancer, stomach cancer, testicular cancer, thymus cancer, thyroid cancer, uterine cancer, uterine sarcoma cancer, vaginal cancer, vulvar cancer, waldenstrom macroglobulinemia wilms tumor, castleman disease ewing family of tumor, gastrointestinal carcinoid tumor, gastrointestinal stromal tumor, myelodysplastic syndrome pituitary tumor, and a cancerous disease such as gestational trophoblastic disease, Hodgkin disease, Kaposi sarcoma, malignant mesothelioma, and multiple myeloma.

The invention also relates to the treatment or a method of the treatment.

In some cases, the treatment can be the treatment of a disease such as anemia or iron anemia or of body part or region or of nanoparticles,

Ins some cases, the nanoparticle can be administered to the body part or region of an individual and/or the radiation can be applied to the body part or region, preferentially to reduce or stop or image or localize or measure or modify or increase or control or orientate a disease, body part or region, radiation and/or nanoparticle.

In some cases, the disease can be selected from the group consisting of: Iron or metal deficiency anemia, Vitamin deficiency anemia, Anemia of chronic disease, Aplastic anemia, Anemia associated with bone marrow disease, Hemolytic anemia, Sickle cell anemia, Thalassemia, Pernicious anemia, Fanconi anemia, Sideroblastic Anemia, Congenital Dyserythropoietic Anemia (CDA), Diamond Blackfan Anemia, and Megaloblastic Anemia.

The invention also relates to the probe or system or part of the system according to the invention, wherein a radiation transmission material such as preferentially water or a gel preferentially for ultrasound or gas preferentially for electromagnetic radiation, which transmits radiations, is preferentially located between the radiation emitting element or Radiation emitting element surface and the surface of the body part or region or body part or region or individual or rectum,

In some cases, the radiation transmission material can be or is maintained at a temperature that does not differ by more than 0.1, 1, 10, 100, $10^3$ or $10^5$ °C from the temperature of at least one location of the radiation emitting element or emission region, the heated or interfering or imaging region, or at least one region of the body part or region or surface of the body part or region or individual or rectum.

In one embodiment of the invention, the at least one source of radiation or radiation emitting element or radiation emitting element surface emits at least one radiation beam.

The invention also relates to the probe or system or part of the system according to the invention, where at least one radiation beam emitted by at least one source of radiation or heating radiation emitting element has at least one property selected in the group consisting of: i) it is orientated towards the body part or region to be treated, ii) it crosses or meats the body part or region to be treated, iii) it has a width or diameter or size that does not differ by more than 10, 50 or 100% from the width or diameter or size of the radiation emitting element surface or body part or region to be treated, iv) it has an intensity lower than 100, 20, 10, 5, 2 or 1 W or Watt per cm or Watt per $cm^2$ or Watt per $cm^3$ of radiation emitting element surface, body part or region to be treated, and v) it has a frequency that is larger than 1, 10, 100, or $10^3$ kHz, wherein the percentage in iii) is preferentially equal to $(S_{UB}-S_{BP})/S_{UB}$, with $S_{UB}$ preferentially being the width or diameter or size of the radiation beam and $S_{BP}$ preferentially being the width or diameter or size of the body part or region.

The invention also relates to the probe or system or part of the system according to the invention, where at least one radiation beam propagates in or along at least one direction, preferentially designated as direction of radiation beam propagation or emission, wherein the at least one radiation beam has at least one property selected in the group consisting of:

    a) It is the axis or axis of radiation emitting element or radiation emitting element surface, which is preferentially perpendicular to the radiation emitting element surface or which forms an angle that is between 0, 1, 5 or 30 ° and 45, 90, 150 or 360 ° with the radiation emitting element surface,

b) It is orientated towards the body part or region to be treated or heated region or imaging region or interference region or body part or region or pathological site or tumor,

c) It crosses or meats the body part or region to be treated or heated region or imaging region or interference region or body part or region or pathological site or tumor,

d) It forms an angle with the radiation emitting element surface that is larger than 0, 0.1, 10, 50, 80, or 85 °,

e) It forms an angle with the axis of the radiation emitting element surface that is larger than 0, 0.1, 10, 50, 80, or 85 °, where the axis of the radiation emitting element surface is preferentially the axis that is perpendicular to the radiation emitting element surface

f) It forms an angle with the radiation emitting element surface that is lower than 180, 150, 100, or 95 °,

g) It forms an angle with the axis of the radiation emitting element surface that is lower than 180, 150, 100, or 95 °,

h) It has a width or diameter or size that is lower than the width or diameter or size of the radiation emitting element surface or heated region or imaging region or interference region or body part or region to be treated or pathological site or tumor,

i) It has a width or diameter or size that is lower than 50, 10, 5 or 2 cm or $cm^2$ or $cm^3$ preferentially per cm, $cm^2$ or $cm^3$ of body part or region to be treated or heated region or imaging region or interference region or body part or region or pathological site or tumor,

j) It has a width or diameter or size that is lower than the width or diameter or size of the radiation emitting element surface or heated region or imaging region or interference region or body part or region or pathological site or tumor,

k) It has a width or diameter or size that does not differ by more than 1, 25, 50 or 99% than the width or diameter or size of the radiation emitting element surface or heated region or imaging region or interference region or body part or region or pathological site or tumor, where this percentage is preferentially equal to (DUB-DK)/DK, where DUB and DK are preferentially the diameters of the radiation beam for DUB and of the radiation emitting element surface or heated region or imaging region or interference region or body part or region or pathological site or tumor for DK,

l) It has a width or diameter or size that is larger than 0, 0.1 or 1 cm or $cm^2$ or $cm^3$ preferentially per cm, $cm^2$ or $cm^3$ of body part or region,

m) It has a width or diameter or size that is larger than the width or diameter or size of the radiation emitting element surface or heated region or imaging region or interference region or body part or region or pathological site or tumor,

n) It has a width or diameter or size that does not differ by more than 1, 25, 50 or 99% from the width or diameter or size of the radiation emitting element surface or heated region or imaging region or interference region or body part or region or pathological site or tumor,

o) It has a penetration depth that is larger than 0, 0.1 or 1 cm,

p) It has a penetration depth that is larger than the distance that separates at least one surface of the treated individual and the heated region,

q) It has a penetration depth that is lower than 200, 100, 50, 20 or 10 cm,

r) It has a penetration depth that is lower than the width of an individual,

s) It has an intensity that is larger than 0, 0.1, 0.5 or 1 W or W per cm or $W/cm^2$ or $W/cm^3$ of radiation emitting element surface, body part or region, or heated region,

t) It has an intensity that is above a certain threshold above which the radiation beam can reach the heated region and produce heat in this region, preferentially following interference with at least one other radiation beam in the heated region,

u) It has an intensity that is lower than 1000, 100, 20, 10, 5, 2 or 1 W or W per cm or $W/cm^2$ or $W/cm^3$ of radiation emitting element surface, body part or region, or heated region,

v) It has an intensity that is maintained below a certain threshold above which the radiation beam heats at least one region or volume located between the radiation emitting element surface or surface of the surface of the body art and the heated region,

w) It has a frequency that is larger than 1, 10, 100, $10^3$ or $10^4$ kHz, preferentially sufficiently large to enable the production of heat in the heated region,

x) It has a frequency that is lower than $10^6$, $10^5$ or $10^4$ kHz, preferentially sufficiently low to enable the radiation beam to penetrate in the body part or region sufficiently deep to reach the heated region, preferentially sufficiently low to enable the radiation beam to cover the heated region,

where the at least one radiation beam preferentially is or represents an assembly of at least one radiation wave propagating in the direction defined above.

The invention also relates to the radiation heating probe or system or part of the system according to the invention comprising more than 1, 2, 5, 10, 50, 75 or 100 radiation beams or source(s) of radiation or radiation emitting elements or radiation emitting element surfaces.

In some other cases, the radiation heating probe or system or part of the system according to the invention comprises less than $10^{10}$, $10^5$, $10^3$, 100, 50, 10, 5, 4, 3, 2, 1 or 0 5, 10, 50, 75, 50, 10, 5, 2, 1 or 0 radiation beams or source(s) of radiation or radiation emitting elements or radiation emitting element surfaces.

In some cases, at least one source of radiation or radiation emitting element or radiation emitting element surface emits at least 1, 2, 5 or 10 radiation beam(s).

In some other cases, at least one source of radiation or radiation emitting element or radiation emitting element surface emits less than $10^{10}$, $10^5$, $10^3$, 100, 50, 10, 5, 4, 3, 2, 1 or 0 5, 10, 50, 75, 50, 10, 5, 2, 1 or 0 radiation beam(s).

In some cases, at least one radiation beam has an intensity $i_k$ ($1 < k < j$),

In some cases, the difference in intensity between at least 2, 3, 4, 5, 10 or 50 different radiation beams is lower than $10^5$, 100, 75, 50, 25, 10, 5 or 1%, preferentially ($i_k$-$i_{k'}$/$i_k$) or ($i_k$-$i_{k'}$/$i$+$i_{k'}$) is lower than $10^5$, 100, 75, 50, 25, 10, 5 or 1%, where $i_k$ and $i_{k'}$ are the intensities of at least two different radiation beams k and k'.

In some other cases, the difference in intensity between at least 2, 3, 4, 5, 10 or 50 different radiation beams is larger than $10^{-10}$, $10^{-5}$, $10^{-1}$, 0, 1, 5, 10, 25, 50 or 100%, preferentially ($i_k$-$i_{k'}$/$i_k$) or ($i_k$-$i_{k'}$/$i$+$i_{k'}$) is larger than $10^{-10}$, $10^{-5}$, $10^{-1}$, 0, 1, 5, 10, 25, 50 or 100%, where $i_k$ and $i_{k'}$ are the intensities of at least two different radiation beams k and k'.

In some other cases, at least one source of radiation or radiation emitting element or radiation emitting element surface emits at least radiation beam at least 1, 2, 3 or 5 frequency $f_k$ ($1 < k < j$),

In some cases, the difference in frequencies between the at least 2 different radiation beams of frequencies k and k' is lower than $10^5$, 100, 75, 50, 25, 10, 5 or 1%, preferentially ($f_k$-$f_{k'}$/$f_k$) or ($f_k$-$f_{k'}$/$f_k$+$f_{k'}$) is smaller than $10^5$, 100, 75, 50, 25, 10, 5 or 1%, where $f_k$ and $f_{k'}$ are the frequencies of the at least two different radiation beams k and k'.

In some other cases, the difference in frequencies between the at least 2 different radiation beams of frequencies k and k' is larger than $10^{-10}$, $10^{-5}$, $10^{-1}$, 0, 1, 5, 10, 25, 50 or 100%, preferentially ($f_k$-$f_{k'}$/$f_k$) or ($f_k$-$f_{k'}$/$f_k$+$f_{k'}$) is larger than $10^{-10}$, $10^{-5}$, $10^{-1}$, 0, 1, 5, 10, 25, 50 or 100%, where $f_k$ and $f_{k'}$ are the frequencies of the at least two different radiation beams k and k'.

The invention also relates to the radiation heating probe or system or part of the system according to the invention, wherein the heated or interference region is characterized by the presence of a radiation beam with an intensity $i_{hR}$ in the emission or interference region having at least one property selected in the group consisting of:

    g. $i_{hR}$ is smaller than or equal to the sum of the intensities of each radiation beam $i_k$ preferentially in the emission region or in at least one region outside the interference region, *i.e.* $i_{hR} \pounds \sum_{k=1}^{k=j} i_k$, and

    h. $i_{hR}$ is larger than $i_k$, *i.e.* $i_{hR} > i_k$

In some cases, the at least one or two radiation beam(s) of or emitted by the at least one or two radiation emitting elements or radiation emitting element surfaces or source(s) of radiation has/have an intensity $i_k$, where k is an integer equal to 1 for beam 1, 2 for beam 2, j for beam j, or where $1 < k < j$.

The invention also relates to the radiation heating probe or system or part of the system according to the invention, where the heated or interference region is the region where constructive interference between the different radiation beams $i_k$ ($1 < k < j$) occurs,

In some cases, the region where constructive interference occurs is preferentially the region where:

    a. the intensities of the different radiation beams add up, at least partly, and/or

    b. the frequencies of the different radiation beams don't add up or are equal to an average frequency of the frequencies of the different radiation beams.

The invention also relates to the radiation heating probe or system or part of the system according to the invention and at least one nanoparticle, wherein the at least one nanoparticle is comprised in the heated or interference region or body part or region to be treated.

The invention also relates to a method for increasing the heating homogeneity in the heated or interference region or body part or region to be treated and/or for decreasing the production of heat in the region separating the at least one source of radiation or surface of the at least one radiation emitting element from the heated or interference region or body part or region to be treated and/or for increasing the depth in the individual of the heated or interference region preferentially by selecting or adjusting a property of the heating probe or system or part of the system according to the invention.

In some cases, at least one property of the heating probe or system or part of the system according to the invention is selected in the group consisting of:

    a. The number of different radiations emitting element surfaces emitting different radiation beams orientated in the direction of the heated region that is preferentially larger than or increased above 2, 5 or 10 and

    b. The intensity of at least one radiation beam emitted by at least one source of radiation or radiation emitting element or radiation emitting element surface that is preferentially reduced, preferentially below $10^5$, $10^3$, 10 or 1 W/cm$^2$, preferentially reduced with increasing number of different radiation emitting elements or radiation emitting element surfaces.

The invention also relates to the radiation heating probe or system or part of the system according to the invention, for treating a body part or region wherein the probe or system or part of the system is preferentially transrectal or part of a body part or region or organ.

The invention also relates to the probe or system or part of the system according to the invention, which is transrectal or inside or outside or above or below or at the surface of a body part or region or organ, partly or fully.

The invention also relates to the heating probe or system or part of the system according to the invention, which comprises at least two sources of radiation or radiation emitting elements, preferentially mono-element radiation emitting elements, which are preferentially positioned in the following manner:

a. The two sources of radiation or radiation emitting elements are attached to or embedded in or associated with or linked with a support or transrectal support or part of the system; and
b. The angle between surface of the source of radiation or the radiation emitting element and the horizontal or vertical axis or the axis of elongation of the rectum or body part or region or support preferentially on to or with which the at least one source of radiation or radiation emitting element is attached, is comprised between 0, 1, 5 or 20 ° and 5, 10, 45, 80, 90 or 180°;

In some cases, the support, transrectal or not, probe or system or part of the system is connected to a signal generator and an amplifier to generate radiation,

In some cases, the radiation emitting elements are the radiation emitting part or piezo elements of the probe or system or part of the system.

The invention also relates to the probe or system or part of the system according to the invention, which comprises at least two radiation emitting elements, preferentially mono-element radiation emitting elements, which are preferentially positioned in the following manner:

a. The at least two radiation emitting elements are preferentially attached to at least two different supports, which preferentially fit into or with each other; and
b. The angle between the heating radiation emitting element surface and the horizontal axis or the axis of elongation of the rectum or the axis of elongation of the support on two which the at least one source of radiation or heating bradiation emitting element is attached, is preferentially larger than 0, 1, 5 or 10°, preferentially comprised between 0, 2, 5, 10, 20, 30 ° and 40, 50, 70 or 80 ° (degree);
c. The angle between the imaging radiation emitting element surface and the horizontal axis or the axis of elongation of the rectum or the axis of elongation of the support on two which the at least one imaging radiation emitting element is attached, is preferentially smaller than 90, 45, 30, 20, 10, 5, 2 or 1 °, preferentially comprised between 0 ° and 10 °;

In some cases, the probe or system or part of the system is preferentially connected to a signal generator and/or an amplifier to generate radiations,

In some cases, the radiation emitting elements are preferentially the radiation emitting part or piezo elements of the probe or system or part of the system.

The invention also relates to the heating probe or system or part of the system according to the invention, wherein the distance between the two radiation emitting elements is comprised between 0, $10^{-3}$ or 1 and $10^3$, $10^5$ or $10^{10}$ mm, preferably between 20 and 60 mm, more preferably between 40 and 45 mm, where the distance between two radiation emitting elements is preferentially the distance separating the centers or the bottom parts of the two radiation emitting elements.

The invention also relates to the heating probe or system or part of the system according to the invention, wherein the radiation emitting element has at least one dimension comprised between 0, $10^{-3}$ or 1 and 100, 500, $10^3$ or $10^5$ mm, preferentially between 5 and 100 mm, preferably between 20 and 45 mm,

In some cases, the dimension of the radiation emitting element is preferentially selected among the group consisting of: a width, a length, a diameter, a size and a thickness of the radiation emitting element, radiation emitting element surface, and/or radiation emitting element volume.

The invention also relates to the probe or system or part of the system according to the invention, wherein the source of radiation or heating radiation emitting element has at least one dimension with at least one property selected in the group consisting of: i) it is comprised between between 0, $10^{-3}$ or 1 and 100, 500, $10^3$ or $10^5$ mm, preferably between 5 and 100 mm, more preferably between 20 and 45 mm, ii) it is larger than $10^{-3}$, 0.5 or 1 cm, iii) it is lower than $10^5$, 100, 50, 20, 10, 5 or 2 cm, and ii) its diameter $D_T$ does not differ by more than 0, 5, 50, 100 or $10^5$% from the diameter of the body part or region to be treated or tumor $D_{BP}$,
where this percentage is preferably equal to $(D_T-D_{BP})/D_T$ or $(D_T-D_{BP})/(D_T+D_{BP})$,

In some cases, the dimension of the source of radiation or radiation emitting element is selected among the group consisting of: a width, a length, a diameter, a size and a thickness of the radiation emitting element, Radiation emitting element surface, and radiation emitting element volume.

The invention also relates to the heating probe or system or part of the system according to the invention, wherein the source of radiation or radiation emitting element has an emitting surface with a geometry selected among the group consisting of: circular, rectangular, oval, spheric, cylindrical, truncated, and non-truncated.

The invention also relates to the heating probe or system or part of the system according to the invention, wherein the at least one source of radiation or radiation emitting element has an emitting surface comprised between 0, $10^{-5}$ or 1 and $10^6$ or $10^{10}$ mm², preferentially between 10 and $10^4$ mm², more preferably between 800 and 1000 mm².

In some cases, the emitting surface or radiation emitting element surface S1 is different from the surface of the body part or region to be treated S2 in that: preferentially S1 is metallic and S2 is organic, preferentially in part or in full, and/or preferentially S1 is smaller or larger than S2.

In still some other cases, S1 is separated from S2 by a distance of more than 0, $10^{-5}$, $10^{-1}$, 1, 2, or 5 cm. In still some other cases, S1 is separated from S2 by a distance of less than $10^5$, $10^3$, 50, 10, 5, 2, 1 or 0.1 cm.

In still some other cases, S1 is separated from S2 by a material transmitting radiations such as a gel and/or by a part of body part or region that is healthy or that is different from at least one pathological site or tumor.

The invention also relates to the heating probe or system or part of the system according to the invention, wherein the frequency of the radiation emitted by the at least one source of radiation or radiation emitting element or the frequency at which each radiation emitting element emits or produces radiations is between $10^{-5}$ or 0.1 and $10^9$ or $10^{20}$ KHz, preferentially between 10 and $10^6$ KHz, more preferably between 500 kHz and 3 MHz.

The invention also relates to the heating probe or system or part of the system according to the invention, wherein the intensity of the radiations emitted by the at least one source of radiation or radiation emitting element is between $10^{-20}$ or $10^{-5}$ and $10^4$ or $10^{20}$ W/cm², $10^{-3}$ and $10^3$ W/cm², 0.1 and 10 W/cm²,

In some cases, watt designates the radiation power preferentially at the surface of the radiation emitting element or near this surface

In some cases, cm² designates a surface unit preferentially of the radiation emitting element preferentially from which the radiations are emitted.

The invention also relates to the heating probe or system or part of the system according to the invention, wherein the distance between the at least two sources of radiation or parts of the system or radiation emitting elements is larger than $10^{-5}$ or 0.1 cm, preferentially larger than 1 cm, preferentially sufficiently large to be able to insert an imaging probe or system or part of the system in-between the two sources of radiation or radiation emitting elements, or preferentially sufficiently large to avoid an interference, preferentially in part or in full, between the radiation beams emitted by the at least two sources of radiation or radiation emitting elements outside of the heated or interference region or of the body part or region to be treated.

In some cases, the distance between two sources of radiation or radiation emitting elements is the distance separating the centers or the bottom parts of the two radiation emitting elements.

In some cases, the region of the body part or region to be treated is a tumor or cancer or at least one pathological site.

The invention also relates to the heating probe or system or part of the system according to the invention which further comprises a cooling part, where the cooling part has preferentially at least one property selected in the group consisting of:

i) it is a balloon, preferentially a balloon that is cooled down by cooling water inserted or comprised in the balloon;

ii) it is located above the two radiation emitting elements and/or in-between the two radiation emitting elements and the rectal wall or body part or region;

iii) It is or is maintained at a temperature that is lower preferentially than the temperature of the rectal wall or body part or region or physiological temperature or interference region, preferentially than or by at least $10^{-5}$, $10^{-1}$,1, 5, 10 or 100 °C;

iv) It enables or does not prevent the propagation of radiations, preferentially between the at least two radiation emitting elements and the rectal wall or body part or region;

v) It does not contain gazes or air, or it contains less than 1, 5, 10 or 50% in volume or mass of gazes or air, preferentially when the radiation is an acoustic wave or ultrasound and/or preferentially requires well-ordered and/or compactly distributed and/or arranged atoms to propagate;

vi) It cools down at least one region that is outside the interference or emission region or body part or region to be treated, partly or fully, preferentially below $10^5$, $10^3$, 100, 75, 50, 20, 10, 5, 2, 1 or 0°C preferentially below the temperature of the body part or region before or after or without the use or application of at least one part of the system or radiation, preferentially by at least $10^{-5}$, $10^{-1}$,1, 5, 10 or 100 °C, preferentially to avoid overheating or temperature variation or side effects preferentially induced by at least one part of the system or radiation or preferentially to stabilize or maintain the temperature preferentially at or close to the physiological temperature or less $10^{-5}$, $10^{-1}$,1, 5, 10 or 100 °C above or below the physiological temperature than preferentially in at least one of these regions;

and

vii) It cools down the interference or emission region or body part or region to be treated, partly or fully, preferentially below $10^5$, $10^3$, 100, 75, 50, 20, 10, 5, 2, 1 or 0°C preferentially to maintain at least one of these regions at the desired temperature preferentially during the use or application of at least one part of the system or radiation, preferentially at a temperature that is lower by at least $10^{-5}$, $10^{-1}$,1, 5, 10 or 100 °C preferentially than the temperature reached by the at least one region or part of the system without the cooling part, preferentially to avoid overheating or side effects or temperature variation preferentially induced by at least one part of the system or radiation preferentially in at least one of these regions and/or to stabilize the temperature preferentially at the desired temperature preferentially in at least one of these regions.

In some cases, gazes or air can block the propagation of radiations, preferentially acoustic waves, preferentially between the at least two radiation emitting elements and the rectal wall or body part or region.

In some cases, the body part or region can be the region to be treated or pathological site.

The invention also relates to the heating probe or system or part of the system according to the invention, which is preferentially surrounded by an unheated region or volume

In some cases, the unheated volume or region is located in the rectal wall or body part or region or at least one region outside the interference region or body part or region to be treated.

In some cases, the unheated, heated, emission, interference, region, at least one region outside the interference or common region and/or body part or region to be treated has/have at least one property selected in the group consisting of:

a. it/they is/are located above or in contact with or near the cooling part or the at least one radiation emitting element;

b. it/they is/are located at a distance from the cooling part or the at least one radiation emitting element, which is smaller than $10^5$, $10^3$, 10, 5, 2, 1 or 0 cm or mm;

c. it/they is/are located at a distance from the cooling part or the at least one radiation emitting element, which is larger than $10^{-20}$, $10^{-5}$, 0.1, 0, 1, 5 or 10 cm or mm

d. it/they has/have a temperature lower than 100, 50, 40, or 37 °C,

e. it/they has/have a thickness larger than $10^{-3}$, $10^{-1}$ or 1 cm,

f. it/they has/have a thickness lower than $10^3$, 10, or 5 cm,

g. it/they has/have a width smaller the 1 meter or the width of an individual, and

h. it/they has/have a width larger than $10^{-3}$, $10^{-1}$ or 1 cm.

The invention also relates to the source of radiation, heating probe or system or part of the system according to the invention, which is used in continuous, sequential of pulsed mode.

In some cases, the continuous mode consists of the mode in which the radiation is applied continuously in time and/or space, *i.e.* without at least 1, 2, 5, 10, 50 or 100 interruption(s) of radiation emission in time and/or space.

In some cases, the continuous mode consists of the mode in which the radiation is applied sequentially in time and/or space, *i.e.* with at least 1, 2, 5, 10, 50 or 100 interruption(s) of radiation emission in time and/or space.

In some cases, an interruption of radiation emission in time is an interruption after more or less than 0, 1, 5, 10, 100, $10^3$ or $10^5$ second(s), minute(s), hour(s) or day(s).

In some cases, an interruption of radiation emission in space is an interruption of radiation propagation after more or less than 0, $10^{-5}$, $10^{-3}$, $10^{-1}$, 0, 1, 5, 10, 100, $10^3$ or $10^5$ cm of radiation propagation or after radiations have propagated through more or less than 0, $10^{-3}$, 1, 5, 10, 25, 50, 100, 200 or 500% preferentially in mass or volume of body part or region of the body part or region or tumor.

In some cases, the pulsed mode consists of the mode in which the radiations are generated in a pulsed manner, where two pulses preferentially last or are preferentially separated by a lapse of time of more or less than 100, 10 or 1 second(s).

The invention also relates to the heating probe or system or part of the system according to the invention, wherein the body part or region of the individual preferentially treated by hyperthermia is preferentially designated by heated region.

In some cases, the unheated, heated, emission, interference, region, at least one region outside the interference or common region and/or body part or region to be treated has/have preferentially at least one property selected in the group consisting of:

1) It/they has/have at least one dimension, preferentially selected among a volume, surface, length, width, thickness, size, and diameter, which is larger than $10^{-3}$, $10^{-1}$, 1, 10, 100 or $10^5$ mm$^3$, mm$^2$ or mm;

2) It/they is/are surrounded by another region, where the other region is characterized by at least one property selected in the group consisting of:

a. It separates at least one element of the transrectal probe or system or part of the system from the heated or interference region;

b. It has a temperature that is lower than the temperature of the heated or interference region;

c. It does not comprise at least one pathological cell, a pathological site, a tumor or it comprises less pathological cells than the heated region, or it comprises a pathological site or a tumor of smaller size or dimension than the heated or interference region;

3) It/they comprise(s) at least one pathological cell, one pathological site or one tumor.

The invention also relates to a method to determine the characteristics of the transrectal probe or system or part of the system according to the invention preferentially to heat the region of the body part or region to be heated by hyperthermia,

In some cases, the method or system according to the invention comprises at least one of the following steps or parts selected in the group consisting of:

a) identifying the position and/or the size of the region of the body part or region that needs to be heated;
b) preferentially (choosing) a radiation emitting element among focalized and non-focalized radiation emitting elements, preferentially a non-focalized plane Radiation emitting element;
c) preferentially (choosing) a radiation emitting element of shape preferentially selected among the group of: circular, rectangular, oval, square, truncated, and oval shape;
d) preferentially (choosing) an emitting element or source of radiation of preferentially selected among a surface emitting area that is smaller or larger than the region of the body part or region that needs to be heated, preferentially a surface emitting area that is smaller than the size of the region of the body part or region that needs to be heated;
e) preferentially (choosing) at least two radiation emitting elements or radiation sources that are separated by a distance that is sufficiently large to be able to insert an imaging probe or system or part of the system in-between the at least two radiation emitting elements or radiation sources.

In some cases, the size of the heated or interference region or volume can be determined by simulations as a function of or using at least one the following varied parameters: i) the position and/or size of the body part or region that needs to be heated or treated, ii) the radiation emitting element type, iii) the emitting surface area, and iii) the distance separating the at least two radiation emitting elements or sources of radiation.

The invention also relates to a method for determining the at least one dimension of the body part to be treated or heated by the transrectal probe or system or part of the system according to the invention, by varying at least one parameter selected among: i) the emitting surface area of the radiation emitting elements or radiation sources, ii) the acoustic or radiation power, intensity, frequency of the radiations generated by the radiation emitting elements, iii) the frequency of the radiations generated by the radiation emitting elements, and iv) the duration of application of the radiations generated by the radiation emitting elements,

In some cases, the radiation emitting elements or sources of radiation have at least one property selected in the group consisting of:

a. the emitting surface area of the radiation emitting elements can be increased to increase the at least one dimension and/or homogeneity of the heated or interference volume or region;
b. the acoustic or radiation power or intensity and/or frequency of the radiations generated by the radiation emitting elements can be increased to increases the at least one dimension and/or homogeneity of the heated or interference volume or region;
c. the duration of application of the radiations generated by the radiation emitting elements can be increased to increase at least one dimension and/or homogeneity of the heated or interference volume or region;
and/or
d. the power, intensity, and/or frequency of the radiations generated by the radiation emitting elements can be decreased to increase at least one dimension and/or homogeneity of the heated or interference volume or region.

The invention also relates to a method according to the invention to determine the position of the region or volume heated or treated by the heating probe or system or part of the system, preferentially the transrectal probe or system or part of the system, according to the invention, by varying the angle between the emitting surface of the radiation emitting elements or radiation sources and the horizontal or elongation axis of the probe or system or part of the system, $\zeta$.

In some cases, $\zeta$ has at least one property selected in the group consisting of:

i) an increase of the angle $\zeta$ will bring the heated or interference volume or region closer to the horizontal or elongation axis of the probe or system or part of the system,
ii) a decrease of the angle $\zeta$ will bring the heated or interference volume or region farther apart from the horizontal or elongation axis of the probe or system or part of the system.

In some cases, the region to be heated or treated is the body part or region or pathological site or tumor. In another embodiment of the invention, the region that needs to be heated or treated or the pathological site or the tumor has or is characterized by: i) an acoustic or radiation power, intensity, and/or frequency that is/are larger than the acoustic or radiation power, intensity, and/or frequency of the beam $B_i$ or $B_j$ taken individually, and ii) a temperature that is larger than the temperature produced or associated with $B_i$ or $B_j$ taken individually.

In another embodiment of the invention, the at least one region that is outside the interference region or region that needs to be heated or outside the pathological site or outside the tumor is characterized by: i) an acoustic or radiation power, intensity, and/or frequency that is/are smaller than the acoustic or radiation power, intensity, and/or frequency of the sum of the acoustic or radiation powers, intensity, and/or frequency of the

beams $B_i$ and $B_j$, and ii) a temperature that is smaller than the temperature produced by the beams $B_i$ and $B_j$ in the region where they interfere or meet each other.

In some cases, the heating or interfering area or the region that needs to be heated is or comprises or includes or contains or is associated with or corresponds to at least one pathological site.

In one embodiment of the invention, the body part or region, preferentially the separating body part or region, region that needs to be heated, heating or interference or emission region, probe or system or part of the system, pathological site, and/or probe or system or part of the system, preferentially at least one part of the probe or system or part of the system has at least one dimension selected in the group consisting of: length, preferentially a length along a given axis such as a length along the X, Y or Z axis, a height, a width, thickness, diameter, surface, and volume. In some cases, the dimension is larger than 0, 0.1, 0.2, 0.5, 1, 2, 3, 5, 10, 20, 50 or 100 cm. In some other cases, the dimension is smaller than 100, 50, 20, 10, 5, 2, 1, 0.2 0.1, or 0 cm.

In some other cases, the heated or interference or emission region or the region that needs to be heated or at least one of its dimensions is smaller, preferentially by a factor of at least 0, 0.5, 1, 1.1, 2, 5, 10 or 100 or by at least 0, 0.5, 1, 5, 10, 50, 100 or 1000 cm than at least one pathological site or at least one of its dimensions.

In still some other cases, the heated or interference or emission region or the region that needs to be heated or at least one of its dimensions is larger, preferentially by a factor of at least 0, 0.5, 1, 1.1, 2, 5, 10 or 100 or by at least 0, 0.5, 1, 5, 10, 50, 100 or 1000 cm than at least one pathological site or at least one of its dimensions.

In one embodiment of the invention, the temperature in the heated or interference or emission area or region that needs to be heated or pathological site is larger than the physiological temperature, the temperature of the separating body part or region, 37, 40, 41, 42, 45, 50, 55, 60, 70, 80, 90, 100 or 200 °C.

In another embodiment of the invention, the temperature in the heated or interference or emission area or region that needs to be heated or pathological site is smaller than the physiological temperature, the temperature of the separating body part or region, 37, 40, 41, 42, 45, 50, 55, 60, 70, 80, 90, 100 or 200 °C.

In some preferential cases, the temperature in the heated or interference or emission area or region that needs to be heated or pathological site is that of hyperthermia or is comprised between 1 and 1000 °C, 10 and 100 °C, 40 and 60 °C, preferentially between 42 and 55 °C, preferably between 43 and 50 °C. In one embodiment of the invention, the temperature is the macroscopic temperature or the temperature measured over a length, surface or volume that is larger than the size of 0.1, 1, 10, 100 or 1000 mm, $mm^2$, $mm^3$ or that is larger than the size of at least one nanoparticle.

In another embodiment of the invention the temperature is the nanoscopic temperature or the temperature measured over a length, surface or volume that is smaller than the size of 0.1, 1, 10, 100 or 1000 mm, $mm^2$, $mm^3$ or that is smaller than or equal to the size of at least one nanoparticle.

In another embodiment of the invention, the heated or interference or emission area or region that needs to be heated is located between a first value of X measured along a X axis that is preferentially smaller or larger than or equal to: 0, -1, -2, -5, -10, -15, -20, -50, or -100 cm and a second value of X measured along the X axis that is preferentially smaller or larger than or equal to 0, 1, 2, 5, 10, 15, 20, 20, 50 or 100 cm.

In some cases, the X axis is the axis of elongation of the probe or system or part of the system.

In some cases, X=0 corresponds to the entry of the rectum or the surface of the individual or body part or region.

Preferentially, the spread or width of the heated or interference or emission area or region along the X axis is the difference between the second and first values of X. It can preferentially be increased by increasing the size of the emitting surface of the radiation emitting element in the X direction.

In another embodiment of the invention, the heated or interference or emission area or region that needs to be heated is located between a first value of Y measured along a Y axis that is preferentially smaller or larger than or equal to: 0, -1, -2, -5, -10, -15, -20, -50, or -100 cm and a second value of Y measured along the Y axis that is preferentially smaller or larger than or equal to 0, 1, 2, 5, 10, 15, 20, 20, 50 or 100 cm.

In some cases, the X, Y and/or Z axis(axes) is/are perpendicular.

In some other cases, the X, Y and/or Z axis(axes) cross each other or cross at least one part of the system, preferentially its center, preferentially at X=0, Y=0 and/or Z=0.

In some cases, $a_i$ is the angle between at least one first part of the system such as the support, preferentially its axis, elongation axis, diameter, or surface, and a second part of the surface such as the radiation source, preferentially its axis, elongation axis, diameter, or surface.

In some cases, ai is larger than $10^{-3}$, 0, 1, 5, 25, 45, 90, 90, 100, 180 or 360 °.

In some other cases, ai is smaller than 360, 180, 100, 90, 45, 25, 20, 10, 5, 21, 1 or 0°.

Preferentially, the spread or width of the heated or interference or emission area or region along the Y axis is the difference between the second and first values of Y. It can preferentially be increased by increasing the size of the emitting surface of the radiation emitting element in the Y direction or by increasing the value of the angle $a_i$, preferentially from a first value of $a_i$ that is smaller than or equal to 90, 80, 60, 45, 30, 20, 10, 5, 2, 1 ° to a second value of $a_i$ that is larger than or equal to 0, 1, 2, 5, 10, 20, 30, 45, 60, 80 or 90 °.

In another embodiment of the invention, the heated or interference or emission area or region that needs to be heated is located between a first value of Z measured along a Z axis that is preferentially smaller or larger than or equal to: 0, -1, -2, -5, -10, -15, -20, -50, or -100 cm and a second value of Z measured along the Z axis that is preferentially smaller or larger than or equal to 0, 1, 2, 5, 10, 15, 20, 20, 50 or 100 cm.

Preferentially, the spread or width of the heated area along the Z axis is the difference between the second and first values of Z.

Preferentially, the heated area along the Z axis is above or behind or below or outside the separating body part or region.

In some cases, D2 is the distance separating at least one part of the system, preferentially its center, border, inside or surface or volume, and the region of interference or body part or region to be treated, preferentially its center, border, inside or surface or volume.

In another embodiment of the invention, the distance $D_2$ can be increased, by decreasing the value of $a_i$, preferentially from a first value of $D_2$ smaller than or equal to 20, 10, 7, 5 or 2 mm for $a_i$ larger than 30, 35, 40, 50 or 60 ° to a second value of $D_2$ larger than or equal to é, 5, 10 or 20 mm for $a_i$ smaller than or equal to 60, 50, 40, 35 or 30 °.

In another embodiment of the invention, the heated or interference or emission volume or area or region can be increased by increasing the value of $a_l$, preferentially from a first value of the heated volume smaller than or equal to 10, 5, 2, 1, 0 cm$^3$ for $a_i$ smaller than 60, 40, 35, 30, 20, 10, 5, 2, 1 or 0 ° to a second value of the heated volume larger than or equal to 0, 1, 5, 10 or 20 cm$^3$ for $a_i$ larger than 0, 1, 2, 5, 10, 20, 30, 40, 50 or 60 °.

Preferentially the heated or interference or emission volume or region varies as a function of the angle $a_i$ along the Y axis or axis of elongation of the probe or system or part of the system or axis from which the angle $a_i$ is formed.

Preferentially, when the angle $a_i$ is increased, the heated or interference or emission volume or region varies less along the X and Z axis than along the Y axis or does not vary along the X and Z axis or varies by less than a factor of 3 along the X or Z axis, or decreases along the X or Z axis.

D1 is the distance separating at least two parts of the system such as two radiation sources, preferentially their center, border, inside or surface or volume.

In some cases, D1 and/or D2 is/are larger than 10$^{-20}$, 10$^{-3}$, 0, 0.5, 1, 5, 10 or 20 cm.

In some other cases, D1 and/or D2 is/are smaller than 10$^{20}$, 10$^{10}$, 10$^5$, 10$^3$, 10, 5, 2, 1 or 0 cm.

In still another embodiment of the invention, the distance $D_2$ increases with increasing value of $D_1$, preferentially from a first value of $D_2$ smaller than or equal to 20, 10, 5, 2, 1 or 0 mm when $D_1$ is smaller than or equal to 50, 40, 30, 20, 10, 5, 2, 1 or 0 mm, to a second value of $D_2$ larger than or equal to 0, 1, 5, 10 or 20 mm when $D_1$ is larger than or equal to 0, 1, 2, 5, 10, 20, 30 or 40 mm.

In still another embodiment of the invention, the heated or interference or emission volume decreases with increasing value of D1, preferentially from a first Volume larger than or equal to 0, 1, 2, 4, 5, 9, 10 or 20 cm$^3$ when $D_1$ is smaller than or equal to 50, 40, 30, 20, 10, 5, 2, 1 or 0 mm, to a second Volume smaller than or equal to 20, 10, 9, 8, 7, 5, 4, 3, 2, 1 or 0 cm$^3$ when $D_1$ is larger than or equal to 0, 1, 2, 5, 10, 20, 30 or 40 mm.

Preferentially, the heated or interference or emission volume varies as a function of $D_1$ along the Y axis or axis of elongation of the probe or system or part of the system or axis from which the angle $a_i$ is formed.

Preferentially, when $D_1$ is increased, the heated or interference or emission volume varies less along the X and Z axis than along the Y axis or does not vary along the X and Z axis or varies by less than a factor of 3 along the X or Z axis, or decreases along the X or Z axis.

In one embodiment of the invention, the acoustic or radiation power or pressure preferentially in the heated or interference or emission area or volume or region is comprised between a first value smaller than or equal to 5, 2, 1, 0.5, 0.2 MPa and a second value larger than or equal to 0, 0.2, 0.5, 1, 2, or 5 MPa.

In another embodiment of the invention, the acoustic or radiation power or pressure preferentially in the heated or interference or emission area or volume or region is larger than 10$^{-20}$, 10$^{-5}$, 10$^{-1}$, 0, 1, 5, 10 or 10$^3$ MPa.

In still another embodiment of the invention, the acoustic or radiation power or pressure preferentially in the heated or interference or emission area or volume or region is smaller than 10$^{20}$, 10$^{10}$ 10$^5$, 100, 50, 2, 1, 0, 10$^{-3}$ or 10$^{-10}$ MPa.

In one embodiment, the acoustic power or pressure and/or the heat produced in the heated or interference or emission area or region or pathological site are generated by at least one of the following actions: i) switching on or activating the transrectal probe or system or part of the system, and ii) positioning or locating the probe or system or part of the system in a region that enables the probe or system or part of the system to heat the heating area.

In some cases, the ssytem or at least on part of the system can be activated or witched on by being plugged or powered by electricity or current, or by having electricity or current transformed into or generating a radiation.

In another embodiment of the invention, the acoustic or radiation power or pressure in the heated or interference or emission area or region is increased to increase the amount of heat produced in the heated or interference or emission area or region.

In another embodiment of the invention, the acoustic power or pressure in the heated or interference or emission area or region is decreased preferentially to decrease the amount of heat produced in the heated area.

In another embodiment of the invention, the acoustic power or pressure exists or is generated or occurs in the acoustic pressure area.

In some cases, the acoustic pressure area has at least one dimension that is smaller than at least one dimension of the heated area.

In some other cases, the acoustic pressure area has at least one dimension that is larger than at least one dimension of the heated or interference or emission area or region.

In another embodiment of the invention, the acoustic pressure area or region is located between a first value of X, Y, or Z measured along a X, Y, or Z axis that is preferentially smaller or larger than or equal to: 0, -1, -2, -5, -10, -15, -20, -50, or -100 cm and a second value of X, Y, or Z measured along the X, Y, or Z axis that is preferentially smaller or larger than or equal to 0, 1, 2, 5, 10, 15, 20, 20, 50 or 100 cm.

In still another embodiment, the temperature preferentially in the heated or interference or emission area or region and/or the acoustic power or pressure preferentially in the acoustic power area is/are kept sufficient low preferentially to avoid coagulative necrosis or thermal ablation or cavitation or inertial cavitation.

In still another embodiment of the invention, when an entity such as the probe or system or part of the system or body part or region or radiation has a property or parameter with a value of P1 that is higher, longer, or larger by a factor $\alpha$ than another property or other parameter with a value of P2, it means that: P1=$\alpha$.P2 ($\alpha$ preferentially larger than 1) or P1=$\alpha$+P2 ($\alpha$ preferentially larger than 0).

In still another embodiment of the invention, when an entity such as the probe or system or part of the system or body part or region or radiation has a property or parameter with a value of P1 that is lower, smaller, or shorter by a factor $\beta$ than another property or other parameter with a value of P2, it means that: P1=$\beta$.P2 ($\beta$ is preferentially smaller than 1), P1=P2/$\beta$ ($\beta$ is preferentially larger than 1), P1=P2-$\beta$ ($\beta$ is preferentially larger than 0) or P1=$\beta$- P2.

In some cases, a is the angle between at least one first part of the system, such as the support, preferentially its axis, elongation axis, diameter, or surface, and at least one second part of the system such as the radiation source, preferentially its axis, elongation axis, diameter, or surface.

In some cases, $a_1$ is the angle between at least one first part of the system and a first radiation source. In some other cases, $a_2$ is the angle between at least one first part of the system and a second radiation source.

In one embodiment of the invention, the angle a designates $a_1$ or $a_2$ with a = $a_1$ = $a_2$.

The invention also relates to the heating probe or system or part of the system according to the invention, which is not or does not comprise at least one component or radiation emitting element generating focused radiation, preferentially high intensity focused radiation.

In some cases, a focused radiation is a radiation beam focused on a surface or volume of less than $10^3$, $10^2$, 10, 5, 2, 1, $10^{-5}$ cm or $cm^2$ or $cm^3$ preferentially of body part or region.

In some other cases, a radiation of high intensity is a radiation of intensity larger than $10^{-3}$, 0, 1, 10, 100 or $10^3$ W per cm, $cm^2$ or $cm^3$ of radiation emitting element, radiation emitting element surface or body part or region length, surface or volume.

The invention also relates to the heating probe or system or part of the system according to the invention, which is or comprises at least one component or radiation emitting element generating an unfocused radiation.

In some cases, an unfocused radiation is a radiation beam focused on or covering or heating a surface or volume of more than $10^3$, $10^2$, 10, 5, 2, 1, $10^{-5}$ cm or $cm^2$ or $cm^3$ preferentially of body part or region. In some cases, an unfocused radiation is a radiation beam focused on or covering or heating more than $10^{-5}$, $10^{-3}$, $10^{-1}$, 0, 1, 5, 10, 25, 50, 75, 99 or 100% of body part or region preferentially in mass or volume. The invention also relates to the heating probe or system or part of the system according to the invention, which is or comprises at least one component or radiation emitting element generating radiations of low intensity.

In some other cases, a radiation of low intensity is a radiation of intensity lower than $10^{-3}$, 0, 1, 10, 100 or $10^3$ W per cm, $cm^2$ or $cm^3$ of radiation emitting element, radiation emitting element surface or body part or region length, surface or volume.

The invention also relates to the heating probe or system or part of the system according to the invention, which comprises at least two radiation emitting elements selected in the group consisting of:

    a. at least one imaging radiation emitting element, and
    b. at least one heating radiation emitting element.

In some cases, an imaging radiation emitting element is a radiation emitting element serving to image a body part or region or a radiation emitting element that is inserted in or is part of an imaging radiation probe or system or part of the system, preferentially to enable imaging the body part or region.

In some cases, a heating radiation emitting element is a radiation emitting element serving to heat a body part or region or a radiation emitting element that is inserted in or is part of a heating radiation probe or system or part of the system, preferentially to enable heating the body part or region.

In some cases, a radiation emitting element is, is comprised in or comprises a source of radiation, preferentially in part or in full.

In some cases, the heating radiation emitting element can generate radiations of higher intensities than the imaging radiation emitting element.

In some cases, the imaging radiation emitting element does not produce heat.

In some other cases, the heating radiation emitting element does not provide imaging.

In some cases, the heating and imaging radiation emitting elements are two different radiation emitting elements or are two radiation emitting elements separated from each other or are two radiation emitting elements whose radiation emitting radiation beams interfere in the interfering or heating or imaging region and preferentially do not interfere outside of the interfering or heating or imaging region.

In some cases, the interference of at least two beams, preferentially heating and/or imaging, is concomitant, preferentially in time and/or space.

In some other cases, the interference of at least two beams is not concomitant.

In some cases, the heating and imaging radiation emitting elements are two radiation emitting elements that are embedded in or attached to the same support or probe or system or part of the system or to two different supports or probe or system or part of the system.

The invention also relates to a method to determine the at least one dimension of the body part or region heated by the probe or system or part of the system, preferentially the transrectal probe or system or part of the system according to the invention, by varying at least one parameter selected among: i) the emitting surface area of the radiation emitting elements, ii) the acoustic power of the radiations generated by the radiation emitting elements, iii) the frequency of the radiations generated by the radiation emitting elements, and iv) the duration of application of the radiations generated by the radiation emitting elements.

In some cases, the radiation emitting elements have at least one property selected in the group consisting of:

a. the emitting surface area of the radiation emitting elements can be increased preferentially to increase the at least one dimension and/or homogeneity of the heated or interference volume or region;

b. the acoustic power of the radiations generated by the radiation emitting elements can be increased preferentially to increases the at least one dimension and/or homogeneity of the heated or interference volume or region;

c. the duration of application of the radiations generated by the radiation emitting elements can be increased preferentially to increase at least one dimension and/or homogeneity of the heated or interference volume or region; and

d. the frequency of the radiations generated by the radiation emitting elements can be decreased preferentially to increase at least one dimension and/or homogeneity of the heated or interference volume or region.

The invention also relates to a method to determine the position of the volume heated by the transrectal probe or system or part of the system according to the invention, preferentially by varying the angle $\beta$ between the emitting surface of the radiation emitting elements and the horizontal or elongation axis of the probe or system or part of the system.

In some cases, the angle $\beta$ has at least one property selected in the group consisting of:

i) an increase of $\beta$ brings the heated or interference volume or region closer to the horizontal or elongation axis of the probe or system or part of the system,

ii) a decrease of $\beta$ will bring the heated or interference volume or region farther apart from the horizontal or elongation axis of the probe or system or part of the system.

The invention also relates to a method for treating a body part or region using the probe or system or part of the system according to the invention, wherein:

- In a first step, at least one nanoparticle is injected in the body part or region,
- In a second part, the body part or region to be treated is heated,

wherein the first and/or second step(s) is/are preferentially carried out under radiation imaging guidance using the probe or system or part of the system.

The invention also relates to the use of the probe or system or part of the system or at least one method or process for treating a body part or region, preferentially by the method according to the invention. The invention also relates to a system, preferentially a radiation system or radiation heating system, preferentially for treating a body part or region preferentially of an individual comprising:

- A first part comprising at least two or three different sources of radiation preferentially emitting at least three different beams of radiation, said sources being preferentially positioned and/or orientated so that at least two or three beams are preferentially orientated in the direction of at least one common or interference region, and/or spatially overlap in at least one common or interference region, which is preferentially distinct from the emission region or near field region or focal point or region preferentially of at least one individual radiation source and/or is comprised in the far-field and/or Fraunhofer region preferentially of at least one individual radiation source and/or is distinct of the focal point or region preferentially of the assembly of at least two radiation sources and/or is comprised in the near-field region preferentially of the assembly of at least two radiation sources;

wherein the at least two or three different sources of radiation or the beam(s) that they emit have at least one property selected in the group consisting of:

a) At least one different location and/or orientation and/or inclination, preferentially relatively to the support on to which or with which they are associated or linked preferentially comprised in the system,

b) At least one angle $\beta$ between the emitting part or piezo element of at least one source of radiation, preferentially its emitting surface, and at least one dimension of the support preferentially on to which it is attached such as its elongation axis or the horizontal axis, which is preferentially in some cases larger than 0, 1, 2, preferentially 5, 10, or 25 °

c) At least one angle $\beta$ between the emitting part or piezo element of at least one source of radiation, preferentially its emitting surface, and at least one dimension of the support preferentially on to which it is attached such as its elongation axis or the horizontal, which is preferentially in some other cases smaller than 360, 180, 90, preferentially 45, 30, 25 or 10°

d) At least one difference between the angle of the emitting part or piezo element of the source of radiation i, preferentially its emitting surface, $\beta_i$, and the emitting part or piezo element of the source of radiation j, preferentially its emitting surface, $\beta_j$, preferentially $\beta_i - \beta_j$ that is preferentially in some cases larger than 0, $10^{-10}$, $10^{-3}$, preferentially 0.1, 1, 2, 5 or 10 °,

e) At least one difference between the angle of the emitting part or piezo element of the source of radiation i, preferentially its emitting surface, $\beta_i$, and the emitting part or piezo element of the source of radiation j, preferentially its emitting surface, $\beta_j$, preferentially $\beta_i - \beta_j$ that is preferentially in some other cases smaller than 360, 180, 90, 45, 30, preferentially 20, 10, 5, 2 or 1 °,

f) At least one angle $\zeta$ between the axis or main axis or central axis or direction preferentially of propagation of the radiation or beam or radiation and the emitting part or piezo element of the source of radiation, preferentially its surface, which is preferentially in some cases larger than or equal to 0, 1, 2, 5, 10, 25, preferentially 30, 45, 60, 75, 80 or 90 °,

g) At least one angle $\zeta$ between the axis or main axis or central axis or direction preferentially of propagation of the radiation or beam or radiation and the emitting part or piezo element of the source of radiation, preferentially its surface, which is preferentially in some other cases smaller than or equal to 360, 180, 120, 100, 90, 50, 40 or 30°, preferentially smaller than or equal to 120, 100 or 90 °;

h) At least one difference between the angle $\zeta_i$ for a first source of radiation i and the angle $\zeta_j$ for a second source of radiation j, preferentially $\zeta_i - \zeta_j$ that is preferentially in some cases larger than 0, $10^{-10}$, $10^{-3}$, preferentially 0.1, 1, 2, 5 or 10 °,

i) At least one difference between the angle $\zeta_i$ for a first source of radiation i and the angle $\zeta_j$ for a second source of radiation j, preferentially $\zeta_i - \zeta_j$ that is preferentially in some other cases smaller than 360, 180, 90, preferentially 45, 30, 25 or 10 °;

j) The emission or composition of the same types of radiation in some cases;

k) The emission or composition of different types of radiation in some other cases;

l) At least one opening angle or divergence angle or beam spread or opening beam angle that is in some cases larger than 0, 0.1, preferentially 1, 2, 5, 10 or 45 °;

m) An opening angle or divergence angle or beam spread or opening beam angle that is in some other cases smaller than 360, 180 °, preferentially 90, 45, 20, 10, 5, 2 or 1 °; and

n) The orientations of at least two or three beams that are such that a first combination or superposition of at least two beams overlap in a first common region and a second combination or superposition of at least two beams, preferentially different from the first one, overlap in a second common region, where preferentially the set or sum of the at least two common regions constitutes, preferentially partly or fully or predominantly, or is comprised in the body part or region to be treated or heated region, where preferentially the at least one common region is different or separated or located at a distance of more than $10^{-10}$, $10^{-5}$, $10^{-1}$, 0, 1, 2, 5, 10 or $10^3$ nm, cm or m from the emitting part or piezo element of the source of radiation, emission region, focal point, focal region and/or near-field region preferentially of at least one source of radiation, where preferentially at least one common region is in the far field or Fraunhofer region of at least one source of radiation;

- A second part comprising at least one nanoparticle preferentially magnetic or metallic nanoparticle preferentially comprised or located in the far field or Fraunhofer region of at least one source of radiation or said common region and preferentially exposed to the overlapping beams of radiation;

wherein the first and second parts are preferentially separated from each other by a distance larger than the Fresnel or near-field distance preferentially of at least one source of radiation or 1 nm;

where the at least one nanoparticle or the system preferentially is not or does not comprise or does not operate through the generation or does not have the function of generating at least 1, 2, 5, 10 or 100 nanobubble(s), bubble(s), or microbubble(s),

where the system preferentially does not comprise at least one fluorophore.

In one embodiment of the invention, the opening or divergent angle or beam spread or its/their value of the radiation, preferentially ultrasound or radiation or ultrasound beam, is proportional or related to the width or spreading of the beam.

Preferentially, the larger opening angle, the larger the beam width or spreading.

Preferentially, the larger the opening angle, the more the beam widens or broadens as it propagates away from the source of radiation or transducer or piezo element or as it travels through a medium or body part or region.

Preferentially, the opening angle is the angle between the central axis of the beam and the edge of the beam or main lobe, in some cases defined as located at the -6 dB intensity point, preferentially where the intensity drops or decreases to half its maximum.

Preferentially, a narrow opening angle, preferentially lower than 360, 180, 90, 45, 20, 10, 5, 2 or 1 °, is associated with a collimated beam, preferentially with enhanced lateral resolution, preferentially with shallower depth of field.

Preferentially, a wide opening angle, preferentially larger than 0, 1, 2, 5, 10, 45, 90, 180 or 300 °, is associated with a divergent beam, preferentially with a lower lateral resolution, preferentially with a larger coverage, preferentially of the body part or region or region to be treated.

In some cases, $\sin(\theta)=1.22c/f.D$ or $\sin(\theta)$ or $\theta$ is inversely proportional to $f.D$ or to $F$ or to $D$, where preferentially $\theta$ is preferentially half preferentially opening or diffracting angle preferentially in radians, $c$ is preferentially the speed of sound or radiation in the medium, preferentially $c \approx 1540$ m/s in soft tissue for sound, $f$ is preferentially the ultrasound or radiation frequency preferentially in Hz, $D$ is preferentially the transducer or piezo element diameter or aperture diameter or the diameter or aperture diameter of the source of radiation or of the emitting part or piezo element of the source of radiation, preferentially in m.

In some other cases, a distribution of wide opening angles of a series or assembly of sources of radiations, preferentially having their beam(s) orientated in the direction of the interference or common region or body part to be treated is desired to or results in or is associated with or comprises a spread of energy or radiation or radiation parameter and/or preferentially production of moderate and/or uniform effects of radiation such as mechanical or thermal small or moderate variation, increase of temperature of less than 100, 50, 25, 10, 5 or 2 °C, preferentially within the interference or common region or body part to be treated, preferentially temperature increase or mechanical effects larger in the interference or common region or body part to be treated than outside at least one of these regions.

In some cases, the radiation can be or can be associated with the radiation field.

In some cases, the mechanical variation of the radiation or radiation field is or is associated with the variation of at least one of the following parameters of the radiation or exerted by the radiation: pressure, intensity, force, and mechanical displacement/strain.

In some cases, the mechanical variation can be small, i.e. that preferentially the variation of at least one of the above parameters is smaller by factor of at least 100, 50, 10, 5 or 2, preferentially between at least two different regions of the interfering or common region or body part or region to be treated.

In some cases, the thermal variation can be small, i.e. that preferentially the variation of temperature is smaller by factor of at least 100, 50, 10, 5 or 2, preferentially between at least two different regions of the interfering or common region or body part or region to be treated.

In some cases, the thermal and/or mechanical variations can be larger in a region comprising at least one nanoparticle than in a region without nanoparticle(s).

In some cases, the opening angle is large or the beam spreads more when the frequency of the radiation and/or the diameter and/or aperture diameter D is/are low or decreased.

In some cases, a large opening angle and/or a small, low-frequency transducer or piezo element produces a wide beam, preferentially associated with a large insonified volume and/or a large heating or stimulated region.

In some cases, by reducing the intensity of the radiation, preferentially below $10^5$, $10^3$, 100, 10 or 1 W preferentially per cm, $cm^2$ or $cm^3$, preferentially of at least one dimension of the emitting part or piezo element or body part or region to be treated, the temperature or temperature rise is the heated region or body part or region to be treated can be maintained below $10^5$, $10^3$, 100, 70 or 60 °C.

In some cases, preferentially by using a wide beam, the radiation or ultrasound can cross or travel through or interact with a large region or body part or region, interfering or common region, preferentially larger than $10^{-10}$, $10^{-5}$, $10^{-3}$, $10^{-1}$, 0, 1, 2, 5 10 or $10^3$ mm or cm, preferentially allowing mild heating, preferentially below or by less than $10^5$, $10^3$, 100, 70 or 60, 50, 10 °C, in some cases relatively to the physiological temperature, and/or uniform heating, preferentially with a variation in temperature of less than $10^5$, $10^3$, 100, 50, 10, 5 or 0.1 °C, preferentially within the heated region or body part or region to be treated preferentially per cm, $cm^2$ or $cm^3$, or mild mechanical stimulation, preferentially a displacement, movement or oscillation of the nanoparticle of less than $10^9$, $10^6$, $10^3$ or 1 nm, preferentially over a broad region or a region of size or diameter or volume larger than $10^{-5}$, $10^{-3}$, $10^{-1}$, 0, 1, 2 5 or 10 cm or $cm^2$ or $cm^3$.

In some cases, the ultrasound beam is associated with or has or comprises or produces mechanical or thermal stimulation or increase preferentially in the common or interference or nanoparticle region or body part to be treated.

In some cases, in a first configuration, for at least one, two, three beam(s) of radiation, preferentially for an individual beam of radiation, with preferentially opening half-angle(s) of 2 ° or smaller than 5 or 10 °, preferentially converging, with beam radius preferentially at more than 0.1 cm or at 3 cm or at less than 100 or 10 cm preferentially from the surface of the emitting part or piezo element preferentially smaller than 100, 10, 5 or 2 mm, a cross section intensity preferentially larger than 0.01, 0.1 or 1 $W/cm^2$, the increase of temperature, preferentially in or of the emission region, interfering or common region or region between or outside the emission and/or interfering or common region, is preferentially larger than 0.1 or 1 °C.

In some other cases, in a second configuration, for at least one, two, three beam(s) of radiation, preferentially diverging, with preferentially opening half-angle(s) of 8 ° or larger than 0.1, 1 or 5 ° or larger than in a first configuration, beam radius preferentially at more than 0.1 cm or at 3 cm or at less than 100 or 10 cm preferentially from the surface of the emitting part or piezo element preferentially larger than $10^{-5}$, 0.1, 1 or 2 mm or larger than in a first configuration, a cross section intensity preferentially smaller than 100, 10, 1 or 0.1 $W/cm^2$ or smaller than in the first configuration, the increase of temperature, preferentially in or of the interfering or common region, is preferentially smaller than $10^4$, $10^3$, 100, 10, 5, 1 or 0.5 °C or smaller than in the first configuration.

In still some other cases, for at least n beams of radiation, preferentially diverging, overlapping or interfering in the common or interfering or far field or Fraunhofer region, the resulting increase of temperature in the overlapping or interfering region is larger than the increase of temperature obtained in the first or second configuration preferentially for a single beam of radiation or is larger than the increase of temperature of at least one region outside the overlapping or interfering region or of the focal or near-field region, preferentially by at least $10^{-5}$, $10^{-3}$, 0.1, 0, 1, 5 or 10 °C.

In some cases, the opening angle is increased or the radiation source is chosen to have a large opening angle or has a large angle, preferentially above $10^{-20}$, $10^{-10}$, $10^{-5}$, $10^{-3}$, $10^{-1}$ 0, 1, 2, 5, 10, 25 preferentially degree or radian, preferentially to increase or result in the size or length or diameter of the heated region, preferentially larger than $10^{-20}$, $10^{-10}$, $10^{-5}$, $10^{-3}$, $10^{-10}$, 1, 2, 5, 10, 25 m, cm or mm or nm, preferentially to decrease the temperature increase produced by at least one transducer or piezo element, preferentially below $10^5$, $10^3$, 100, 5, 2, 1, 0, $10^{-1}$ or $10^{-5}$ °C.

In some cases, the common or interference region is the region where at least two beams 1 and 2, preferentially emitted by two different sources of radiation, interfere or overlap, preferentially resulting in a third beam 3.

In some other cases, the common or interference region is the region where i individual beams (1, 2 or 3 < i < n), preferentially emitted by i difference sources of radiations, interfere or overlap, preferentially resulting in a m beam, which is preferentially a superposition of the different i individual beams, preferentially in the interference or common region or far-field region of at least one individual source of radiation or near field region of the assembly of at least two sources of radiation, where n is preferentially in some cases larger than 3, 5, 10, 50 and in some other cases smaller than $10^{10}$, $10^5$ or $10^3$.

In some cases, the third beam 3 or beam m has at least one of its parameters that differ(s), is larger or smaller, from at least one parameter of beam(s) 1, 2 and/or i.

In some cases, the region A is the focal point or region of the radiation or source of radiation or the region where the energy, power, intensity of the radiation is the largest or the most uniform or where the radiation is the most convergent or where the radiation beam width is the narrowest or the radiation is the most convergent.

In some cases, region A can be subdivided in region A1 and A2.

In some cases, region A1 is preferentially comprised in the emission or focal region or near the emitting part or piezo element of the emission or near field region of preferentially at least one individual source of radiation, preferentially less than $10^{10}$, $10^5$, $10^3$, 100, 50, 20, 10 or 5 cm away from the emitting part or piezo element.

It is some other cases, region A2 is preferentially comprised in the near field region preferentially of the assembly of at least two sources of radiation or in the far field region of at least one individual source of radiation, preferentially more than $10^{-10}$, $10^{-5}$, $10^{-3}$, 0.1, 0, 1 or 2 cm away from the emitting part or piezo element.

In some other cases, the near-field length or Fresnel distance of the assembly of at least two sources of radiations is NFL1, where preferentially the near field region extends up to NFL1 preferentially from at least one location of the support, radiation source, preferentially its center, bottom or upper part, where NFL1 = $D1^2.f/4c$ or NFL1 = $D1^2/41$ or NFL1 is proportional to f, D1, an/or $D1^2$ and/or inversely proportional to 1, where D1 is preferentially the diameter of support comprising the at least two sources of radiation or their emitting or piezo element(s), c is preferentially the speed of radiation or sound in the medium, preferentially c ≈ 1540 m/s in tissue, f is preferentially the frequency of radiation or ultrasound, 1 is preferentially the wavelength of radiation or ultrasound.

In some other cases, the region B is the near field region, preferentially of at least one individual radiation source. It preferentially comprises at least one region comprised between the emitting part or piezo element of the at least one individual source of radiation or transducer or piezo element and a distance, preferentially called the near-field length or Fresnel distance NFL2 preferentially of an individual radiation source, where preferentially NFL2 = D2$^2$.f/4c or NFL2 = D$^2$/4l or NFL2 is proportional to f, D, an/or D$^2$ and/or inversely proportional to 1, where D2 is preferentially the diameter of the emitting part or piezo element of the source of radiation or transducer or piezo element, c is preferentially the speed of radiation or sound in the medium, preferentially c ≈ 1540 m/s in tissue, f is preferentially the frequency of radiation or ultrasound, l is referentially the wavelength of radiation or ultrasound. It preferentially comprises at least one region comprised between the emitting part or piezo element of the at least one individual source of radiation or transducer or piezo element and at least one common region, the interference or common region and/or body part or region to be treated or heated. In some cases, the emitting part or piezo element of the source of radiation can be an antenna, in part or in full.

In some cases, NFL1 and/or NFL2 is smaller than $10^{20}$, $10^{10}$, $10^5$, $10^3$ cm, preferentially smaller than 100, 50, 10, 5, 2 or 1 cm.

In some cases, NFL1 and/or NFL2 is larger than $10^{-20}$, $10^{-10}$, $10^{-5}$ or $10^{-3}$, 0, 1, 2 or 5 cm.

In some other cases, NFL1 is larger than NFL2, preferentially by a factor of at least 1, 1.1, 2, 5 or 10 or by at least $10^{-20}$, $10^{-10}$, $10^{-5}$ or $10^{-3}$, $10^{-1}$, 1, 2 or 5 cm.

In still some other cases, the region C is the far field or Fraunhofer region, preferentially of at least one individual radiation source or is the near field or Fresnel region, preferentially of the assembly of at least two radiation sources. It preferentially comprises, preferentially partly or fully or predominantly, at least one region comprising at least one common or interference region and/or body part or region to be treated or heated. Preferentially, it is comprised, preferentially partly or predominantly or fully, at a distance larger than NFL2. Preferentially, it is comprised, preferentially partly or predominantly or fully, at a distance smaller than NFL1.

In some cases, the region C is the region, where the at least one or each individual beam of radiation preferentially emitted by an individual source of radiation diverges smoothly and/or predictably, and/or has a stable profile, and/or produces pressure waves that resemble planar waves.

In one embodiment of the invention, the at least one nanoparticle is comprised in at least one region selected in the group consisting of: the region A, the region B and the region C.

Preferentially, the at least one nanoparticle is comprised, preferentially partly or predominantly or fully, in at least one region, preferentially in the region C.

The invention also relates to a radiation heating system for treating a body part or region of an individual comprising:

- A first part comprising at least two or three different sources of radiation emitting at least two or three different beams of radiation, preferentially orientated in the direction of the body part or region to be treated, said at least two or three beams spatially overlapping within the far field or Fraunhofer region of at least one individual or each source of radiation and/or in the near field region of the assembly of at least two sources of radiation;
- A second part comprising at least one nanoparticle comprised the far field or Fraunhofer region of at least one individual or each source of radiation and/or in the near field region of the assembly of at least two sources of radiation.

In some cases, the far field or Fraunhofer region of at least one individual or each source of radiation is or comprised the near field region of the assembly of at least two sources of radiation.

In some cases, the near field region of the assembly of at least two sources of radiation is or comprises the far field or Fraunhofer region of at least one individual or each source of radiation.

In some cases, a beam of radiation can have at least one orientation in the direction of the body part or region to be treated when it crosses or interferes or overlaps, preferentially with at least at least one other beam of radiation in the body part or region to be treated, partly or fully.

In some cases, the source of radiation or beam of radiation or nanoparticle can be or can be characterized by or generates at least one parameter, preferentially selected in the group consisting of: beam or nanoparticle or source of radiation energy, spreading or opening or diffracting or angle, incident angle preferentially relative to at least one axis crossing the body part or region to be treated, wavelength, frequency, intensity, flux, dose rate, total dose, energy deposited in matter, direction, divergence, spatial profile, shape, width, radius, diameter, coherence, penetration depth, temporal coherence, spatial coherence, polarization, extinction coefficient, particle type, quality, hardness, temporal structure such as continuous or pulsed one, pulsed duration or repetition rate, emittance, spread preferentially in position and/or momentum space, peak positive pressure (PPP), peak negative or rarefactional pressure (PNP), Mechanical Index (MI), spatial-peak temporal-average intensity, spatial-peak pulse-average intensity, spatial-average temporal-average, radiation Force, displacement or oscillation or movement or strain preferentially of at least one nanoparticle, acoustic pressure field, absence of cavitation and presence of cavitation.

In one embodiment of the invention, the at least one parameter of the beam is larger than $10^{-20}$, $10^{-5}$, $10^{-1}$, 0, 1, 2, 5, 10, 90, 180, 360, $10^3$, $10^5$, $10^{10}$ or $10^{20}$,

preferentially in some cases with a positive sign, preferentially in some other cases with a negative sign, preferentially in some cases without unit, preferentially in some other cases with at least one unit preferentially selected in the group consisting of: with or without eV, keV, MeV, m, cm, mm, nm, Å, Watt (W), $W/m^2$, particles/$cm^2$/s or photons/$cm^2$/s, Gray (Gy), Gy/min or Gy/s, degrees (°) or radians (rad), seconds (s), ms, $\mu$s, ns, fs, species such as electrons, protons, e$^-$, p$^+$, mm of at least one chemical element such as Al, Cu, Hz, KHz, MHz, Joules (J), mm·mrad, m·rad, Pa, MPa, Newton (N), Volt (V) and %,

In another embodiment, the at least one parameter is smaller than $10^{20}$, $10^{10}$, $10^5$, $10^3$, 360, 180, 90, 45, 20, 10, 5, 2, 1, 0, $10^{-1}$, $10^{-3}$, $10^{-5}$, $10^{-10}$ or $10^{-20}$, preferentially in some cases with a positive sign, preferentially in some other cases with a negative sign, preferentially in some cases without unit, preferentially in some other cases with at least one unit preferentially selected in the group consisting of: with or without eV, keV, MeV, m, cm, mm, nm, Å, Watt (W), $W/m^2$, particles/$cm^2$/s or photons/$cm^2$/s, Gray (Gy), Gy/min or Gy/s, degrees (°) or radians (rad), seconds (s), ms, $\mu$s, ns, fs, species such as electrons, positrons, protons, antiprotons, alpha particles, muons, pions, deuterons, carbon ions, heavy ions, neutrons, photons, neutrinos, beta particles, gamma rays, X-ray photons, mm of at least one chemical element such as Al, Cu, Hz, KHz, MHz, Joules (J), mm·mrad and m·rad, Pa, MPa, Newton (N), Volt (V) and %.

In one embodiment, the at least one chemical element, preferentially comprised in the nanoparticle and/or beam of radiation or crossed by or surrounding the beam of radiation and/or nanoparticle, is selected in the group consisting of: Hydrogen, Helium, Lithium, Beryllium, Boron, Carbon, Nitrogen, Oxygen, Fluorine, Neon, Sodium, Magnesium, Aluminum, Silicon, Phosphorus, Sulfur, Chlorine, Argon, Potassium, Calcium, Scandium, Titanium, Vanadium, Chromium, Manganese, Iron, Cobalt, Nickel, Copper, Zinc, Gallium, Germanium, Arsenic, Selenium, Bromine, Krypton, Rubidium, Strontium, Yttrium, Zirconium, Niobium, Molybdenum, Technetium, Ruthenium, Rhodium, Palladium, Silver, Cadmium, Indium, Tin, Antimony, Tellurium, Iodine, Xenon, Cesium, Barium, Lanthanum, Cerium, Praseodymium, Neodymium, Promethium, Samarium, Europium, Gadolinium, Terbium, Dysprosium, Holmium, Erbium, Thulium, Ytterbium, Lutetium, Hafnium, Tantalum, Tungsten, Rhenium, Osmium, Iridium, Platinum, Gold, Mercury, Thallium, Lead, Bismuth, Polonium, Astatine, Radon, Francium, Radium, Actinium, Thorium, Protactinium, Uranium, Neptunium, Plutonium, Americium, Curium, Berkelium, Californium, Einsteinium, Fermium, Mendelevium, Nobelium, Lawrencium, Rutherfordium, Dubnium, Seaborgium, Bohrium, Hassium, Meitnerium, Darmstadtium, Roentgenium, Copernicium, Nihonium, Flerovium, Moscovium, Livermorium, Tennessine, and Oganesson.

The invention also relates to a radiation heating system for treating a body part of an individual comprising:

- A first part comprising at least one source of radiation or different sources of radiation, where each source preferentially emits at least one beam of radiation:

  wherein each radiation source is preferentially positioned at a different location preferentially relatively to a horizontal support axis preferentially of the system, such that preferentially the radial or angular positions of the sources around or relatively to said axis are different,
  wherein at least two different radiation sources have preferentially at least two different orientations preferentially of their emitting part or piezo element, surface or beam, defined by at least two different emission angles preferentially measured with or with respect to a common reference axis, and
  wherein said sources of radiation are preferentially positioned and orientated so that the beams spatially intersect or overlap preferentially within a common or interference region preferentially distinct from the emission or focal or Near-field region of at least one individual emitting or piezo element and/or is comprised in the near-field region of the assembly of emitting or piezo elements preferentially comprised in the system or at least one transducer or source of radiation of the system,
  wherein the number of sources of radiation is preferentially at least 3, 4, 5, 10, 20, 50, 60, 70, 80, and preferentially at most $10^{20}$, $10^3$, or 100,
  wherein each source of radiation is preferentially configured to have at least one property selected in the group consisting of:

    i) an opening or diffracting angle (T) preferentially of the beam emitted by the source of radiation larger than 0, 1, 2, 5, 25 or 45°, preferentially measured between two opposite directions or points of the beam perimeter preferentially at the level of the emission or focal or near field region,
    ii) a diameter or width or thickness or its largest dimension smaller than one half the diameter or width or thickness of the body part to be treated or body part into or onto which the system or at least one part of the system is positioned or inserted or than 100, 10, 1 or 0.5 cm, and
    iii) a .frequency preferentially of the beam that it emits that is smaller than 100, 10, 5, 2 or 1.5 MHz,

- A second part comprising at least one nanoparticle, preferentially a magnetic or metallic nanoparticle, preferentially located or comprised in said common or interference region and/or exposed to the intersecting or overlapping beams of radiation.

The invention also relates to the system according to the invention, where the at least one nanoparticle or the system preferentially is not or does not comprise or does not operate through the generation or does not have the function of generating at least 1, 2, 5, 10 or 100 nanobubble(s), bubble(s), or microbubble(s).

The invention also relates to the system according to the invention, which does not comprise at least one fluorophore.

In some cases, the source of radiation or transducer or piezo element comprises a number larger than or equal to 0, 1, 2, 3, 4, 5, 6, 7, 8, 9, 10, 25, 32, 64, 128 or 256 piezo or piezo element(s).

In some other cases, the source of radiation or transducer or piezo element comprises a number smaller than or equal to 1000, 500, 256, 128, 64, 32, 10, 5, 2 or 1 piezo or piezo element(s).

In one embodiment of the invention, the at least 1 piezo element emits one beam of radiation.

In another embodiment of the invention, the source of radiation or transducer or piezo element emits a number of beams of radiation equal to the number of piezo elements that it comprises.

In still some cases, the source of radiation or transducer or piezo element emits a number of radiation beams larger than or equal to 0, 1, 2, 3, 4, 5, 6, 7, 8, 9, 10, 25, 32, 64, 128 or 256 piezo or piezo element(s).

In some other cases, the source of radiation or transducer or piezo element emits a number of radiation beams smaller than or equal to 1000, 500, 256, 128, 64, 32, 10, 5, 2 or 1 piezo or piezo element(s).

In some cases, the number of piezo elements per transducer or piezo element is reduced, preferentially lower than for HIFU, preferentially to increase the size of the heated region.

In some cases, the radiation is ultrasound.

In another embodiment of the invention, the source of radiation or transducer or emitting part comprises at least one component selected in the list consisting of: transducer, piezoelectric element, backing layer, layer that absorbs preferentially backward radiation preferentially acoustic waves, matching layer preferentially for impedance adaptation, protective or acoustic window, electrode, cabling, electrical connection, housing, casing, lens preferentially radiation or Acoustic lens preferentially concave or convex, element for beam shaping, damping material preferentially for internal radiation absorption, integrated electronic, preamp, multiplexer, and filter.

In some cases, the system comprises a lens or support preferentially curved preferentially in the direction of the region to be treated preferentially concave preferentially on two which the emitting or piezo element(s) are attached or the system comprises at least two sources of radiations emitting at least two beams B1 and B2, where B2 is preferentially father away from the center of the body part to be treated than B1 and B2 is preferentially emitted before B1, to preferentially enable the convergence or interference or overlapping or superposition of several beams emitted by different sources of radiation or emitting or piezo elements or to rectify or compensate the diffracting effect of at least one source of radiation or emitting or piezo element preferentially in the region to be treated or far-field region that is preferentially different from the focal region preferentially of at least one source of radiation or emitting or piezo element.

In some other cases, the system comprises a lens or support preferentially curved preferentially in the opposite direction to that of the region to be treated preferentially convex preferentially on two which the piezo element(s) or emitting part(s) are attached or the system comprises at least two sources of radiations emitting at least two beams B3 and B4, where B3 is preferentially father away from the center of the body part to be treated than B4 and B3 is preferentially emitted after B1, to preferentially enable the divergence or prevent the interference or overlapping or superposition of several beams emitted by different sources of radiation or emitting or piezo element(s) or to accentuate the diffracting effect of at least one source of radiation or emitting or piezo element preferentially in the emission region or near field region or focal region preferentially of at least one source of radiation or emitting or piezo element. In some cases, the emitting parts can be designated as emitting elements.

In some other cases, the concave lens of concave support acts as beam collimator or concentrator, preferentially in the region to be treated or far-field region preferentially to rectify or compensate from the beam spreading preferentially arising from the diffracting effect preferentially of at least one piezo element or emitting part.

In another embodiment of the invention, the source of radiation or transducer or emitting part comprises at least one component selected in the list group consisting of: Piezoelectric ceramics, preferentially comprising Lead zirconate titanate, Barium titanate, Lead titanate, Lithium niobate, Lithium tantalate, Single-crystal piezo materials, preferentially comprising Lead magnesium niobate-lead titanate, Lead zinc niobate-lead titanate, Silicon dioxide, quartz, Piezoelectric polymer, preferentially comprising Polyvinylidene fluoride, Polyvinylidene fluoride-trifluoroethylene copolymer, Piezoelectric composite, preferentially comprising One-three composite or ceramic rods embedded in a polymer, Two-two composite or layered ceramic and polymer structure, Three-three composite or interconnected ceramic and polymer network, Micro-Electro-Mechanical Systems and emerging piezo materials, Aluminum nitride, Zinc oxide, Gallium orthophosphate, and Biological piezoelectric materials.

In another embodiment of the invention, the source of radiation or transducer or emitting part comprises at least one shape selected in the list group consisting of: Disk, circular, Square plate, Rectangular plate, Ring, annular shape, Solid cylinder, Hollow cylinder, tube, Spherical shell, Hemispherical element, Bar or rod, Unimorph strip, Bimorph strip, Hexagonal or other polygonal shapes, Composite structured elements, Micro-Electro-Mechanical Systems thin-film diaphragms or membranes.

In some cases, the support preferentially of the system can be or comprise at least one or two or four transducer(s).

In another embodiment of the invention, the source of radiation or transducer or emitting part comprises at least one shape selected in the list group consisting of: Flat or planar transducer, spherically curved front transducer preferentially Focused, Cylindrical or curved in one dimension transducer, Spherically focused transducer, Concave transducer, Convex transducer, Phased-array transducer, Linear-array transducer, Curvilinear preferentially convex array, Sector-scan preferentially phased array, Endocavity transducer, Annular array transducer, Ring transducer, Tube or cylindrical transducer, Spherical transducer, Hemispherical transducer, Horn-loaded transducer, Immersion transducer, Contact transducer, Dual-element transducer, Angle-beam transducer.

In some cases, the shape of the transducer is the same as the shape of the piezo element.

In some other cases, the shape of the transducer is different from the shape of the piezo element.

In some cases, the piezo element is focused, preferentially its shape is curved preferentially to focus the beam it generates, for example it has a Spherical curvature preferentially leading to a focus point or it has a Cylindrical curvature leading preferentially to a focus line or it has a curved form or shape, , preferentially partly or predominantly or fully..

In some cases, the piezo element is unfocused, preferentially its shape is flat or is not curved or with a Spherical or Cylindrical curvature, preferentially partly or predominantly or fully.

In some cases, the transducer is focused, preferentially by comprising a lens, preferentially an a radiation or acoustic lens, a curved housing, support, backing, fac, or electronically preferentially using phased arrays preferentially through delaying pulses preferentially of at least two different piezo elements.

In some cases, the piezo element is focused and the transducer is focused.

In some cases, the piezo element is focused and the transducer is unfocused.

In some cases, the piezo element is unfocused and the transducer is focused.

In some cases, the piezo element is unfocused and the transducer is unfocused.

The invention further relates to an interfering system preferentially for treating a body part or region of an individual preferentially through or by the interference of at least two beams of radiation preferentially in an interference region preferentially comprising at least one nanoparticle, where the system comprises two parts:

- A first part comprising at least one or two different sources of radiation preferentially emitting at least two different beams of radiation,

> wherein the at least two different sources of radiation preferentially comprise or emit or are associated with at least two different beams of radiation,
> wherein the at least two different sources and/or beams of radiation preferentially have a different location, orientation and/or are of different types,
> wherein the at least two different sources of radiation are preferentially selected in the group consisting of: a spire, a conductor or emitter of current, electromagnetic wave or radiation, a probe, an optical fiber, and a transducer or piezo element, whether in full or in part,
> where the at least two different sources of radiation preferentially have at least one property selected in the group consisting of:

> > i) they are arranged so that the at least two beams of radiation interfere in an interference region, where the interference region is preferentially distinct or separate, preferably by at least 1 nm, from the emission region;
> > ii) they are positioned in at least two different locations, such that:

> > - The at least two sources of radiation comprise at least two different inclinations or form at least two different angles, preferably with respect to an axis that passes through the part of the body of the individual to be treated, a horizontal or vertical axis,
> > - The at least two sources of radiation are located in at least two distinct or opposite locations preferentially within a geometric figure,

> > iii) they are present in sufficiently large number or are characterized by a sufficiently large number of different inclinations or orientations or incidence angle with respect to an axis crossing the body part or region to be treated, where this number is preferentially larger than or equal to 2, 4, 5, 10, 20, 50, or 100, where such large number preferentially enables: a) lowering the intensity of the radiation emitted by each source of radiation, b) avoiding heating the region between the emission and interference regions, preferentially by more than $10^{-20}$, $10^{-5}$, $10^{-3}$, $10^{-1}$, 0, 0.5, 1, or 10°C, and/or c) obtaining homogeneity in the distribution of the radiation beams or the temperature in the interference region,

iv) they are present in a number that is lower than or equal to $10^{20}$, $10^3$, or 100 (without unit or in cm), where such limited number preferentially avoids: a) cluttering or excessive size of the system, b) excessive superimposition of different beams of radiation in the interference region which can cause overheating,

where the at least two beams of radiation have at least one property selected from the group consisting of:

i) They are oriented so as to:

- Minimize interference between the at least two beams of radiation outside the interference region, and/or
- Maximize interference between the at least two beams of radiation in the interference region, and

ii) They have at least two different axis or direction of propagation preferentially oriented in the direction of the interference region, preferentially from a different or opposite angle, preferentially relatively to the axis of elongation of the first part or to the perpendicular to this axis;

- A second part comprising at least one nanoparticle, preferentially a magnetic or metallic nanoparticle,

wherein the first part and the second part are preferentially linked to each other or characterized by at least one property selected from the group consisting of:

i) The first and second parts are separated from each other preferentially by a larger distance than 1 nm;
ii) The second part is exposed preferentially simultaneously or successively to at least one or two distinct beams of radiation preferentially emitted by the first part;
iii) The second part occupies a smaller volume than the first part, preferentially by a factor of at least $10^{-1}$, 1.1, 2, 5 or 10.
iv) The second part is a contrasting agent preferentially for the beam of radiation of or emitted by the first part;
v) The second part reflects, absorbs or transmits at least two beams of radiation of or emitted by the first part, where such mechanism preferentially occurs, preferentially at its maximum or optimal strength, at different locations of the second part for the at least two beams of radiation;
vi) The second part is moved or heated or transported or internalized or brought into interaction with at least one cell or biological material by the first part;

where the at least one nanoparticle or the system or at least one part of the system is preferentially not or preferentially does not comprise or preferentially does not generate or preferentially does not have the function of generating at least 1, 2, 5, 10 or 100 nanobubble(s), bubble(s), or microbubble(s),
where preferentially the at least one or two beam(s) or radiation preferentially does/do not narrow preferentially by more than 0, 1, 5, 10, 50, 75, 80, 90, 99 or 100% or half preferentially between the emission region and the interference region,
where preferentially the absence of significant beam narrowing is preferably desirable for the at least one or two beam(s) or radiation to be able to heat a sufficiently large interference region preferentially homogeneously,
where preferentially the system in part or in full preferentially does not comprise at least one fluorophore,
wherein optionally the system further comprises at least one cooling part such as a cooling balloon that is preferentially positioned above or below or at the surface of the at least two sources of radiations that is preferentially maintained at a temperature that is below 100, 50, 40, 37, 25, 20, 15, 10, 5, 2, 1 or 0°C or below the temperature of the at least two sources or beams of radiations preferentially by at least 100, 50, 40, 37, 25, 20, 15, 10, 5, 2, 1 or 0°C;
wherein optionally the system further comprises at least one transmitting part, where the transmitting part comprises a material that transmits or allows the propagation of at least one beam of radiation such as a gel;
wherein optionally the system further comprises at least one thermometer or equipment measuring the temperature directly or indirectly such as a thermocouple that is preferentially connected or linked to the first part and enables to adjust at least one parameter of the first part, preferentially its intensity or frequency, preferentially to maintain or adjust the temperature of the body part or region to be treated at the desired temperature;

wherein optionally at least one parameter of the first part such as the intensity of the radiation is sufficiently large, preferably larger than $10^{-20}$, 0.1, 0, 1, 5, 10, 100 or $10^3$ preferentially W or W per cm or $cm^2$ or $cm^3$, preferentially of source of radiation or of emitting part or piezo element of source of radiation or of body part or region to be treated, preferentially to enable the at least one or two beams of radiation to heat sufficiently, preferentially to enable the body part or region to be treated to reach the desired temperature;

wherein optionally at least one parameter of the first part such as the frequency of the radiation is sufficiently large, preferably larger than $10^{-20}$, 0.1, 0, 1, 5, 10, 100 or $10^3$ H or KH or MH (Herz), preferentially to enable the at least two beams of radiation to be localized in a sufficiently small region, preferentially a region of size lower than $10^{10}$, $10^5$, $10^3$, 100, 50, 10, 5, 2, 1, 0 or $10^{-1}$ m or lower than 100, 99, 90, 80, 70, 50, 40, 25, 10, 5, 2, or 1% preferentially in mass or volume of the body part or region to be treated;

wherein optionally at least one parameter of the first part such as the intensity of the radiation is sufficiently small, preferentially smaller than $10^{20}$, $10^{10}$, $10^5$, $10^3$, 100, 50, 10, 5, 2, 1, $0.10^{-1}$, or $10^{-3}$ preferentially W or W per cm or $cm^2$ or $cm^3$, preferentially of source of radiation or of emitting part or piezo element of source of radiation or of body part or region to be treated, preferentially to avoid that the at least one or two beams of radiation overheat, preferentially the body part or region to be treated, preferentially to avoid cavitation such as inertial cavitation;

wherein optionally at least one parameter of the first part such as the frequency of the radiation is sufficiently small, preferentially smaller than $10^{20}$, $10^{10}$, $10^5$, $10^3$, 100, 50, 10, 5, 2, 1, $0.10^{-1}$, or $10^{-3}$ H or KH or MH (Herz), preferentially to enable the at least two beams of radiation to be localized in a sufficiently large region, preferentially a region of size larger than $10^{-20}$, 0.1, 0, 1, 5, 10, 100 or $10^3$ m or cm or mm or larger than $10^{-10}$, $10^{-5}$, $10^{-1}$, 0, 1, 5, 10, 25, 50 99, 100, 200, 500 or $10^3$% preferentially in mass or volume of the body part or region to be treated;

wherein optionally at least one parameter of the second part such as the nanoparticle concentration is or is kept sufficiently small, preferentially smaller than $10^{10}$, $10^5$, $10^3$, 100, 10, 5, 2, 1, 0.1 gram or milligram preferentially per meter or cm preferentially of body part or region to be treated, preferentially to avoid that the presence of too concentrated nanoparticles in the interference region increase the temperature of the body part or region to be treated preferentially by more than 0, $10^{-5}$, $10^{-1}$, 1, 5, 10, or $10^3$ °C preferentially per second or minute or hour or day preferentially above the temperature of or generated by the at least two beams of radiation preferentially in the interference region;

wherein optionally at least one parameter of the second part such as the nanoparticle concentration is or is kept sufficiently large, preferentially larger than $10^{-10}$, $10^{-5}$, $10^{-3}$, 0, 1, 5, or 10 gram or milligram preferentially per meter or cm preferentially of body part or region to be treated, preferentially to enable that the presence of sufficiently concentrated nanoparticles in the interference region enables the at least two beams of radiation to interfere in a region that comprises at least one nanoparticle;

wherein optionally the desired temperature has at least one property selected in the group consisting of: i) it is below the temperature of ablation or the temperature or maximum temperature reached during a method of thermal ablation such as high intensity focused ultrasound or radiofrequency ablation, ii) it is below $10^5$, $10^3$, 100 or 75 °C, iii) it is below the temperature that causes coagulative necrosis, iv) it is above physiological temperature preferentially by at least 0, 1 or 10 °C, v) it is above 5, 10, 20, 25, 30, 35, 37, 40, 41, 42, 43, 44, 45, 50, 55 or 60 °C, vi) it is increased, preferentially during a first period of time, preferentially for a duration smaller than $10^5$, $10^3$, 100, 50, 20, 10, 5, 2 or 1 second(s) or minute(s), preferentially by being varied preferentially by more than $10^{-20}$, $10^{-5}$, $10^{-3}$, $10^{-1}$, 0, 1, 5, 10 °C preferentially per second or cm or $cm^2$ or $cm^3$, preferentially of body part or region to be treated, and vii) it is maintained preferentially during a second period of time, preferentially for a duration longer than 0, 1, 5, 10, 100, 600 or 1000 second(s) or minute(s), preferentially by being varied preferentially by less than 100, 50, 20, 10, 5, 2 or 1 °C preferentially per second or cm or $cm^2$ or $cm^3$, preferentially of body part or region to be treated.

The invention further relates to the system according to the invention, wherein the at least one nanoparticle has at least one property selected in the group consisting of:

- it forms an assembly of at least two nanoparticles, where the at least two nanoparticles have at least one property selected in the group consisting of: i) they are arranged in a chain, ii) they are arranged in a geometric figure, iii) they are linked or associated to each other, preferentially through binding or coating material, iv) they are separated by less than 10, 1, 0.1 $\mu$m or than the diameter of at least one nanoparticle, v) they have a larger absorption, transmission, and/or reflection of the radiation beam emitted by the first part than a single nanoparticle, vi) they have a larger movement or oscillation or interaction or internalization with or in cells or biological material preferentially under the application of the radiation beams emitted by the first part than a single nanoparticle, vii) they heat more, preferentially by at least 0, $10^{20}$, $10^{-5}$, $10^{-3}$, or $10^{-1}$ °C than a single nanoparticle;

- it has at least one magnetic property, preferentially selected in the group consisting of: diamagnetic, paramagnetic, superparamagnetic, ferrimagnetic and ferromagnetic property;
- it comprises at least 1, 2, 5, 10, 100 or $10^3$ metallic atom(s) or more than 1, 5, 10, 50, 75, 80, 90 or 99% in mass or volume of metallic atom(s);
- it is formed before, during or after or results from the application of the at least one or two radiation beam(s) of the first part;
- it heats locally at the nanoparticle or nm scale, preferentially without resulting in a significant temperature increase of the body part or region to be treated, preferentially by more than 0.1, 1 or 10 °C, preferentially under the application of the at least one or two radiation beam(s) of the first part;
- it has a size, preferentially individual or collective, that is smaller than $10^{10}$, $10^5$, $10^4$, $10^3$, $10^2$, 10, 5, 2 or 1 nm, preferentially in at least one, two or three dimension(s);
- it has a size, preferentially individual or collective, that is larger than $10^{-3}$, $10^{-2}$, $10^{-1}$, 0, 1, 2, 5, 10, 20, 50, 100, $10^3$ or $10^5$ nm, preferentially in at least one, two or three dimension(s);

The invention further relates to the system according to the invention, which is a system of double interference characterized by:

- At least two different sources of radiations of the first part emit at least two different beams of radiation that interfere according to a first type of interference by producing or being associated with or comprising a superposition partly or fully of the at least two beams of radiation or a coexistence preferentially without superposition partly or fully of the at least two beams of radiation preferentially in the interference or common region,

    where the superposition of at least two beams of radiation is preferentially the existence or persistence of at least two beams of radiation at the same location of the interference or common region;
    where the coexistence without superposition of at least two beams of radiation is preferentially the existence or persistence of the at least two beams of radiation in at least two different locations of the interference or common region;

- At least one beam of radiation of the first part emits at least one beam of radiation that interferes with at least one nanoparticle of the second part according to the second type of interference by producing or being associated with or comprising:

    ○ the absorption, transmission, reflection, of the at least one radiation beam by the at least one nanoparticle; and/or

    ○ the movement or oscillation of the at least one nanoparticles, and/or the interaction or internalization of the at least one nanoparticle by at least one cell or biological material;

The invention further relates to the system according to the invention, wherein the at least two different source(s) of radiation emit at least two different radiations or beams of radiation, characterized by at least one property selected in the group consisting of:

a) At least two different beams of radiation have at least two different values of parameters selected in the group consisting of: orientations or directions of propagation or angles between their direction of propagation and the horizontal or vertical axis ($O1$, $O2$), intensity ($I_1$, $I_2$), power ($P_1$, $P_2$), energy ($E_1$, $E_2$), width ($W_1$, $W_2$), depth of penetration ($D_1$, $D_2$), frequency ($F_1$, $F_2$), and wavelength ($W_1$, $W_2$);
And
b) The at least two radiations are of at least two different types of radiations;

    wherein the first beam as at least one parameter selected in the group consisting of: $O1$, $I_1$, $P_1$, $E_1$, $W_1$, $D_1$, $F_1$ and $W_1$;
    wherein the second beam as at least one parameter selected in the group consisting of: $O_2$, $I_2$, $P_2$, $E_2$, $W_2$, $D_2$, $F_2$ and $W_2$;
    wherein $(O_1-O_2)/(O_1+O_2)$, $(I_1-I_2)/(I_1+I_2)$, $(I_1-I_2)/I_1$, $(I_1-I_2)/I_2$, $(P_1-P_2)/(P_1+P_2)$, $(P_1-P_2)/P_1$, $(P_1-P_2)/P_2$, $(E_1-E_2)/(E_1+E_2)$, $(E_1-E_2)/E_1$, $(E_1-E_2)/E_2$, $(D_1-D_2)/(D_1+D_2)$, $(D_1-D_2)/D_1$, $(D_1-D_2)/D_2$, $(F_1-F_2)/(F_1+F_2)$, $(F_1-F_2)/F_1$, $(F_1-F_2)/F_2$, $(W_1-W_2)/(W_1+W_2)$, $(W_1-W_2)/W_1$, and/or $(W_1-W_2)/W_2$, in absolute value or not, is/are larger than 0, $10^{-5}$, $10^{-3}$, $10^{-1}$, 1, 5, 10, 50, 75, 100 or $10^4$ preferentially %,
    wherein the at least one parameter is preferentially measured or exists in at least one region selected in the group consisting of:

        a) At least one emission region,
        b) At least one interference or common region,
        and
        c) At least one region separating at least one emission region and at least one interference region.

The invention further relates to the system according to the invention, wherein the at least two different source(s) of radiation emit at least two different radiations or beams of radiation, preferably characterized by at least one property selected in the group consisting of:

a) At least two different beams of radiation have at least two similar or same values of parameters selected in the group consisting of: orientations or directions of propagation or angles between their direction of propagation and the horizontal or vertical axis (O1, O2), intensity ($I_1$, $I_2$), power ($P_1$, $P_2$), energy ($E_1$, $E_2$), width ($W_1$, $W_2$), depth of penetration ($D_1$, $D_2$), frequency ($F_1$, $F_2$), and wavelength ($W_1$, $W_2$); and

b) The at least two radiations are of the same or similar type of radiations;

wherein the first beam as at least one parameter selected in the group consisting of: $O_1$, $I_1$, $P_1$, $E_1$, $W_1$, $D_1$, $F_1$ and $W_1$;
wherein the second beam as at least one parameter selected in the group consisting of: $O_2$, $I_2$, $P_2$, $E_2$, $W_2$, $D_2$, $F_2$ and $W_2$;
wherein $(O_1-O_2)/(O_1+O_2)$, $(I_1-I_2)/(I_1+I_2)$, $(I_1-I_2)/I_1$, $(I_1-I_2)/I_2$, $(P_1-P_2)/(P_1+P_2)$, $(P_1-P_2)/P_1$, $(P_1-P_2)/P_2$, $(E_1-E_2)/(E_1+E_2)$, $(E_1-E_2)/E_1$, $(E_1-E_2)/E_2$, $(D_1-D_2)/(D_1+D_2)$, $(D_1-D_2)/D_1$, $(D_1-D_2)/D_2$, $(F_1-F_2)/(F_1+F_2)$, $(F_1-F_2)/F_1$, $(F_1-F_2)/F_2$, $(W_1-W_2)/(W_1+W_2)$, $(W_1-W_2)/W_1$, and/or $(W_1-W_2)/W_2$, in absolute value or not, is/are smaller than $10^{20}$, $10^{10}$, $10^5$, $10^3$, 100, 75, 50, 10, 5 or 1 preferentially %,
wherein the at least one parameter is preferentially measured or exists in at least one region selected in the group consisting of:

a) At least one emission region,
b) At least one interference or common region,
And
c) At least one region separating at least one emission region and at least one interference region.

The invention further relates to the system according to the invention or its first or second part, which is partly or fully used in or administered to or applied to or comprised in the body part or region to be treated at least 1, 2, 3, 4, 5, 6, 7, 8, 9, 10 or 100 times, preferentially continuously or discontinuously, preferentially for at least 1, 2, 3, 4, 5, 6, 7, 8, 9, 10 or 100 sufficiently long period of times, preferentially of more than 0, 1, 2, 5, 10 or 100 second(s), preferentially to enable the first and/or second part(s) to heal or heat the body part or region to be treated, preferentially for at least 1, 2, 3, 4, 5, 6, 7, 8, 9, 10 or 100 sufficiently short period of times, preferentially of less than $10^{10}$, $10^5$, $10^3$, 10, 5, 2 or 1 minute(s), preferentially to avoid that the first and/or second part(s) generate(s) side effect(s).

The invention further relates to the system according to the invention, which is either used in combination with or comprises or is associated with a third part, which is an imaging element or equipment that has at least one property selected in the group consisting of:

i) it is selected in the group consisting of: X-ray radiography, Fluoroscopy, Computed Tomography (CT), Mammography, Dual-energy X-ray absorptiometry (DEXA), Magnetic Resonance Imaging (MRI), Functional MRI (fMRI), Diffusion Tensor Imaging (DTI), MR Spectroscopy, Cardiac MRI, Ultrasound imaging, Doppler ultrasound, 3D ultrasound, 4D ultrasound, Elastography, Contrast-enhanced ultrasound (CEUS), Scintigraphy, Single Photon Emission Computed Tomography (SPECT), Positron Emission Tomography (PET), PET/CT, PET/MRI, Optical Coherence Tomography (OCT), Fluorescence imaging, Bioluminescence imaging (BLI), Photoacoustic imaging, Thermography, Photoacoustic tomography (PAT), Microwave imaging, Electrical Impedance Tomography (EIT). and a scanner, in part or in full,
ii) it comprises a software or artificial intelligence, preferentially a fusion software, that preferentially enables the fusion of at least one image of the body part or region to be treated taken with at least one first equipment of i) such as MRI with at least one second equipment of i) such as Ultrasound imaging, where the first equipment is preferentially not comprised in the system and the second equipment is comprised in the system;
iii) it comprises at least one imaging element, which is located in the region that receives or absorbs or is exposed to at least one radiation or beam of radiation, which is preferentially emitted or reflected or diffracted partly or fully by the at least one nanoparticle or body part or region or interference or common region or part of it;
iv) it comprises at least two imaging elements, which are located in two different regions that receive or absorb or are exposed to at least two different radiations or two different beams of radiation, which are preferentially emitted or reflected or diffracted partly or fully by at least two different nanoparticles or two different sides or facets of at least one nanoparticle or two different portions of the body part or region or two different portions of the interference or common region;
v) it receives or absorbs or is exposed to at least one radiation or one beam of radiation,
vi) it detects or has sufficient sensitivity or has a sufficiently large number or more than one sensor or transducer or piezo element, preferentially located in different regions, to detect the difference in at least one value of at least one parameter between the at least two beams of radiation, which are preferentially emitted or reflected or diffracted partly or fully by at least two different nanoparticles or two different sides or facets of at least one nanoparticle or two different portions of the body part or region or two different portions of the interference or common region;

vii) it is comprised between or in a different region of the at least two emitting elements of the first part;

viii) it serves to detect or has sufficient imaging sensitivity or contrasting strength or ability to detect at least one entity, partly or fully, selected in the group consisting of: i) at least one nanoparticle of the second part, ii) at least one body part or region, iii) at least one interference or common region, iv) at least one emitting region, and v) at least one region located between the emitting and interference or common regions;

ix) it has an imaging sensitivity or imaging strength that is increased or enhanced by the presence of the at least one nanoparticle of the second part;

x) it serves to detect or has sufficient imaging sensitivity or contrasting strength or ability to detect the at least one beam emitted by the at least one source of radiation or the at least two different beams emitted by the at least two different beams of radiation, preferentially through the detection

The invention further relates to the system according to the invention, wherein the interference or common region and/or body part or region to be treated is/are selected in the group consisting of:

- A pathological site;
- A tumor or primary or metastatic tumor or cancer or cancer site;
- A site of infection, preferentially a site that is infected by at least one virus, bacterium, tumor cell or pathological biological material;
- A region that comprises more pathological cells than healthy cells; and
- A region that comprises at least one pathological site that is larger than at least one healthy site;

The invention further relates to the system according to the invention, wherein the interference region is larger than the body part or region to be treated, preferentially by a factor of more than $10^{-3}$, 1.1, 2, 5 or 10, where the size of the body part or region to be treated is preferentially measured by the third part of the system, where the interference region preferentially comprises at least 1 or 2 common regions or the sum of the common regions or is larger than at least one common region.

The invention further relates to the system according to the invention, wherein the source of radiation is selected in the group consisting of: i) source of X-ray such as X-ray tube, ii) source of radioisotopes, iii) accelerator such as linear accelerators (LINAC), iv) source of Gamma ray, v) Particle accelerator, vi) magnet such as Superconducting magnet, vii) coli such as Radiofrequency coil, viii) transducer or piezo element such as Ultrasound transducer or piezo elements, ix) Laser, x) LED, xi) Light source, xii) Electrical current or electromagnetic wave producer or generator or injector, xiii) Microwave emitter, xi) transmitter or sensor or producer or generator of UV, visible, infrared light, electron, particle, neutron, positrons, X-ray, magnetic field, radio wave, laser light, ultrasound, xi) positron emission tomography, xii) computed tomography, xiii) magnetic resonance imaging, and xiv) photoacoustic imaging.

The invention further relates to the system according to the invention, wherein:

- Each source of radiation or radiation beam that it emits is associated with or produces or has, preferentially under the conditions of use of the system, preferentially outside the interference or common region:

   ∘ a temperature of less than 100, 50, 10, 5, 2, 1, or 0.1 °C;
   ∘ a temperature distribution that is lower than 100, 50, 10, 5, 2, 1, or 0.1 °C per meter, cm or mm preferentially of the body part or region to be treated or of at least one region outside the body part or region to be treated;

- The at least two sources of radiation or radiation beams that they emit are associated with or produce or have preferentially under the conditions of use of the system, preferentially in the common or interference region:

   ∘ a temperature of more than 0, 0.1, 1 5 or 10 °C;
   ∘ a temperature distribution that is larger than 0, 0.1, 1 5 or 10 °C per meter, cm or mm preferentially of the body part or region to be treated or of at least one region outside the body part or region to be treated or per body part or region to be treated in part or in full;

where preferentially, as defined here, the temperature is the heat temperature of the beam of radiation; where preferentially, as defined here, the temperature distribution is the temperature or temperature variation of the beam of radiation within a given region such as the interference or common region in part or in full.

The invention further relates to the system according to the invention, for use in a method of treatment of a body part or region, wherein preferentially:

- In a first optional step, the body part or region to be treated is imaged preferentially by a first equipment such as MRI or CT or PET;

- In a second optional step, the body part or region to be treated is imaged preferentially by a second equipment such as an ultrasound imaging probe that is preferentially attached to or embedded in or associated with the first part of the system, preferentially using a software or artificial intelligence that preferentially enables integrating the image of the body part or region to be treated obtained by the first equipment in the second equipment so that the second equipment images and detects the body part or region to be treated;

- In a third step, at least one nanoparticle of the second part and/or the thermometer is/are injected or administered or switched on preferentially in the body part or region to be treated, preferentially under imaging guidance provided by the second equipment;

- In a third preferential step, the at least two different sources of radiation are positioned, preferentially under imaging guidance provided by the second equipment, in a region of the body part or region that has at least one property selected in the group consisting of:

    o it enables partly or fully the at least two different beams of radiation to cover or reach or target or be orientated in the direction of the body part or region to be treated;
    o it is below or above or on the side of the body part or region to be treated;
    o it is separated, preferentially by more than 1, 10, $10^3$ or $10^6$ or $10^8$ nm, from the region of the body part or region to be treated by a region that separates the body part or region to be treated from the emission region such as a healthy site, a site surrounding the body part or region to be treated;

- In a fourth step, the system, the at least two different sources of radiation and/or the cooling part, is/are switched on and/or at least one parameter of the system, and/or of the at least two different sources of radiation such as its/their intensity, frequency, is/are increased, and/or at least one parameter of the cooling part such as its temperature is decreased, preferentially resulting in:

    o the body part or region to be treated to be exposed to the at least two different beams of radiation and/or be heated by at least 0, 0.1, 1, 5 or 10 °C;
    o at least one region outside the body part or region to be treated to be exposed by less beams of radiation than the body part or region to be treated and/or to be heated by less than 0, 0.1, 1, 5 or 10 °C;

- In a fifth optional step, the thermometer is switched on, the temperature of the body part or region to be treated is preferentially measured, and at least one parameter of the source of radiation is preferentially adjusted preferentially to maintain the temperature of the body part or region to be treated at the desired temperature;

- In a sixth preferential step, the system, the at least two different sources of radiation and/or the cooling part, is/are switched off and/or the at least one parameter of the system, and/or of the at least two different sources of radiation such as its/their intensity, frequency, temperature is decreased and/or the at least one parameter of the cooling part such as its temperature is increased, preferentially resulting in the body part or region to be treated and/or at least one region outside the body part or region to be treated to return to physiological temperature or 37°C or to a temperature differing from the physiological temperature or 37°C by less than 100, 50, 10, 5, 2, 1 or $10^{-1}$ °C;

- In a seventh optional step, the size or content or nature or composition of the body part or region to be treated is measured, preferentially to examine if the number, volume, of pathological site or cell or biological material has decreased, preferentially following at least one step of the method,

    wherein optionally at least one step of the method, preferentially the fourth one, is repeated more than 1, 2, 3, 4, 5, 6 or 10 times,
    wherein optionally at least one step of the method, preferentially the fourth one, is repeated a larger number of times than another step of the method, preferentially the third one,
    wherein optionally the duration of at least one step of the method, preferentially the fourth one, is sufficiently long, preferentially larger than 0, 0.001, 0.1, 1, 2, 5 or 10 minute(s), preferentially to enable the temperature of the body part or region to be treated to be maintained during a sufficiently long period of time at the desired temperature preferentially in order for the treatment to be efficient;
    wherein optionally the duration of at least one step of the method, preferentially the fourth one, is sufficiently short, preferentially shorter than $10^{20}$, 100, 50, 20 or 10 minute(s), preferentially to avoid overheating the body part or region to be treated, preferentially to avoid side effects;
    wherein preferably any of the at least two steps of the method follow or precede each other in any given order, step 1 after or before step 2, 3, 4, 5, 6 or 7, step 2 after or before step 1, 3, 4, 5, 6 or 7, step 3 after or before step 1, 2, 4, 5, 6 or 7, step 5 after or before step 1, 2, 3, 4, 6 or 7, step 6 after or before step 1, 2, 3, 4, 5, or 7, and/or step 7 after or before step 1, 2, 3, 4, 5, or 6.

In some cases, the number of sources of radiation is lower or equal than the number of foci, focal point or region.

In some other cases, the number of sources of radiation is larger or equal than the number of foci, focal point or region.

In one embodiment of the invention, a foci is selected in the group consisting of:

i) a region where the pathological cells or materials are concentrated, preferentially above 0, $10^{-5}$, $10^{-1}$, 1, 5, 10 or 100 milligram, $\mu g$ or nanogram, preferentially per nm, $nm^2$, $nm^3$, $\mu m$, cm or m,

ii) a pathological site that is preferentially imaged or detected or delineated by an imaging method such as MRI.

In one embodiment of the invention, the foci, focal point or region, is the region where the radiation beam is the most concentrated and/or heats up and/or heats up more than in at least one location outside this region.

In some cases, the temperature variation of the at least one or two beams of radiation or of the interference region is larger than $10^{-5}$, $10^{-1}$, 1 or 0 °C, preferentially per cm or mm or between the center and the border of the at least one or two beams or interference region.

In some other cases, the temperature variation of the at least one or two beams of radiation or of the interference region is smaller than $10^5$, $10^3$, 10, 2, 1, 0.1 or 0 °C, preferentially between the center and the border of the at least one or two beams or interference region.

In still some other cases, the number of foci, focal point or region, preferentially in the interfering region is larger than 0, 1, 2 or 5, and/or increases when the number and/or frequency of at least one or two beam(s) of radiation increase(s).

In one embodiment of the invention, the region that is outside the interference or emission region or is between the interference and emission region has at least one property selected in the group consisting of:

i) it comprises at least one healthy site or tissue or cell or biological material, the rectum, rectal wall, the erectile nerve, at least one bone, the urethra, the sphincter, at least one organ, in part or in full

ii) it is not heated by more than 0, 0.1, 1, 5 or 10 °C.

In one embodiment of the invention, the frequency of the at least one or two beams of radiation is increased or maintained above $10^{-3}$, 0.1 or 10 Hz, Kz or MHz and/or the number of sources of radiation is decreased or maintained below $10^3$, 100 or 10, preferentially to avoid heating or overheating at least one healthy site or tissue or cell or biological material, the rectum, rectal wall, the erectile nerve, at least one bone, the urethra, the sphincter, at least one organ, in part or in full.

In one embodiment of the invention, the frequency of the at least one or two beams of radiation is decreased or maintained below $10^5$, $10^3$, 10, 5, 2, 1 or 0 Hz, Kz or MHz or the number of sources of radiation is increased or maintained above 0, 1, 5, 10, $10^3$ or $10^5$, preferentially to heat homogenously the interference region.

In another embodiment of the invention, the frequency of the at least one or two beams of radiation is maintained between 0 or 0.1 and $10^3$ or $10^5$ Hz, Kz or MHz an/or the number of sources of radiation is maintained between 0, 1, 2, 4 or 10 and 5, 10, 50, 100 or 1000, preferentially to enable uniform heat distribution within the interference region, i.e. preferentially a variation of temperature of less than 100, 10, 1 or 0.1 °C, while avoiding overheating, i.e. preferentially a variation of temperature of more than 0.1, 2, 5, 10 or 100 °C, preferentially in at least one region or healthy site preferentially outside the interference region.

In another embodiment of the invention, the interference region is the region in which at least two beams of radiation emitted by at least two different sources of radiation are separated by a distance that is less than half of diameter of the interference region or body part or region to be treated.

In another embodiment of the invention, the distance separating the source of radiation and the body part or region to be treated is larger than 0, 0.1, 0.5, 1 or 10 cm.

In another embodiment of the invention, the distance separating the source of radiation and the body part or region to be treated is smaller than 100, 10, 5, 2, 1, 0 or 0.1 cm.

In another embodiment of the invention, the at least one or two source(s) of radiation has/have at least one or two aperture radius of curvature, diameter, surface preferentially of emission, concavity, that is/are sufficiently large or pronounced to cover a sufficiently large part of the interfering region or body part or region to be treated.

In another embodiment of the invention, the at least one or two source(s) of radiation has/have at least one or two aperture radius of curvature, diameter, surface preferentially of emission, convexity, that is/are sufficiently large or pronounced to avoid a strong overlap of the at least one or two beam(s) of radiation outside of the interference region or body part or region to be treated.

In another embodiment of the invention, the at least one or two source(s) of radiation comprise(s) at least one or two radiation-emitting element(s), that are preferentially positioned on or within or on top or at the surface of a geometric figure, preferentially a circle or arc of a circle, preferably having a radius or aperture radius or length or surface, preferentially in some cases larger than the radius of the body part or region to be treated, preferentially in some other cases smaller than the radius of the body part or region to be treated.

In some cases, the at least two radiation-emitting elements are offset from each other, preferentially in such ways that they enable the at least two beams that they emit to:

i) enter the body part or region to be treated through a different angle;
ii) in some cases cover the same portion of the body part or region to be treated;
iii) in some other cases cover a different portion of the body part or region to be treated.

In one embodiment of the invention, the at least two sources of radiation or radiation-emitting elements or beams of radiation are positioned at a distance of at least 0, 0.5, 10 or 100 mm from each other.

In another embodiment of the invention, the at least two sources of radiation or radiation-emitting elements or beams of radiation are positioned at a distance of less than $10^{10}$, $10^5$, $10^3$, 10, 5, 2, 1, 0.1 0, 0.5 or 0.1 mm from each other.

In another embodiment of the invention, at least two axes, preferably perpendicular to at least two sources of radiation or radiation-emitting elements or parallel to the two beams of radiation, form an angle of at least 0, 0.1, 1, 2, 5, 10, 25 or 45° with each other, preferentially sufficiently large to enable the interference region or body part or region to be treated to be penetrated by the at least two beams of radiation or heated above 0, 0.1, 1, 2, 5 or 10 °.

In still another embodiment of the invention, at least two axes, preferably perpendicular to at least two sources of radiation or radiation-emitting elements or parallel to the two beams of radiation, form an angle of less than 360, 180, 100, 90, 45, 20, 10, 5, 2 or 1° with each other, preferentially sufficiently small preferably to avoid that at least one region outside the interference region or body part or region to be treated is penetrated by the at least two beams radiation or heated above 0, 0.1, 10, 37, 40, 45, 50, 70 or 100 °C.

In still another embodiment of the invention, the radius of the geometric figure supporting the at least one or two source(s) of radiation or radiation emitting element(s), or the angle between the at least two beams of radiation, the distance between two source(s) of radiation or radiation emitting element(s) and/or the number of source(s) of radiation or radiation emitting element(s), is/are smaller than $10^{10}$, $10^3$, 100, 50, 25, 10, 5, 2, 1 cm or °, preferably to avoid that at least one region outside the interference region or body part or region to be treated is penetrated by the at least two beams radiation or heated above 0, 0.1, 10, 37, 40, 45, 50, 70 or 100 °C.

In still another embodiment of the invention, the radius of the geometric figure supporting the at least one or two source(s) of radiation or radiation emitting element(s), or the angle between the at least two beams of radiation, the distance between two source(s) of radiation or radiation emitting element(s), and/or the number of source(s) of radiation or radiation emitting element(s), is/are larger than $10^{-10}$, $10^{-3}$, $10^{-1}$, 0, 1, 2, 5, 10, 50, 100, $10^3$ or $10^5$ cm or °, preferably to enable the interference region or body part or region to be treated to be penetrated by the at least two beams of radiation or heated above 0, 0.1, 10, 37, 40, 45, 50, 70 or 100 °C.

In still another embodiment of the invention, the size(s) of the source(s) of radiation, radiation emitting element(s), radiation beam(s), is increased or widened, preferentially to decrease the number of source(s) of radiation, radiation emitting element(s).

In still another embodiment of the invention, the size(s) of the source(s) of radiation, radiation emitting element(s), radiation beam(s), is decreased or narrowed, preferentially to increase the number of source(s) of radiation or radiation emitting element(s).

In still another embodiment, the focal gain of the system is increased by reducing the sizes of the sources of radiation, preferentially their emitting surface, and/or by increasing the number of sources of radiation, where the focal gain is preferentially the level of focalization or presence of the at least two beams of radiation in the interference region.

In still another embodiment of the invention, the system delivers more heat or energy at the edges than at the center of the interference region or body part or region to treated, preferentially to achieve uniform heating.

In still another embodiment of the invention, the system achieves uniform heating preferentially of the interference region or body part or region to be treated on a short time scale, preferentially smaller than $10^3$, $10^2$, 10, 5, 2 or 1 second.

In still another embodiment of the invention, the system operates over at least 1, 2, or 5 heating cycle(s), where at least one heating cycle comprises sonicating at least one focal point or region at least 1, 2 or 5 times, preferentially in some cases within less than 1000, 100, 10, 5, 2 or 1 second(s) or minute(s), preferentially in some other cases within more than 0, 1, 2, 5, 10, 60, 600, 10 000 second(s) or minute(s). In still another embodiment of the invention, the system operates over less than 10, 5, 2 or 1 heating cycle(s),

In still another embodiment of the invention, the system operates over a time-averaged intensity (ISPPA) that is smaller than $10^{10}$, $10^5$, $10^3$, 100 or 10 W/cm². 

In still another embodiment of the invention, the system operates over a time-averaged intensity (ISPPA) that is larger than $10^{-10}$, $10^{-5}$, 0.1, 1 or 10 W/cm².

In still another embodiment, at least one part of the system generates a pressure that is lower than 100, 10, 5, 2, 1 or 0.5 MPa, preferentially in the interfering region or body part or region to be treated, preferentially to avoid cavitation.

In still another embodiment, at least one part of the system generates a pressure that is larger than $10^{-5}$, $10^{-3}$, 0.1, 0, 1, 2, 5, or 10 MPa, preferentially in the interfering region or body part or region to be treated, preferentially to generate cavitation.

In still another embodiment of the invention, the at least one heating cycle comprises more than 1, 2, 5, 10, 100, 1000 or 2000 sonication.

In some cases, each sonication occurs less than 100, 50, 10, 5, 2 or 1 time(s) preferentially in some cases at a different location preferentially focal point or region preferentially in some other cases at the same location preferentially focal point or region preferentially in some cases within the interference region or body part or region to be treated preferentially in some other cases outside the interference region or body part or region to be treated.

In still another embodiment of the invention, the spatial peak pressure, intensity, dwell time, and/or pulse duration preferentially of at least one part of the system is/are weighted or adjusted preferentially for each focus or sonication, preferentially to achieve the optimum time averaged intensity,

In still another embodiment of the invention, the pulse durations for each of the focal point or region or sonication is larger than 0, 1, 5 or 10 millisecond(s).

In still another embodiment of the invention, the pulse durations for each of the focal point or region or sonication is smaller than $10^{10}$, $10^5$, $10^3$, 100, 50, 20, 10 or 5 millisecond(s).

In still another embodiment, the distance between the at least two sources of radiation is smaller than the diameter of the interfering region or body part or region to be treated.

In still another embodiment, at least two beams of radiation form an angle with respect to the axis perpendicular to the surface of the at least one radiation emitting element or to the elongation axis of the third part of the system that are different.

In still another embodiment, the number of sources of radiation is larger than the ratio between the diameter of the interference region or body part or region to be treated and the diameter of the at least one source of radiation or emitting surface of this source.

In still another embodiment of the invention, the system is configured so that the number of sonication, focal point(s) or region(s) is some cases larger than 0, 1, 2 5 and 10, in some other cases smaller than 1000, 100, 20, 10, 5 or 2.

In still another embodiment of the invention, the system is configured so that at least two different sonication occur at least two different focal points or regions or locations preferentially within the interference region or body part or region to be treated.

In still another embodiment of the invention, the system is configured to generate a heating cycle comprising at least one sonication covering each or at least 1, 2, 5 or 10 focal point(s) or region(s) of the interference region or body part or region to be treated, preferentially during a period of at least one nanosecond, microsecond, millisecond or second.

In still some cases, the volume of the body part or region to be treated or interference region Vtot is equal to the sum of the volume of each focal region fi, where the total number of focal regions is n, where each focal region is the region covered by at least one beam emitted by at least one source of radiation in the interfering region or body part or region to be treated.

$$Vtot = \sum_{i=1}^{i=n} fi$$

In still some other cases, $V_{tot}$ is smaller than the sum of the volume of each focal region fi, preferentially when the various radiation beams emitted by the different sources if radiation don't fully cover the interference region or body part or region to be treated.

In still some other cases, $V_{tot}$ is larger than the sum of the volume of each focal region fi, preferentially when the various radiation beams emitted by the different sources if radiation fully cover the interference region or body part or region to be treated and at least one region outside the interference region or body part or region to be treated.

In still another embodiment, the sources of radiation are positioned such that the interference of the at least two beams of radiation that they emit occurs partly or fully:

- outside the emission region ;
- outside the region that separates the emission region from the interference region or body part or region to be treated;
- inside the interference region or body part or region to be treated.

In another embodiment of the invention, the geometric figure is selected in the group consisting of: Point, Line, Line segment, Ray, Angle, Triangle, Equilateral triangle, Isosceles triangle, Scalene triangle, Right triangle, Acute triangle, Obtuse triangle, Quadrilateral, Square, Rectangle, Parallelogram, Rhombus, Trapezium, Trapezoid, Kite, Pentagon, Regular pentagon, Irregular pentagon, Hexagon, Regular hexagon, Heptagon, Octagon, Nonagon, Decagon, Hendecagon, Dodecagon, Polygon, Regular polygon, Star polygon, Circle, Semicircle, Sector, Segment, Annulus, Ellipse, Oval, Reuleaux triangle, Lens, Crescent, Arc, Tetrahedron, Triangular pyramid, Square pyramid, Pentagonal pyramid, Hexagonal pyramid, Frustum, Cube, Cuboid, Rectangular prism, Triangular prism, Pentagonal prism, Hexagonal prism, Parallelepiped, Octahedron, Dodecahedron, Icosahedron, Prism, Pyramid, Bipyramid, Antiprism, Trapezohedron, Sphere, Hemisphere, Ellipsoid, Oblate spheroid, Prolate spheroid, Cylinder, Right circular cylinder, Oblique cylinder, Cone, Right circular cone, Oblique cone, Torus, Solid of revolution, Spherical cap, Spherical segment, Paraboloid, Elliptic paraboloid, Hyperbolic paraboloid, Hyperboloid, Hyperboloid of one sheet, Hyperboloid of two sheets, Cylindroid, Wedge, Polyhedron, Regular polyhedron, Irregular polyhedron, Platonic solid, Archimedean solid, Catalan solid, Johnson solid, Prismatoid, Frustum of pyramid, Frustum of cone, and Capsule.

The invention is followed by the non-limiting examples below.

*Experimental example 1*

We use 24-well plates, with each well containing 400,000 adherent PC3 prostate cells, exposed to a suspension of magnetosomes coated with carboxymethyl dextran and formulated with 5% sorbitol, at an iron concentration of 1 mg/mL. The cells are incubated with the magnetosomes for 3 hours before undergoing laser heat treatment. The laser emits light at $808 \pm 3$ nm, with a maximum power of 3 W, a beam collimation diameter of 1 cm after 5 cm, and a fiber diameter of 400 $\mu$m.

In the first condition, two wells were irradiated from the top (15 cm distance between the collimator and the well) at a power of 0.75 W/cm$^2$. In the second condition, two wells were irradiated simultaneously, with one fiber illuminating the bottom of the well and the other the top, at a 15 cm distance and a power of 0.75 W/cm$^2$ each. The two fibers were horizontally offset by 0.5 mm to avoid excessive overlap of the laser beams in the same direction. In the third condition, two wells were irradiated from the top at a power of 1.5 W/cm$^2$, again at a 15 cm distance.

The fourth and fifth conditions serve as controls: one with untreated cells, neither by laser nor magnetosomes, and the other with magnetosomes only. For all three laser treatment conditions, the laser was applied for 30 minutes.

The third condition reached 45 °C in 153 seconds, faster than the first condition, which reached 45 °C after 299 seconds. The first condition, in fact, did not reach 45 °C. The maximum temperatures reached after 30 minutes were 49.41 °C for the first condition, 28.09 °C for the second, and 54.60 °C for the third.

After 48 hours, microscopic observation of the cells from conditions 1, 2, and 3 showed morphological changes: the cells became more rounded. The cells in condition 2, however, were more elongated and resembled those of the control wells (conditions 4 and 5, without irradiation). The cell death rate was 20% higher in condition 1 compared to condition 3, and 60% higher in condition 2. In conditions 4 and 5, all cells remained alive.

*Experimental example 2*

This example details the simulation approach that was implemented to determine the design of an ultrasound array probe suitable for heating prostate tumours, within the design specifications (Volume of 2.5 cm in diameter 0.5 cm above the rectal wall).

This example concludes with a transducer or piezo element design which comprises element positions, size and operating frequency, and a recommendation for use to obtain the desired heating which comprises focal target locations, source and focal pressure/intensity and pulse durations which generate tumour heating on a 1 second time scale.

The simulations were completed using:

1. MATLAB 2025a + Optimization toolbox
2. k-Wave: A MATLAB toolbox for the time domain simulation of acoustic fields

The design recommended is a 78-element array consisting of two strips of pseudo-randomly distributed 2.4 mm diameter elements operating at 1 MHz, placed laterally to the B&K imaging probe. The strips should be spherically focused, with a radius of curvature of 6.25 cm. The distance between the heating probe and the rectal wall should be 1 cm; smaller distances are acceptable but will increase rectal heating. An array driving regime was created to optimize ultrasound intensity distributions to primarily heat the edges of the tumour; heat from the edges will dissipate inwards, improving heating uniformity when compared to a homogeneous intensity delivered throughout the tumour. This driving regime requires selection of 1000-2000 foci (randomly or homogeneously) distributed over a 0.5 cm shell within the tumour, with pulse durations optimized for each of the targets. This optimized intensity distribution, when scaled to 8.5 W/cm$^2$, can heat the tumour to 45°C in less than five minutes, and can maintain this temperature indefinitely using an algorithm that turns the heating on/off depending on the mean temperature within the tumour. Standard deviations in the equilibrium tumour temperature are 1.6°C for a 2.5 cm diameter spherical tumour, and 0.9°C for a 2 cm diameter spherical tumour.

**Simulation-based array optimisation**

Repeated simulations were used to identify an optimal array operating frequency, the optimal minimal distance between the heating array and the rectal wall, the optimal array radius of curvature, and the optimal element radius.

The number of targets would be greater for higher frequencies, due to the reduction of the focal size at higher frequencies.

Six targets were selected within the 2.5 cm diameter tumour volume to assess the steering range of the array needed for homogeneous heating. These targets span the steering range required to focus ultrasound throughout the prostate tumour.

The first parameter optimised was frequency. Frequency modifications can improve the intensity ratio between tumour and rectal wall.

500 kHz sonications fail to generate a uniform intensity within the tumour, subject to a constraint that minimizes the intensity in the rectal wall.

Higher frequencies may generate sufficiently small foci allowing reasonable intensity to be generated at the edges of the tumour without continually increasing intensity at the centre of the tumour.

The selection of an intermediate frequency (e.g., 1 MHz) can balance these considerations. 1 MHz ultrasound is used for the following simulations due to success in prior small animal work. Simulations show that the minimum separation between the heating array and the rectal wall should be around 1 cm.

Simulations tested the effect of aperture radius of curvature and element diameter.

We placed lower limit of 5 cm on the radius of curvature, as smaller radii of curvature (and a fixed aperture diameter of 6.3 cm) will either exceed the allowed height/thickness of the array or become shadowed by the imaging probe.

Element diameter was also optimised, keeping a maximum element number of 128 to restrict the end cost of the device.

For element diameters of 2 mm and above, the arrays contain fewer elements due to space limitations. for the radius of curvature.

The original cost function (unweighted by focal volume) has a minimum at 6.25 cm, while the focally weighted cost function has a minimum at 16 cm. The arrays simulated to determine optimum radius of curvature all use 2 mm diameter elements.

We proceeded with the 6.25 cm radius of curvature array for the element diameter optimisation to preserve gain. The element diameter optimisation shows a minimum in the cost function 1 for element diameters of 2.2-2.4 mm. Larger elements result in fewer elements in the aperture, and the array with 2.4 mm diameter elements only has 78 elements. Excessively large elements reduce focal gain, improving cost function 2 but reducing field control. Fewer elements reduce cost, so we selected the array with seventy-eight 2.4 mm diameter elements.

The final optimised array has 78 randomly distributed 2.4 mm diameter elements placed on a spherically curved surface (radius of curvature: 6.25 cm). The element positions are restricted by the maximum lateral dimensions (width < 3 cm) and by the position of the imaging transducer or piezo element. The heating elements are placed laterally to the main imaging elements such that the field of view overlaps with the prostate heating region.

**Driving algorithm development**

We designed an algorithm that can efficiently compute the focal positions that are necessary to generate heating that is close to homogeneous throughout the 10+ minute exposure. This demand is challenging due to heat diffusion from the edges of the tumour region. This means that more energy must be deposited at the tumour edges compared to the center. This is opposite to the natural focusing of the array towards the center of the tumour volume. Targets near the edges of the tumour will be favourable, so an algorithm was developed to rapidly compute the optimal intensity distribution.

The algorithm has the following steps: Define the source positions, rectal wall position, and tumour position; Simulate the field produced by each individual element in the region of the rectal wall and the tumour volume, producing N1 amplitude and N1 phase fields in 3D for an array that has N1 elements, Selecting N2 target points within the tumour, where these target points may be distributed homogeneously or randomly throughout the tumour volume, or homogeneously or randomly throughout a 'shell' that emphasizes focusing ultrasound to the edges of the tumour volume for heating homogeneity, where N2 values between 1000 and 2000 were chosen, for each of the N2 target points, calculating the complex weighting factor w (phase correction, or phase and amplitude correction) for each of the N1 complex pressure (amplitude and phase) fields to focus the elements to the targets, calculating the intensity from the pressure values and save the intensity field for each of the N2 targets, using these intensity fields to create a matrix 'C', with one dimension being N2 and the second dimension being the number of grid points within the tumour (size of d), and a matrix 'A', with one dimension being N2 and the second dimension being the number of grid points within the rectal wall (size of b), defining a desired intensity distribution 'd' for the tumour. defining a threshold intensity distribution 'b' for the rectal wall, defining lower and upper boundaries for 'xtime', Solving for xtime using the linear least squares optimisation algorithm, summing the N1 intensity fields, multiplied by their respective and optimised xtime values, to obtain the optimized intensity distribution.

To achieve uniform heating on a short timescale (e.g., one second), one heating cycle requires sonicating each useful focal point once within a one second period. Small peaks and minima in the intensity distribution will rapidly homogenize during the evolution of the thermal distribution. However, the relatively sharp interface at the tumour edge means that heat will quickly dissipate from the tumour edge into the surrounding tissues, resulting in lower temperatures at the tumour edge and higher temperatures at the centre of the tumour. An optimised heating distribution will favour increased intensities at the tumour edges. The 'xtime' values optimised for the uniform distribution shows a heterogeneous intensity distribution that is intended to increase heating at the tumour edges relative to the centre of the tumour, to counteract heat diffusion away from the tumour edges.

**Intensity scaling to therapeutic values**

The intensity values up until this point have been relative values, with the optimizing aiming for e.g. 100 W/cm$^2$ in the tumour and 0 elsewhere. A time-averaged acoustic intensity of 100 W/cm2 is likely to be excessive for the application. The optimised intensity distribution should be scaled to values that are conducive to the application.

Initial thermal simulations suggest that a time-averaged intensity (ISPPA) of 8.5 W/cm$^2$ would generate heating on the order required here. This corresponds to a pressure of 0.5 MPa where the spatial-peak pulse-averaged intensity for a plane wave.

The time averaged intensity can then be calculated from the ISPPA multiplied by the duty factor, which for a square pulse envelope is equal to the pulse duration divided by the pulse repetition period. In the case presented here, one full heating cycle is formed of 2000 sonications, each with a different focal position, performed once each over a period of time. To achieve the optimum time averaged intensity, the spatial peak pressure or intensity and the dwell time or pulse duration is weighted for each focus. The time averaged intensity distribution (ITA) can be scaled such that the ISPTA is 8.5 W/cm$^2$. The spatial peak pulse-averaged intensity (ISPPA) can then be calculated for each target using the optimised dwell times and intensity weightings.

The pulse durations for each of the foci range from zero (i.e., can be skipped) to 15 milliseconds. Focal switching on a 1 millisecond duration timescale is necessary. The peak pressures and ISPPA values needed to obtain a peak ISPTA value of 8.5 W/cm2 range from 1.8-2.1 MPa and 100- 150 W/cm$^2$ respectively. These intensities are well below those used in HIFU (>1kW/cm$^2$), and the pressures are below the expected threshold for de novo cavitation.

### Coupled Acoustic-Thermal Simulations: How do the optimized fields perform when generating heat in a tumour?

The simulation domain was defined using the acoustic properties of water, the rectal wall, and the prostate tumour. Heat deposition is then calculated from the acoustic intensity distribution for every voxel in the 3D simulation domain. The evolution of temperature due to the acoustic heat source and thermal conduction is modelled using the Bioheat Equation solver from the k-Wave toolbox.

Heating using optimized intensity distribution, with the rectum filled with 30°C water, where heating is applied when the mean tumour temperature is below 45°C, and turned off when the mean tumour temperature exceeds 45°C, the heating distribution is not perfectly uniform, with lower temperatures at the tumour edges close to the rectal wall and distal to the rectal wall. However, the heat distribution in the centre of the tumour (e.g., within a 2 cm diameter tumour rather than the depicted 2.5 cm diameter tumour) is uniform to within $\pm$1°C. Further optimisation of the heating pattern could improve the homogeneity of the tumour heating for larger tumours. In these simulations, rectal cooling with 30°C water is sufficient to keep the rectal wall temperature below 41°C.

The heating is turned off when the mean temperature in the 2.5 cm diameter tumour exceeds 45°C. The equilibrium heating/cooling may be maintained indefinitely. The standard deviation of the temperature in a 2.5 cm diameter tumour after it reaches 45°C is 1.6°C. The standard deviation of the temperature in the central 2 cm diameter tumour is 0.9°C. This shows that cooling due to heat dissipation at the tumour edges is the main challenge to obtaining heating uniformity within the tumour.

Heating rate was not specified in the scope of work, but rather the aim to heat to 45°C for 10 minutes. The heating rate must be in the >degree/minute range, to reach the target temperature within a reasonable time frame. We suggest that it should take less than 5 minutes for the probe to generate an average temperature within the tumour of 45°C. Simulations were performed with a range of ISPTA values of 2.1 W/cm$^2$, 8.4 W/cm$^2$, 34 W/cm$^2$, and 76 W/cm$^2$. Continuous heating was simulated for each intensity for one minute.

The heating rate increases linearly with intensity, and quadratically with pressure. The pressures for the ISPTA of 2.1 W/cm$^2$, 8.4 W/cm$^2$, and 76 W/cm$^2$ correspond to peak pressures around 1 MPa, 2 MPa, 4 MPa and 6 MPa respectively. ISTPA values around 10 W/cm$^2$ are sufficient for this application and should be expected to heat the tumour to 45°C in less than four minutes. ISPTA values above 10 W/cm$^2$ will result in faster but potentially less controllable heating.

Summary

Optimized array: driving frequency (1 MHz), separation between the rectal wall and the heating array (1 cm), the array radius of curvature (6.25 cm), and the array element diameter (0.24 cm). This optimized array consists of 78 elements.

Optimized intensity distribution to an ISPTA regime for moderate ultrasonic heating, near 10 W/cm$^2$. We show that ISPTA values in this range can heat the tumour to a mean temperature of 45°C within five minutes, then maintain this temperature indefinitely. This ISPTA range corresponds to pulse-averaged intensities in the 100-150 W/cm$^2$ range and pressures in the 2 MPa range.

This report demonstrates that 2 cm diameter tumours that are not directly in contact with the rectal wall (0.25 cm offset) can be heated to a uniform temperature of 45°C with a standard deviation in tumour temperature of under 1°C.

### Claims

1. A radiation heating system for treating a body part of an individual comprising:

- A first part comprising different sources of radiation, where each source emits at least one beam of radiation:

wherein each radiation source is positioned at a different location relatively to a horizontal support axis preferentially of the system, such that the radial or angular positions of the sources around or relatively to said axis are different,

wherein at least two different radiation sources have at least two different orientations defined by at least two different emission angles measured with respect to a common reference axis, and

wherein said sources of radiation are positioned and orientated so that the beams spatially intersect or

overlap within a common region that is distinct from the emission or focal or Near-field region of at least one emitting or piezo element and/or is comprised in the near-field region of the assembly of emitting or piezo elements preferentially comprised in the system,

wherein the number of sources of radiation is at least 3, 4, 5, 10, 20, 50, 60, 70, 80, and at most $10^{20}$, $10^3$, or 100,

wherein each source of radiation is configured to have at least one property selected in the group consisting of:

i) an opening or diffracting angle (T) preferentially of the beam emitted by the source of radiation larger than 0, 1, 2, 5, 25 or 45°, preferentially measured between two opposite directions or points of the beam perimeter at the level of the emission region;

ii) a diameter or width or thickness or its largest dimension smaller than one half the diameter or width or thickness of the body part to be treated or body part into or onto which the system or at least one part of the system is positioned or inserted or than 100, 10, 1 or 0.5 cm, and

iii) a frequency, preferentially of the beam that it emits, that is smaller than 100, 10, 5, 2 or 1.5 MHz,

- A second part comprising at least one magnetic or metallic nanoparticle located in said common region and/or exposed to the intersecting or overlapping beams of radiation.

2. The system according to claim 1, where the at least one nanoparticle or the system preferentially is not or does not comprise or does not operate through the generation or does not have the function of generating at least 1, 2, 5, 10 or 100 nanobubble(s), bubble(s), or microbubble(s).

3. The system according to claim 1, which does not comprise at least one fluorophore.

4. The system according to claim 1, wherein the at least one nanoparticle has at least one property selected in the group consisting of:

- it forms an assembly of at least two nanoparticles, where the at least two nanoparticles have at least one property selected in the group consisting of: i) they are arranged in a chain, ii) they are arranged in a geometric figure, iii) they are linked or associated to each other, preferentially through binding or coating material, iv) they are separated by less than 10, 1, 0.1 $\mu$m or than the diameter of at least one nanoparticle, v) they have a larger absorption, transmission, and/or reflection of the radiation beam emitted by the first part than a single nanoparticle, vi) they have a larger movement or oscillation or interaction or internalization with or in cells or biological material preferentially under the application of the radiation beams emitted by the first part than a single nanoparticle, vii) they heat more, preferentially by at least 0, $10^{-20}$, $10^{-5}$, $10^{-3}$, or $10^{-1}$ °C than a single nanoparticle;
- it has at least one magnetic property, preferentially selected in the group consisting of: diamagnetic, paramagnetic, superparamagnetic, ferrimagnetic and ferromagnetic property;
- it comprises at least 1, 2, 5, 10, 100 or $10^3$ metallic atom(s) or more than 1, 5, 10, 50, 75, 80, 90 or 99% in mass or volume of metallic atom(s);
- it is formed before, during or after or results from the application of the at least one or two radiation beam(s) of the first part;
- it heats locally at the nanoparticle or nm scale, preferentially without resulting in a significant temperature increase of the body part to be treated, preferentially by more than 0.1, 1 or 10 °C, preferentially under the application of the at least one or two radiation beam(s) of the first part;
- it has a size, preferentially individual or collective, that is smaller than $10^{10}$, $10^5$, $10^4$, $10^3$, $10^2$, 10, 5, 2 or 1 nm, preferentially in at least one, two or three dimension(s);
- it has a size, preferentially individual or collective, that is larger than $10^{-3}$, $10^{-2}$, $10^{-1}$, 0, 1, 2, 5, 10, 20, 50, 100, $10^3$ or $10^5$ nm, preferentially in at least one, two or three dimension(s);

5. The system according to claim 1, which is a system of double interference **characterized by**:

- At least two different sources of radiations of the first part emit at least two different beams of radiation that interfere according to a first type of interference by producing or being associated with or comprising a superposition partly or fully of the at least two beams of radiation or a coexistence preferentially without superposition partly or fully of the at least two beams of radiation preferentially in the interference or common region,

where the superposition of at least two beams of radiation is the existence or persistence of at least two beams of radiation at the same location of the interference or common region;
where the coexistence without superposition of at least two beams of radiation is the existence or persistence of the at least two beams of radiation in at least two different locations of the interference or common region;

- At least one beam of radiation of the first part emits at least one beam of radiation that interferes with at least one nanoparticle of the second part according to the second type of interference by producing or being associated with or comprising:

a. the absorption, transmission, reflection, of the at least one radiation beam by the at least one nanoparticle;
and/or
b. the movement or oscillation of the at least one nanoparticles, and/or the interaction or internalization of the at least one nanoparticle by at least one cell or biological material;

6. The system according to claim 1, wherein the at least two different source(s) of radiation emit at least two different radiations or beams of radiation, **characterized by** at least one property selected in the group consisting of:

a) At least two different beams of radiation have at least two different values of parameters selected in the group consisting of: orientations or directions of propagation or angles between their direction of propagation and the horizontal or vertical axis (O1, O2), intensity ($I_1$, $I_2$), power ($P_1$, $P_2$), energy ($E_1$, $E_2$), width ($W_1$, $W_2$), depth of penetration ($D_1$, $D_2$), frequency ($F_1$, $F_2$), and wavelength ($W_1$, $W_2$);
and

b) The at least two radiations are of at least two different types of radiations;

wherein the first beam as at least one parameter selected in the group consisting of: O1, $I_1$, $P_1$, $E_1$, $W_1$, $D_1$, $F_1$ and $W_1$;
wherein the second beam as at least one parameter selected in the group consisting of: $O_2$, $I_2$, $P_2$, $E_2$, $W_2$, $D_2$, $F_2$ and $W_2$;
wherein $(O_1-O_2)/(O_1+O_2)$, $(I_1-I_2)/(I_1+I_2)$, $(I_1-I_2)/I_1$, $(I_1-I_2)/I_2$, $(P_1-P_2)/(P_1+P_2)$, $(P_1-P_2)/P_1$, $(P_1-P_2)/P_2$, $(E_1-E_2)/(E_1+E_2)$, $(E_1-E_2)/E_1$, $(E_1-E_2)/E_2$, $(D_1-D_2)/(D_1+D_2)$, $(D_1-D_2)/D_1$, $(D_1-D_2)/D_2$, $(F_1-F_2)/(F_1+F_2)$, $(F_1-F_2)/F_1$, $(F_1-F_2)/F_2$, $(W_1-W_2)/(W_1+W_2)$, $(W_1-W_2)/W_1$, and/or $(W_1-W_2)/W_2$, in absolute value or not, is/are larger than 0, $10^{-5}$, $10^{-3}$, $10^{-1}$, 1, 5, 10, 50, 75, 100 or $10^4$ preferentially%,
wherein the at least one parameter is preferentially measured or exists in at least one region selected in the group consisting of:

a) At least one emission region,
b) At least one interference or common region,

and

c) At least one region separating at least one emission region and at least one interference or common region.

7. The system according to claim 1, wherein the at least two different source(s) of radiation emit at least two different radiations or beams of radiation, **characterized by** at least one property selected in the group consisting of:

a) At least two different beams of radiation have at least two similar or same values of parameters selected in the group consisting of: orientations or directions of propagation or angles between their direction of propagation and the horizontal or vertical axis (O1, O2), intensity ($I_1$, $I_2$), power ($P_1$, $P_2$), energy ($E_1$, $E_2$), width ($W_1$, $W_2$), depth of penetration ($D_1$, $D_2$), frequency ($F_1$, $F_2$), and wavelength ($W_1$, $W_2$);
and
b) The at least two radiations are of the same or similar type of radiations;

wherein the first beam as at least one parameter selected in the group consisting of: $O_1$, $I_1$, $P_1$, $E_1$, $W_1$, $D_1$, $F_1$ and $W_1$;
wherein the second beam as at least one parameter selected in the group consisting of: $O_2$, $I_2$, $P_2$, $E_2$, $W_2$, $D_2$, $F_2$ and $W_2$;

wherein $(O_1-O_2)/(O_1+O_2)$, $(I_1-I_2)/(I_1+I_2)$, $(I_1-I_2)/I_1$, $(I_1-I_2)/I_2$, $(P_1-P_2)/(P_1+P_2)$, $(P_1-P_2)/P_1$, $(P_1-P_2)/P_2$, $(E_1-E_2)/(E_1+E_2)$, $(E_1-E_2)/E_1$, $(E_1-E_2)/E_2$, $(D_1-D_2)/(D_1+D_2)$, $(D_1-D_2)/D_1$, $(D_1-D_2)/D_2$, $(F_1-F_2)/(F_1+F_2)$, $(F_1-F_2)/F_1$, $(F_1-F_2)/F_2$, $(W_1-W_2)/(W_1+W_2)$, $(W_1-W_2)/W_1$, and/or $(W_1-W_2)/W_2$, in absolute value or not, is/are smaller than $10^{20}$, $10^{10}$, $10^5$, $10^3$, 100, 75, 50, 10, 5 or 1 preferentially %,

wherein the at least one parameter is preferentially measured or exists in at least one region selected in the group consisting of:

a) At least one emission region,
b) At least one interference or common region, and
c) At least one region separating at least one emission region and at least one interference or common region.

8. The system according to claim 1 or its first or second part, which is partly or fully used in or administered to or applied to or comprised in the body part to be treated at least 1, 2, 3, 4, 5, 6, 7, 8, 9, 10 or 100 times, preferentially continuously or discontinuously, preferentially for at least 1, 2, 3, 4, 5, 6, 7, 8, 9, 10 or 100 sufficiently long period of times, preferentially of more than 0, 1, 2, 5, 10 or 100 second(s), preferentially to enable the first and/or second part(s) to heal or heat the body part to be treated, preferentially for at least 1, 2, 3, 4, 5, 6, 7, 8, 9, 10 or 100 sufficiently short period of times, preferentially of less than $10^{10}$, $10^5$, $10^3$, 10, 5, 2 or 1 minute(s), preferentially to avoid that the first and/or second part(s) generate(s) side effect(s).

9. The system according to claim 1, which is either used in combination with or comprises or is associated with a third part, which is an imaging element or equipment that has at least one property selected in the group consisting of:

a) it is selected in the group consisting of: X-ray radiography, Fluoroscopy, Computed Tomography (CT), Mammography, Dual-energy X-ray absorptiometry (DEXA), Magnetic Resonance Imaging (MRI), Functional MRI (fMRI), Diffusion Tensor Imaging (DTI), MR Spectroscopy, Cardiac MRI, Ultrasound imaging, Doppler ultrasound, 3D ultrasound, 4D ultrasound, Elastography, Contrast-enhanced ultrasound (CEUS), Scintigraphy, Single Photon Emission Computed Tomography (SPECT), Positron Emission Tomography (PET), PET/CT, PET/MRI, Optical Coherence Tomography (OCT), Fluorescence imaging, Bioluminescence imaging (BLI), Photoacoustic imaging, Thermography, Photoacoustic tomography (PAT), Microwave imaging, Electrical Impedance Tomography (EIT). and a scanner, in part or in full,
b) it comprises a software or artificial intelligence, preferentially a fusion software, that preferentially enables the fusion of at least one image of the body part to be treated taken with at least one first equipment of i) such as MRI with at least one second equipment of i) such as Ultrasound imaging, where the first equipment is preferentially not comprised in the system and the second equipment is comprised in the system;
c) it comprises at least one imaging element, which is located in the region that receives or absorbs or is exposed to at least one radiation or beam of radiation, which is preferentially emitted or reflected or diffracted partly or fully by the at least one nanoparticle or body part or interference or common region or part of it;
d) it comprises at least two imaging elements, which are located in two different regions that receive or absorb or are exposed to at least two different radiations or two different beams of radiation, which are preferentially emitted or reflected or diffracted partly or fully by at least two different nanoparticles or two different sides or facets of at least one nanoparticle or two different portions of the body part or two different portions of the interference or common region;
e) it receives or absorbs or is exposed to at least one radiation or one beam of radiation,
f) it detects or has sufficient sensitivity or has a sufficiently large number or more than one sensor or transducer or piezo element, preferentially located in different regions, to detect the difference in at least one value of at least one parameter between the at least two beams of radiation, which are preferentially emitted or reflected or diffracted partly or fully by at least two different nanoparticles or two different sides or facets of at least one nanoparticle or two different portions of the body part or two different portions of the interference or common region;
g) it is comprised between or in a different region of the at least two emitting elements of the first part;
h) it serves to detect or has sufficient imaging sensitivity or contrasting strength or ability to detect at least one entity, partly or fully, selected in the group consisting of: i) at least one nanoparticle of the second part, ii) at least one body part, iii) at least one interference region, iv) at least one emitting region, and v) at least one region located between the emitting and interference or common regions;
i) it has an imaging sensitivity or imaging strength that is increased or enhanced by the presence of the at least one nanoparticle of the second part; and

j) it serves to detect or has sufficient imaging sensitivity or contrasting strength or ability to detect the at least one beam emitted by the at least one source of radiation or the at least two different beams emitted by the at least two different beams of radiation, preferentially through the detection

10. The system according to claim **1,** wherein the interference or common region and/or body part to be treated is/are selected in the group consisting of:

A pathological site;

A tumor or primary or metastatic tumor or cancer or cancer site;

A site of infection, preferentially a site that is infected by at least one virus, bacterium, tumor cell or pathological biological material;

A region that comprises more pathological cells than healthy cells; and

A region that comprises at least one pathological site that is larger than at least one healthy site;

11. The system according to claim **1,** wherein the interference or common region is larger than the body part to be treated, where the size of the body part to be treated is preferentially measured by the third part of the system, where the interference region preferentially comprises at least 1 or 2 common regions or the sum or assembly of the common regions or is larger than at least one common region.

12. The system according to claim **1,** wherein the source of radiation is selected in the group consisting of: i) source of X-ray such as X-ray tube, ii) source of radioisotopes, iii) accelerator such as linear accelerators (LINAC), iv) source of Gamma ray, v) Particle accelerator, vi) magnet such as Superconducting magnet, vii) coli such as Radiofrequency coil, viii) transducer or piezo element such as Ultrasound transducer or piezo elements, ix) Laser, x) LED, xi) Light source, xii) Electrical current or electromagnetic wave producer or generator or injector, xiii) Microwave emitter, xi) transmitter or sensor or producer or generator of UV, visible, infrared light, electron, particle, neutron, positrons, X-ray, magnetic field, radio wave, laser light, ultrasound, xi) positron emission tomography, xii) computed tomography, xiii) magnetic resonance imaging, and xiv) photoacoustic imaging.

13. The system according to claim **1,** wherein:

- Each source of radiation or radiation beam that it emits is associated with or produces or has, preferentially under the conditions of use of the system, preferentially outside the interference region:

a. a temperature of less than 100, 50, 10, 5, 2, 1, or 0.1 °C;

b. a temperature distribution that is lower than 100, 50, 10, 5, 2, 1, or 0.1 °C per meter, cm or mm preferentially of the body part to be treated or of at least one region outside the body part to be treated;

- The at least two sources of radiation or radiation beams that they emit are associated with or produce or have preferentially under the conditions of use of the system, preferentially in the interference region:

c. a temperature of more than 0, 0.1, 1 5 or 10 °C;

d. a temperature distribution that is larger than 0, 0.1, 1 5 or 10 °C per meter, cm or mm preferentially of the body part to be treated or of at least one region outside the body part to be treated or per body part to be treated in part or in full;

where, as defined here, the temperature is the heat temperature of the beam of radiation;

where, as defined here, the temperature distribution is the temperature or temperature variation of the beam of radiation within a given region such as the interference region in part or in full;

14. The system according to claim **1,** for use in a method of treatment of a body part, wherein preferentially:

- In a first optional step, the body part to be treated is imaged by a first equipment such as MRI or CT or PET;

- In a second optional step, the body part to be treated is imaged by a second equipment such as an ultrasound imaging probe that is preferentially attached to or embedded in or associated with the first part of the system, preferentially using a software or artificial intelligence that preferentially enables integrating the image of the body part to be treated obtained by the first equipment in the second equipment so that the second equipment images and detects the body part to be treated;

- In a third step, at least one nanoparticle of the second part and/or the thermometer is/are injected or administered or switched on preferentially in the body part to be treated, preferentially under imaging guidance provided by the second equipment;

- In a third step, the at least two different sources of radiation are positioned, preferentially under imaging guidance provided by the second equipment, in a region of the body part that has at least one property selected in the group consisting of:

o it enables partly or fully the at least two different beams of radiation to cover or reach or target or be orientated in the direction of the body part to be treated;

o it is below or above or on the side of the body part to be treated;

o it is separated, preferentially by more than 1, 10, $10^3$ or $10^6$ or $10^8$ nm, from the region of the body part to be treated by a region that separates the body part to be treated from the emission region such as a healthy site, a site surrounding the body part to be treated;

- In a fourth step, the system, the at least two different sources of radiation and/or the cooling part, is/are switched on and/or at least one parameter of the system, and/or of the at least two different sources of radiation such as its/their intensity, frequency, is/are increased, and/or at least one parameter of the cooling part such as its temperature is decreased, preferentially resulting in:

o the body part to be treated to be exposed to the at least two different beams of radiation and/or be heated by at least 0, 0.1, 1, 5 or 10 °C;

o at least one region outside the body part to be treated to be exposed by less beams of radiation than the body part to be treated and/or to be heated by less than 0, 0.1, 1, 5 or 10 °C;

- In a fifth optional step, the thermometer is switched on, the temperature of the body part to be treated is measured, and at least one parameter of the source of radiation is adjusted to maintain the temperature of the body part to be treated at the desired temperature;

- In a sixth step, the system, the at least two different sources of radiation and/or the cooling part, is/are switched off and/or the at least one parameter of the system, and/or of the at least two different sources of radiation such as its/their intensity, frequency, temperature is decreased and/or the at least one parameter of the cooling part such as its temperature is increased, preferentially resulting in the body part to be treated and/or at least one region outside the body part to be treated to return to physiological temperature or 37°C or to a temperature differing from the physiological temperature or 37°C by less than 100, 50, 10, 5, 2, 1 or $10^{-1}$ °C;

- In a seventh optional step, the size or content or nature or composition of the body part to be treated is measured, preferentially to examine if the number, volume, of pathological site or cell or biological material has decreased, preferentially following at least one step of the method,

wherein optionally at least one step of the method, preferentially the fourth one, is repeated more than 1, 2, 3, 4, 5, 6 or 10 times,

wherein optionally at least one step of the method, preferentially the fourth one, is repeated a larger number of times than another step of the method, preferentially the third one,

wherein optionally the duration of at least one step of the method, preferentially the fourth one, is sufficiently long, preferentially larger than 0, 0.001, 0.1, 1, 2, 5 or 10 minute(s), preferentially to enable the temperature of the body part to be treated to be maintained during a sufficiently long period of time at the desired temperature preferentially in order for the treatment to be efficient;

wherein optionally the duration of at least one step of the method, preferentially the fourth one, is sufficiently short, preferentially shorter than $10^{20}$, 100, 50, 20 or 10 minute(s), preferentially to avoid overheating the body part to be treated, preferentially to avoid side effects;

wherein any of the at least two steps of the method follow or precede each other preferentially in any given order, preferentially step 1 after or before step 2, 3, 4, 5, 6 or 7, step 2 preferentially after or before step 1, 3, 4, 5, 6 or 7, step 3 preferentially after or before step 1, 2, 4, 5, 6 or 7, step 4 preferentially after or before step 1, 2, 3, 5, 6 or 7, step 5 preferentially after or before step 1, 2, 3, 4, 6 or 7, step 6 preferentially after or before step 1, 2, 3, 4, 5, or 7, and/or step 7 preferentially after or before step 1, 2, 3, 4, 5, or 6.

European Patent Office

Europäisches Patentamt
European Patent Office
Office européen des brevets

# EUROPEAN SEARCH REPORT

**Application Number**

EP 25 21 8235

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/075611 A1 (REYNOLDS JOHN NOBLE JAMES [NZ]; HARRIS PAUL DAVID [NZ] ET AL.) 4 May 2023 (2023-05-04) | 1-12 | INV. A61N7/00 A61N7/02 |
| Y | * page 2, line 8 - line 10 * | 13,14 | A61K9/00 |
|  | * page 5, line 14 - line 23 * | | |
|  | * page 6, line 23 - line 27 * | | ADD. |
|  | * page 7, line 8 - line 29 * | | A61M37/00 |
|  | * page 8, line 18 - page 9, line 7 * | | |
|  | * page 14, line 3 - line 10 * | | |
|  | * page 26, line 3 - line 11 * | | |
|  | * figures 1A-3,5,11-13 * | | |
|  | ----- | | |
| Y | US 2014/058294 A1 (GROSS YOSSI [IL] ET AL) 27 February 2014 (2014-02-27) * paragraphs [0011], [0013] * | 13 | |
|  | ----- | | |
| Y | US 2008/045865 A1 (KISLEV HANOCH [IL]) 21 February 2008 (2008-02-21) * paragraph [0224] * | 14 | |
|  | ----- | | |

**TECHNICAL FIELDS SEARCHED (IPC)**

A61N
A61M
A61K

The present search report has been drawn up for all claims

| Place of search | Date of completion of the search | Examiner |
|---|---|---|
| The Hague | 19 March 2026 | Milles, Julien |

EPO FORM 1503 03.82 (P04C01)

## ANNEX TO THE EUROPEAN SEARCH REPORT
## ON EUROPEAN PATENT APPLICATION NO.

EP 25 21 8235

19-03-2026

| Patent document cited in search report | Publication date | Patent family member(s) | Publication date |
|---|---|---|---|
| WO 2023075611 A1 | 04-05-2023 | AU 2022376876 A1 | 06-06-2024 |
| | | CA 3235025 A1 | 04-05-2023 |
| | | CN 118678987 A | 20-09-2024 |
| | | EP 4422687 A1 | 04-09-2024 |
| | | JP 2024542051 A | 13-11-2024 |
| | | WO 2023075611 A1 | 04-05-2023 |
| US 2014058294 A1 | 27-02-2014 | CN 103764225 A | 30-04-2014 |
| | | EP 2680923 A2 | 08-01-2014 |
| | | US 2014058294 A1 | 27-02-2014 |
| | | WO 2012120495 A2 | 13-09-2012 |
| US 2008045865 A1 | 21-02-2008 | EP 1819277 A1 | 22-08-2007 |
| | | JP 2008519642 A | 12-06-2008 |
| | | US 2008045865 A1 | 21-02-2008 |
| | | WO 2006051542 A1 | 18-05-2006 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 16486574 B

**Non-patent literature cited in the description**

- **A. NAPOLI et al.** High-intensity focused radiation for prostate cancer. *Expert Rev Med Devices*, 2020, vol. 17, 427-433

- **C. EL HEDJAJ et al.** Full Disappearance of PC3-Luc Prostate Tumors Mediated by Hyperthermia Under Low Intensity Ultrasound Application in the Presence of Magnetosomes. *Adv. Therap.*, 2025, 2400281